(19) Europäisches Patentamt
European Patent Office
Office européen des brevets

(11) EP 1 123 928 A1

(12) EUROPEAN PATENT APPLICATION
published in accordance with Art. 158(3) EPC

(43) Date of publication:
16.08.2001 Bulletin 2001/33

(21) Application number: 99947961.1

(22) Date of filing: 19.10.1999

(51) Int Cl.[7]: C07D 243/12, A61K 31/551, C07D 401/04, C07D 403/04, A61P 3/04, A61P 3/10

(86) International application number:
PCT/JP99/05754

(87) International publication number:
WO 00/23428 (27.04.2000 Gazette 2000/17)

(84) Designated Contracting States:
AT BE CH CY DE DK ES FI FR GB GR IE IT LI LU MC NL PT SE
Designated Extension States:
AL LT LV MK RO SI

(30) Priority: 20.10.1998 JP 29894198

(71) Applicant: Takeda Chemical Industries, Ltd.
Osaka-shi, Osaka 541-0045 (JP)

(72) Inventors:
- OI, Satoru
  Nara-shi Nara 631-0033 (JP)
- SUZUKI, Nobuhiro
  Tsukuba-shi Ibaraki 305-0861 (JP)
- MATSUMOTO, Takahiro
  Kawabe-gun Hyogo 666-0245 (JP)

(74) Representative: Caffin, Lee et al
Takeda Euro Patent Office,
10 Charles II Street
London SW1Y 4AA (GB)

# (54) 1,5-BENZODIAZEPINE COMPOUNDS, PROCESS FOR PRODUCING THE SAME, AND MEDICINE

(57) A compound represented by the formula (I)

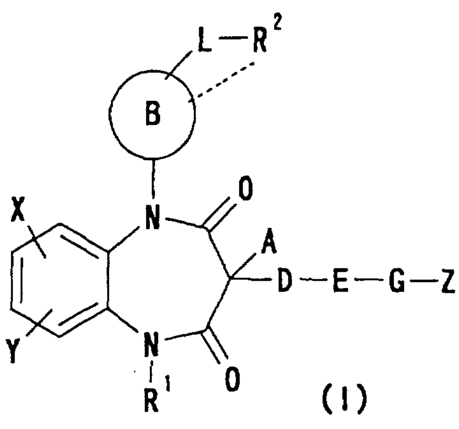

(I)

[wherein ring B represents a cyclic hydrocarbon group which may have substituent(s); Z represents hydrogen atom or a cyclic group which may have substituent(s); $R^1$ represents hydrogen atom, a hydrocarbon group which may have substituent(s), a heterocyclic group which may have substituent(s) or an acyl group; $R^2$ represents amino group which may have substituent(s); D represents a bond or a divalent group; E represents a bond, -CO-, -CON($R^a$)-, -COO-, -N($R^a$)CON($R^b$)-, -N($R^a$)COO-, -N($R^a$)$SO_2$-, -N($R^a$)-, -O-, -S-, -SO- or -$SO_2$- ($R^a$ and $R^b$ each independently represents hydrogen atom or a hydrocarbon group which may have substituent (s)); G represents a bond or a divalent group; L represents a bond or a divalent group; A represents hydrogen atom or a substituent; X and Y each represents hydrogen atom or an independent substituent; and ..... represents that $R^2$ and an atom on ring B may form a ring] or a salt thereof, and a process for producing the same.

EP 1 123 928 A1

## Description

Technical Field

The present invention relates to a novel condensed cyclic compound having a regulating action of a somatostatin receptor, a process for producing the same, and a medicament comprising the same.

Background of Art

Somatostatin was isolated from ovine hypothalamic tissue as a peptide (SST-14) composed of 14 amino acids having an inhibitory action on the secretion of growth hormone. At present, another somatostatin composed of 28 amino acids (SST-28) has also been isolated and identified. The somatostatins are cerebral/intestinal peptides widely distributed over not only the hypothalamus but also the cerebrum, cerebral limbic system, spinal cord, vagus nerve, autonomic ganglia, muscous membrane of digestive tract, pancreatic Langerhans' islets, etc., and inhibit the secretion of growth hormone, thyroid-stimulating hormones, pituitary/gastrointestinal hormones such as gastrin, insulin, glucagon, etc. In addition, they also inhibit gastric-acid secretion, external secretion at the pancreas, and movement/blood flow of the digestive tract.

Hitherto, as Somatostatin receptors, 1-type to 5-type (SSTR1, SSTR2, SSTR3, SSTR4, SSTR5) have been known, and each of them has been found to exhibit different expression at each central and peripheral part.

[1. Life Science, Vol. 57, No. 13, p. 1249, 1995
2. Journal of Clinical Endocrinology and Metabolism, Vol.80, No.6, pp. 1789-1793
3. The New England Journal of Medicine, Jan. 25, 1996
4. Eur J Clin Pharmacol, 1996, 51, 139-144
5. Exp. Opin. Ther. Patents (1998) 8(7): 855-870].

Currently, peptide-type somatostatin analogs which inhibit specific hormone secretion have been developed clinically.

Disclosure of the Invention

At present, the compounds which are developed as somatostatin receptor function regulators are peptide compounds and have many problems in terms of time of action, method of administration, specificity, adverse side effects and the like. For solving these problems, it is very important to devise and develop a non-peptide compound having an excellent regulating action on somatostatin receptor function.

As a result of extensive studies in consideration of the above circumstances, the present inventors have synthesized a compound having a structural characteristic that an amino group is bonded, directly or through a divalent group, to the cyclic hydrocarbon B in the following formula (I), which is represented by the formula (I):

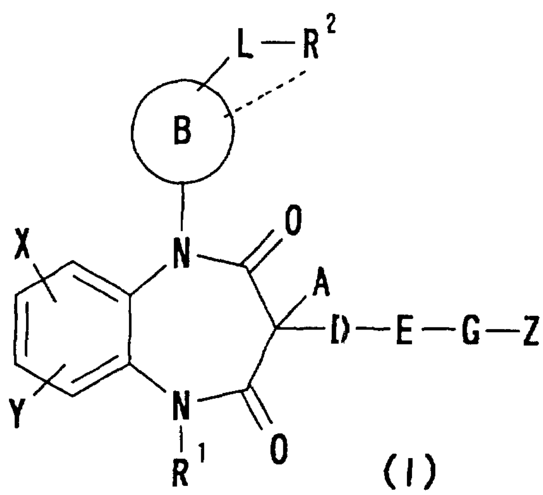

(I)

[wherein ring B represents a cyclic hydrocarbon group which may have substituent(s); Z represents hydrogen atom or

a cyclic group which may have substituent(s); $R^1$ represents hydrogen atom, a hydrocarbon group which may have substituent(s), a heterocyclic group which may have substituent(s) or an acyl group; $R^2$ represents an amino group which may have substituent(s); D represents a bond or a divalent group; E represents a bond, -CO-, -CON($R^a$)-, -COO-, -N($R^a$)CON($R^b$)-, -N($R^a$)COO-, -N($R^a$)$SO_2$-, -N($R^a$)-, -O-, -S-, -SO- or -$SO_2$- ($R^a$ and $R^b$ each independently represents hydrogen atom or a hydrocarbon group which may have substituent(s)); G represents a bond or a divalent group; L represents a bond or a divalent group; A represents hydrogen atom or a substituent; X and Y each represents hydrogen atom or an independent substituent; and ..... represents that $R^2$ and an atom on ring B may form a ring] or a salt thereof, and have found that the compound has excellent properties such as an excellent regulating action of somatostatin receptor function and a low toxicity owing to the unique chemical structure. Based on these findings, they have accomplished the invention.

Namely, the invention relates to:

(1) a compound represented by the above formula (I) or a salt thereof,
(2) the compound according to the above (1), wherein E is -CO-, -CON($R^a$)-, -COO-, -N($R^a$)CON($R^b$)-, -N($R^a$)COO-, - N($R^a$)$SO_2$-, -N($R^a$)-, -O-, -S-, -SO- or -$SO_2$- ($R^a$ and $R^b$ each independently represents hydrogen atom or a hydrocarbon group which may have substituent(s)),
(3) the compound according to the above (1), wherein L is [1] a bond or,
[2] a divalent hydrocarbon group which may contain -O- or -S- and may possess 1 to 5 substituents selected from

i) a $C_{1-6}$ alkyl group,
ii) a halogeno-$C_{1-6}$ alkyl group,
iii) phenyl group,
iv) benzyl group,
v) amino group which may have substituent(s),
vi) hydroxy group which may have substituent(s), and
vii) carbamoyl groups or thiocarbamoyl groups which each may be substituted by:

a) a $C_{1-6}$ alkyl group,
b) a phenyl group which may have substituent(s), or
c) a heterocyclic group which may have substituent(s),

(4) the compound according to the above (1), wherein Z is a cyclic group which may have substituent(s),
(5) the compound according to the above (1), wherein D is a divalent group bonded to the ring through a carbon atom,
(6) the compound according to the above (1), wherein ring B is benzene ring which may have substituent(s) and L is a $C_{1-6}$ alkylene group,
(7) the compound according to the above (1), wherein G represents a divalent hydrocarbon group which may have substituent(s) and ring B does not form a ring together with $R^2$,
(8) the compound according to the above (1), wherein A is hydrogen atom, ring B is benzene ring, Z is a phenyl group substituted by a halogen, and $R^1$ is a $C_{1-6}$ alkyl or $C_{7-14}$ aralkyl group which each may substituted by substituent(s) selected from (1) hydroxy, (2) phenyl, (3) a $C_{1-6}$ alkyl group-carbonyl or a $C_{6-14}$ aryl-carbonyl, and (4) amino groups which may be substituted by a $C_{1-6}$ alkyl group-sulfonyl or a $C_{6-14}$ aryl-sulfonyl,
(9) the compound according to the above (1), wherein X and Y each independently is hydrogen atom, a halogen, hydroxy, a $C_{1-6}$ alkoxy, a halogeno-$C_{1-6}$ alkoxy, a $C_{7-14}$ aralkyloxy, a benzoyl-$C_{1-6}$ alkoxy, a hydroxy-$C_{1-6}$ alkoxy, a $C_{1-6}$ alkoxy-carbonyl-$C_{1-6}$ alkoxy, a $C_{3-14}$ cycloalkyl-$C_{1-6}$ alkoxy, an imidazol-1-yl-$C_{1-6}$ alkoxy, a $C_{7-14}$ aralkyloxy-carbonyl-$C_{1-6}$ alkoxy, or a hydroxyphenyl-$C_{1-6}$ alkoxy;

ring B is benzene ring which may be substituted by a $C_{1-6}$ alkoxy, or tetrahydroisoquinoline ring or isoindoline ring which is formed by combination with $R^2$;
Z is a $C_{6-14}$ aryl group, a $C_{3-10}$ cycloalkyl group, piperidyl group, thienyl group, furyl group, pyridyl group, thiazolyl group, indanyl group or indolyl group which may have 1 to 3 substituents selected from a halogen, formyl, a halogeno-$C_{1-6}$ alkyl, a $C_{1-6}$ alkoxy, a $C_{1-6}$ alkyl-carbonyl, oxo and pyrrolidinyl;
A is hydrogen atom;
D is a $C_{1-6}$ alkylene group;
G is a bond, or a $C_{1-6}$ alkylene group which may contain phenylene and may be substituted by phenyl;
$R^1$ is hydrogen atom, a $C_{1-6}$ alkyl group, a $C_{2-6}$ alkenyl group, a $C_{6-14}$ aryl group or a $C_{7-14}$ aralkyl group which each may be substituted by substituent(s) selected from (1) a halogen, (2) nitro, (3) amino which may have 1 or 2 substituents selected from a $C_{1-6}$ alkyl which may be substituted by a $C_{1-6}$ alkyl-carbonyl, benzoyloxycar-

bonyl and a $C_{1-6}$ alkylsulfonyl, (4) hydroxy which may be substituted by (i) a $C_{1-6}$ alkyl which may be substituted by hydroxy, a $C_{1-6}$ alkyl-carbonyl, carboxy or a $C_{1-6}$ alkoxy-carbonyl, (ii) phenyl which may be substituted by hydroxy, (iii) benzoyl or (iv) a mono- or di- $C_{1-6}$ alkylamino-carbonyl, (5) a $C_{3-6}$ cycloalkyl, (6) phenyl which may be substituted by hydroxy or a halogeno-$C_{1-6}$ alkyl and (7) thienyl, furyl, thiazolyl, indolyl or benzyloxy-carbonylpiperidyl;

$R^2$ is (1) unsubstituted amino group, (2) piperidyl group or (3) amino which may have 1 or 2 substituents selected from (i) benzyl, (ii) a $C_{1-6}$ alkyl which may be substituted by amino or phenyl, (iii) a mono- or di-$C_{1-6}$ alkyl-carbamoyl, or a mono- or di-$C_{1-6}$ alkyl-thiocarbamoyl, (iv) a $C_{1-6}$ alkoxy-carbonyl, (v) a $C_{1-6}$ alkyl-sulfonyl, (vi) piperidylcarbonyl and (vii) a $C_{1-6}$ alkyl-carbonyl which may be substituted by a halogen or amino;

E is a bond, -CON($R^a$)-, -N($R^a$)CO-, -N($R^a$)CON($R^b$)- ($R^a$ and $R^b$ each represents hydrogen atom or a $C_{1-6}$ alkyl group);

L is a $C_{1-6}$ alkylene group which may contain -O- and may be substituted by a $C_{1-6}$ alkyl,

(10) the compound according to the above (1), wherein X and Y each independently is hydrogen atom, a halogen, hydroxy or a $C_{1-6}$ alkoxy;

ring B is benzene ring or, by combination with $R^2$, tetrahydroisoquinoline ring or isoindoline ring;

Z is phenyl group which may be substituted by a halogen, D is a $C_{1-6}$ alkylene group, G is a $C_{1-6}$ alkylene group;

$R^1$ is a $C_{1-6}$ alkyl group or a $C_{7-14}$ aralkyl group which each may be substituted by substituent(s) selected from (1) hydroxy, (2) phenyl and (3) amino which may be substituted by a $C_{1-6}$ alkyl-carbonyl or a $C_{1-6}$ alkylsulfonyl;

$R^2$ is unsubstituted amino group;

E is -CONH-;

L is a $C_{1-6}$ alkylene group,

(11) a prodrug of the compound according to the above (1) or a salt thereof,

(12) a process for producing a compound of the formula (I-a)

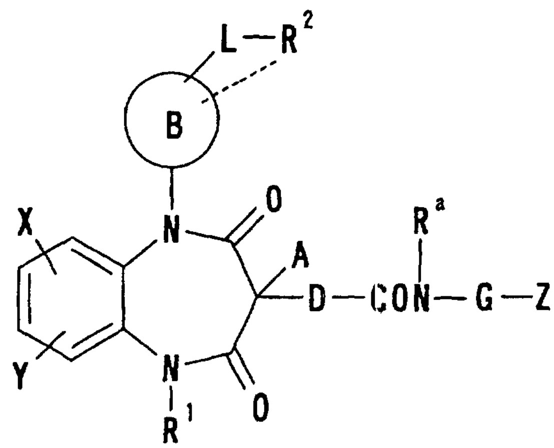

(I-a)

[wherein the symbols have the same meanings as described above] or a salt thereof which comprises:

reacting a compound represented by the formula (IIa)

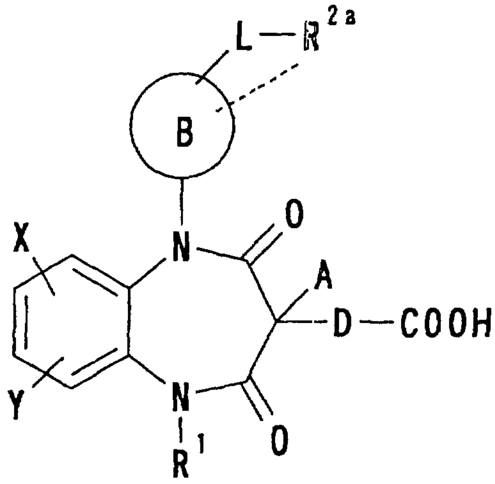

(IIa)

[wherein $R^{2a}$ represents amino group which may be protected and substituted, and other symbols have the same meanings as described in the above (1)], a reactive derivative thereof or a salt thereof, with a compound represented by the formula (III)

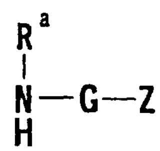

(III)

[wherein the symbols have the same meanings as described in the above (1)] or a salt thereof to produce a compound of the formula (Ia-a)

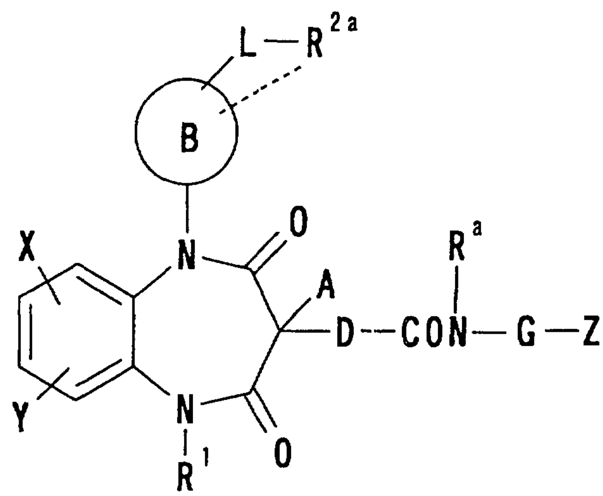

(Ia-a)

[wherein the symbols have the same meanings as described above] or a salt thereof, and optionally, subjecting it to de-protecting reaction,

(13) a pharmaceutical composition which comprises a compound according to the above (1) or a salt thereof,

(14) a pharmaceutical composition according to the above (13) which is a somatostatin receptor function regulator,
(15) a pharmaceutical composition according to the above (14) wherein the somatostatin receptor function regulator is a somatostatin receptor agonist,
(16) a pharmaceutical composition according to the above (13) which is an agent for preventing or treating diabetes, obesity, diabetic complications or intractable diarrhea,
(17) a method for regulating a somatostatin receptor function which comprises:

administering a compound represented by the formula (I)

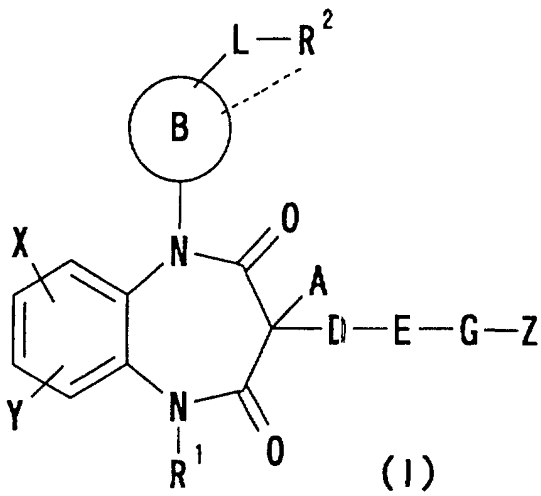

[wherein ring B represents a cyclic hydrocarbon group which may have substituent(s); Z represents hydrogen atom or a cyclic group which may have substituent(s); $R^1$ represents hydrogen atom, a hydrocarbon group which may have substituent(s), a heterocyclic group which may have substituent(s) or an acyl group; $R^2$ represents amino group which may have substituent(s); D represents a bond or a divalent group; E represents a bond, -CO-, -CON($R^a$)-, -COO-, -N($R^a$)CON($R^b$)-. -N($R^a$)COO-, -N($R^a$)$SO_2$-. -N($R^a$)-, -O-, -S-, -SO- or -$SO_2$- ($R^a$ and $R^b$ each independently represents hydrogen atom or a hydrocarbon group which may have substituent(s)); G represents a bond or a divalent group; L represents a bond or a divalent group; A represents hydrogen atom or a substituent; X and Y each represents hydrogen atom or an independent substituent; and ..... represents that $R^2$ and an atom on ring B may form a ring] or a salt thereof,
(18) use of a compound represented by the formula (I)

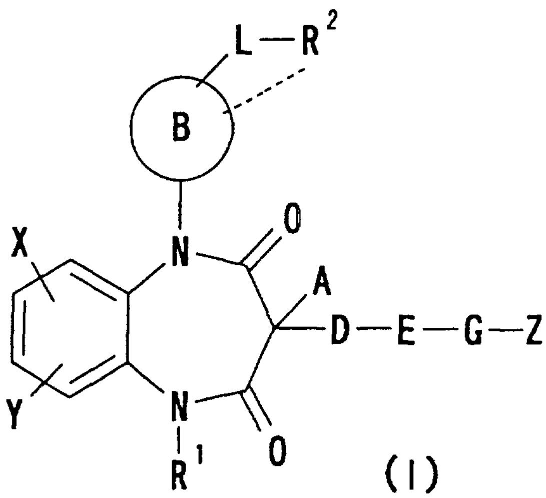

[wherein ring B represents a cyclic hydrocarbon group which may have substituent(s); Z represents hydrogen atom or a cyclic group which may have substituent(s); $R^1$ represents hydrogen atom, a hydrocarbon group which may have substituent(s), a heterocyclic group which may have substituent(s) or an acyl group; $R^2$ represents amino

group which may have substituent(s); D represents a bond or a divalent group; E represents a bond, -CO-, -CON($R^a$)-, -COO-, -N($R^a$)CON($R^b$)-, -N($R^a$)COO-, -N($R^a$)$SO_2$-, -N($R^a$)-, -O-, -S-, -SO- or -$SO_2$- ($R^a$ and $R^b$ each independently represents hydrogen atom or a hydrocarbon group which may have substituent(s)); G represents a bond or a divalent group; L represents a bond or a divalent group; A represents hydrogen atom or a substituent; X and Y each represents hydrogen atom or an independent substituent; and ..... represents that $R^2$ and an atom on ring B may form a ring] or a salt thereof, for manufacturing a medicament for regulating a somatostatin receptor function.

Best Mode for Carrying Out the Invention

In the above formula, ring B shows a cyclic hydrocarbon group which may have substituent(s). Ring B is preferably an aromatic hydrocarbon group which may have substituent(s), and particularly preferred is a phenyl group which may have substituent(s).

The cyclic hydrocarbon group represented by ring B includes, for example, alicyclic hydrocarbon groups composed of 3 to 14 carbon atoms, aromatic hydrocarbon groups composed of 6 to 14 carbon atoms, and the like. Examples of the above "alicyclic hydrocarbon group" include $C_{3-14}$ cycloalkyl groups (e.g., cyclopropyl, cyclobutyl, cyclopentyl, cyclohexyl, etc.), $C_{3-14}$ cycloalkenyl groups (e.g., cyclopentenyl, cyclohexenyl, etc.), $C_{5-14}$ cycloalkadienyl groups (e. g., 2,4-cyclopentadienyl, 1,3-cyclohexadienyl, etc.), indanyl group and the like. Among them, preferred are alicyclic hydrocarbon groups composed of 6 to 10 carbon atoms. Examples of the above "aromatic hydrocarbon group" include aromatic hydrocarbon groups composed of 6 to 14 carbon atoms (e.g., $C_{6-14}$ aryl groups such as phenyl, naphthyl, anthranyl, phenanthryl, etc.) and the like. Among them, preferred are aromatic hydrocarbon groups composed of 6 to 10 carbon atoms. Particularly preferred is phenyl group.

Examples of the substituent which ring B may have or the substituent of A include halogen atoms (e.g., fluorine, chlorine, bromine, iodine, etc.), $C_{1-6}$ alkyl groups (e.g., methyl, ethyl, propyl, butyl, sec-butyl, t-butyl, isopropyl, etc.), halogeno-$C_{1-6}$ alkyl groups (e.g., $C_{1-6}$ alkyl groups substituted by 1 to 5 of said "halogen atom" and the like; e.g., trifluoromethyl, etc.), phenyl group, benzyl group, $C_{1-6}$ alkoxy groups (e.g., methoxy, ethoxy, propoxy, butoxy, sec-butoxy, t-butoxy, isopropoxy, etc.), halogeno-$C_{1-6}$ alkoxy groups (e.g., $C_{1-6}$ alkoxy groups substituted by 1 to 5 of said "halogen atom"; trifluoromethoxy, chloropropyloxy, etc.), phenoxy group, $C_{7-14}$ aralkyloxy groups (e.g., benzyloxy, phenethyloxy, phenylpropyloxy, etc.), formyloxy group, $C_{1-6}$ alkyl-carbonyloxy groups (e.g., acetyloxy, etc.), $C_{1-6}$ alkylthio groups (e.g., methylthio, ethylthio, propylthio, butylthio, sec-butylthio, t-butylthio, isopropylthio, etc.), halogeno-$C_{1-6}$ alkylthio groups (e.g., $C_{1-6}$ alkylthio groups substituted by 1 to 5 of said "halogen atom"; e.g., trifluoromethylthio, etc.), hydroxy group, mercapto group, cyano group, nitro group, carboxyl group, formyl group, $C_{1-6}$ alkyl-carbonyl groups (e. g., acetyl, propionyl, etc.), benzoyl group, $C_{1-6}$ alkoxy-carbonyl groups (e.g., methoxycarbonyl, ethoxycarbonyl, propoxycarbonyl, etc.), phenoxycarbonyl group, amino group, mono- or di-$C_{1-6}$ alkylamino groups (e.g., methylamino, ethylamino, dimethylamino, diethylamino, etc.), formylamino group, $C_{1-6}$ alkyl-carbonylamino groups (e.g., acetylamino, propionylamino, butyrylamino, etc.), carbamoyl group, thiocarbamoyl group, mono- or di-$C_{1-6}$ alkyl-carbamoyl groups (e.g., N-methylcarbamoyl, N-ethylcarbamoyl, N,N-dimethylcarbamoyl, N,N-diethylcarbamoyl, etc.), mono- or di-$C_{1-6}$ alkyl-thiocarbamoyl groups (e.g., N-methylthiocarbamoyl, N-ethylthiocarbamoyl, N,N-dimethylthiocarbamoyl, N,N-diethylthiocarbamoyl, etc.), sulfo group, $C_{1-6}$ alkylsulfonyl groups (e.g., methylsulfonyl, ethylsulfonyl, propylsulfonyl, etc.), benzoyl-$C_{1-6}$ alkoxy groups (e.g., benzoylmethyloxy, etc.), hydroxy-$C_{1-6}$ alkoxy groups (e.g., hydroxyethyloxy, etc.), $C_{1-6}$ alkoxy-carbonyl-$C_{1-6}$ alkoxy groups (e.g., methoxycarbonylmethyloxy, etc.), $C_{3-14}$ cycloalkyl-$C_{1-6}$ alkoxy groups (e.g., cyclohexylmethyloxy, etc.), imidazol-1-yl- $C_{1-6}$ alkoxy groups (e.g., imidazol-1-ylpropyloxy, etc.), $C_{7-14}$ aralkyloxy-carbonyl-$C_{1-6}$ alkoxy groups (e.g., benzyloxycarbonylmethyloxy, etc.), hydroxyphenyl-$C_{1-6}$ alkoxy groups (e.g., [3-(4-hydroxyphenyl)propyl]oxy, etc.), $C_{7-14}$ aralkyloxy-carbonyl groups (e.g., benzyloxycarbonyl, etc.), mono- or di-$C_{1-6}$ alkylamino-$C_{1-6}$ alkoxy (e.g., methylaminomethoxy, ethylaminoethoxy, dimethylaminomethoxy, etc.), mono- or di-$C_{1-6}$ alkylamino-carbonyloxy (e.g., methylaminocarbonyloxy, ethylaminocarbonyloxy, dimethylaminocarbonyloxy, etc.), and the like. The cyclic hydrocarbon group as ring B may have 1 to 4 substituents selected from these substituents. Furthermore, A is preferably hydrogen atom.

Particularly, ring B is preferably benzene ring, a cycloalkane, or the like which each may have substituent(s), especially more preferably benzene ring, a cyclohexane ring, or the like which may be substituted by a $C_{1-6}$ alkoxy (preferably methoxy, etc.), most preferably unsubstituted benzene ring or cyclohexane ring. $R^2$ and an atom on ring B may form a ring, and ring B may form a nitrogen-containing heterocycle which may have substituent(s), for example, by bonding, to amino group represented by $R^2$ or a substituent of the amino group, an atom forming ring B which is adjacent to the atom forming ring B to which L is bonded. In the case that such nitrogen-containing heterocycle is formed, the nitrogen atom of the amino group of $R^2$ may be bonded to the atom forming ring B directly or through a spacer. The spacer means part or whole of the substituent of the amino group of $R^2$.

The "nitrogen-containing heterocycle which may have substituent(s)" formed together with ring B includes, for example, bicyclic condensed nitrogen-containing heterocycles (preferably, bicyclic non-aromatic condensed nitrogen-containing heterocycles) which are formed by condensing a cyclic hydrocarbon which may have substituent(s)

represented by ring B (e.g., benzene ring, etc.) and a 5 or 6-membered monocyclic hetero cycle (preferably, a monocyclic non-aromatic heterocycle) having at least one nitrogen atom and optionally further one or two hetero atoms selected from nitrogen, oxygen and sulfur atoms. Concretely, the examples to be used include tetrahydroisoquinoline (e.g., 1,2,3,4-tetrahydroisoquinoline), tetrahydroquinoline (e.g., 1,2,3,4-tetrahydroquinoline), isoindoline, indoline, 2,3-dihydrobenzothiazole, 2,3-dihydrobenzoxazole, 3,4-dihydro-2H-1,4-benzothiazine, 3,4-dihydro-2H-1,4-benzoxazine, 1,2,3,4-tetrahydroquinoxaline, 2,3,4,5-tetrahydro-1,4-benzoxazepine and the like, and particularly preferred are tetrahydroisoquinoline or isoindoline.

The substituent which the "nitrogen-containing heterocycle which may have substituent(s)" formed together with ring B may have, includes, for example, those similar to the substituents which the above "cyclic hydrocarbon" for ring B may have, and the like. The "nitrogen-containing heterocycle which may have substituent(s)" may have 1 to 4 substituents selected from these substituents.

In the above formula, Z represents hydrogen atom or a cyclic group which may have substituent(s) (preferably, a cyclic group which may have substituent(s)). The "cyclic group" represented by Z include, for example, cyclic hydrocarbon groups, heterocylic groups, and the like. Z is preferably, for example, aromatic hydrocarbon groups which may have substituent(s), aromatic heterocyclic groups which may have substituent(s) and the like, and particularly preferred are phenyl groups which may have substituent(s) and the like.

The "cyclic hydrocarbon group" of Z includes, for example, alicyclic hydrocarbon groups composed of 3 to 14 carbon atoms, aromatic hydrocarbon groups composed of 6 to 14 carbon atoms, or the like. Examples of said "alicyclic hydrocarbon groups" include $C_{3-14}$ cycloalkyl groups (e.g., cyclopropyl, cyclobutyl, cyclopentyl, cyclohexyl, etc.), $C_{3-14}$ cycloalkenyl groups (e.g., cyclopentenyl, cyclohexenyl, etc.), $C_{5-14}$ cycloalkadienyl groups (e.g., 2,4-cyclopentadienyl, 1,3-cyclohexadienyl, etc.), indanyl group and the like. Among them, preferred are alicyclic hydrocarbon groups having 6 to 10 carbon atoms. Examples of said "aromatic hydrocarbon groups" include $C_{6-14}$ aryl groups (e.g., phenyl, naphthyl, anthranyl, phenanthryl, etc.) and the like. Among them, preferred are aromatic hydrocarbon groups having 6 to 10 carbon atoms.

The "heterocyclic groups" of Z includes, for example, monocyclic heterocyclic groups, polycyclic heterocyclic groups, and the like. Said "monocyclic heterocyclic groups" include, for example, 5 or 6-membered monocyclic heterocyclic groups having 1 to 4 hetero atoms selected from nitrogen, oxygen and sulfur atoms other than carbon atom (s). Concretely, there may be used, for example, monocyclic aromatic heterocyclic groups (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, furazanyl, 1,2,3-thiadiazolyl, 1,2,4-thiadiazolyl, 1,3,4-thiadiazolyl, 1,2,3-triazolyl, 1,2,4-,triazolyl, tetrazolyl, pyridyl, pyridadinyl, pyrimidinyl, triazinyl, etc.), monocyclic non-aromatic heterocyclic groups (e.g., oxiranyl, azetizinyl, oxetanyl, thietanyl, pyrrolidinyl, tetrahydrofuryl, thiolanyl, piperidyl, tetrahydropyranyl, morpholinyl, thiomorpholinyl, piperadinyl, etc.) and the like. The above "polycyclic condensed heterocyclic groups" include, for example, bi- or tricyclic aromatic condensed heterocyclic groups which are formed by condensing 2 or 3 of the above "monocyclic aromatic heterocyclic rings", bior tricyclic aromatic condensed heterocyclic groups which are formed by condensing 1 or 2 of the above "monocyclic aromatic heterocyclic rings" and benzene ring (preferably, the bi- or tricyclic aromatic condensed heterocyclic groups which are formed by condensing 1 or 2 of the above "monocyclic aromatic heterocyclic rings" and benzene ring) and partially reduced rings thereof. Concretely, there may be used polycyclic aromatic condensed heterocyclic groups (e. g., benzofuryl, isobenzofuryl, benzo[b]thienyl, indanyl, indolyl, isoindolyl, 1H-indazolyl, benzimidazolyl, benzoxanzolyl, 1,2-benzisoxazolyl, benzothiazolyl, 1,2-benzisothiazolyl, 1H-benzotriazo]yl, quinolyl, isoquinolyl, cinnolyl, quinazolinyl, quinoxalinyl, phthalazinyl, naphthylidinyl, purinyl, pteridinyl, carbazolyl, α-carbolinyl, β-carbolinyl, γ-carbolinyl, acrydinyl, phenoxazinyl, phenothiazinyl, phenazinyl, phenoxathinyl, thianthrenyl, phenantridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-a]pyridazinyl, imidazo[1,2-a]pyrimidinyl, 1,2,4-triazolo[4,3-a]pyridyl, 1,2,4-triazolo[4,3-b]pyridazinyl, etc.), polycyclic non-aromatic condensed heterocycles (e.g., isochromanyl, chromanyl, indolyl, isoindolyl, 1,2,3,4-tetrahydroisoqunolyl, 1,2,3,4-tetrahydroqunolyl, etc.) and the like.

The substituent which the cyclic group represented by Z may have, includes, for example, oxo group, thioxo group and those similar to the substituents which said "cyclic hydrocarbon" for above ring B may have. The "cyclic group" of Z may have 1 to 5 substituents selected from these substituents.

Z is preferably a $C_{6-14}$ aryl group (preferably, phenyl group, etc.), a $C_{3-10}$ cycloalkyl group, piperidyl group, thienyl group, furyl group, pyridyl group, thiazolyl group, indanyl group, indolyl group and the like which may have 1 to 3 substituents selected from halogens, formyl, halogeno-$C_{1-6}$ alkyl, $C_{1-6}$ alkoxy, $C_{1-6}$ alkyl-carbonyl, oxo and pyrrolidinyl. Among them, preferred are a phenyl group substituted by halogen (preferably, fluorine, etc.) and the like.

The substituting position of the substituent in the cyclic group represented by Z is preferably ortho position in the case that Z is phenyl group, and the number of the substituent is preferably one.

In the above formula, D represents a bond or a divalent group. The divalent group may have, for example, substituent(s) and may contain -O-, -S-, or -N($R^a$)- ($R^a$ represents hydrogen atom or a hydrocarbon group which may have substituent(s)). Among them, the divalent group bonded through a carbon atom is preferred, and particularly

preferred is a divalent hydrocarbon group which may have substituent(s).

The divalent group represented by D to be used includes, for example, linear divalent hydrocarbon groups having 1 to 10 carbon atoms. Concretely, the examples include $C_{1-10}$ alkylene groups (e.g., methylene, ethylene, propylene, butylene, pentamethylene, hexamethylene, heptamethylene, octamethylene, etc.), and particularly preferred are $C_{1-6}$ alkylene groups (e.g., methylene, ethylene, propylene, butylene, pentamethylene, hexamethylene, etc.) and the like. The above "divalent group" may contain, at any position, $C_{3-6}$ cycloalkylenes (e.g., 1,4-cyclohexylene, etc.), phenylenes (e.g., 1,4-phenylene, 1,2-phenylene, etc.) and the like.

The substituents for the divalent group represented by D include, for example, $C_{1-6}$ alkyl groups (e.g., methyl, ethyl, propyl, isopropyl, etc.), halogeno-$C_{1-6}$ alkyl groups (e.g., $C_{1-6}$ alkyl groups substituted by 1 to 5 of said "halogen atoms"; e.g., trifluoromethyl, etc.), phenyl group, benzyl group, amino group which may have substituent(s), hydroxy group which may have substituent(s), carbamoyl group which may have substituent(s), and thiocarbamoyl group which may have substituent(s). The above "divalent group" may have 1 to 3 of these substituents.

Among them, D is preferably a $C_{1-6}$ alkylene group (e.g., methylene, ethylene, propylene, etc., preferably methylene, etc.).

In the above formula, G represents a bond or a divalent group. As the "divalent group" represented by G, there may be used those similar to the above "divalent group" represented by D, for example.

G is, for example, preferably a bond or a $C_{1-6}$ alkylene group which may contain a phenylene and which may be substituted by phenyl. For example, $C_{1-6}$ alkylene groups (e.g., methylene, ethylene, propylene, etc.) are preferably used. For the $C_{1-6}$ alkylene group represented by G, phenylene may be present between the $C_{1-6}$ alkylene group and E or Z, or phenylene may be contained in the $C_{1-6}$ alkylene group.

In the above formula, $R^1$ represents hydrogen atom, a hydrocarbon group which may have substituent(s), a heterocyclic group which may have substituent(s) or an acyl group. $R^1$ is preferably a hydrocarbon group which may have substituent(s) or an acyl group.

The hydrocarbon group represented by $R^1$ includes, for example, aliphatic hydrocarbon groups, alicyclic hydrocarbon groups, aryl groups, aralkyl groups and the like, and particularly preferred are aliphatic hydrocarbon groups and the like.

The aliphatic hydrocarbon groups for $R^1$ include aliphatic hydrocarbon groups having 1 to 10 carbon atoms (e.g., $C_{1-10}$ alkyl groups, $C_{2-10}$ alkenyl groups, $C_{2-10}$ alkynyl groups, etc.). Examples of said "$C_{1-10}$ alkyl groups" include methyl, ethyl, propyl, isopropyl, butyl, isobutyl, sec-butyl, t-butyl, pentyl, isopentyl, neopentyl, 1-methylpropyl, hexyl, isohexyl, 1,1-dimethylbutyl, 2,2-dimethylbutyl, 3,3-dimethylbutyl, 3,3-dimethylpropyl, 2-ethylbutyl, heptyl and the like, preferably $C_{3-5}$ alkyl groups (e.g., propyl, isopropyl, isobutyl, neopentyl, etc.), and particularly preferred are isobutyl, neopentyl and the like. Examples of the above "$C_{2-10}$ alkenyl groups" include vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, and the like, and particularly preferred are $C_{2-6}$ alkenyl groups (e.g., vinyl, allyl, isopropenyl, 2-methylallyl, 2-methyl-1-propenyl, 2-methyl-2-propenyl, 3-methyl-2-butenyl, etc.). Examples of the above "$C_{2-10}$ alkynyl groups" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, and the like, and particularly preferred are $C_{2-6}$ alkynyl groups (e.g., ethynyl, 1-propynyl, 2-propynyl, etc.).

The alicyclic hydrocarbon groups for $R^1$ include, for example, alicyclic hydrocarbon groups having 3 to 10 carbon atoms (e.g., $C_{3-10}$ cycloalkyl groups, $C_{3-10}$ cycloalkenyl groups, $C_{5-10}$ cycloalkadienyl groups, etc..) and the like. Examples of the "$C_{3-10}$ cycloalkyl groups" include cyclopropyl, cyclobutyl, cyclopentyl, cyclohexyl, cycloheptyl, cyclooctyl, cyclononyl, and the like. Examples of the "$C_{3-10}$ cycloalkenyl groups" include 1-cyclobuten-1-yl, 1-cyclopenten-1-yl, 2-cyclopenten-1-yl, 3-cyclopenten-1-yl, 2-cyclohexen-1-yl, 3-cyclohexen-1-yl, and the like. Examples of the "$C_{5-10}$ cycloalkadienyl groups" include 2,4-cyclopentadien-1-yl, 2,5-cyclohexadien-1-yl, and the like.

The aryl groups for $R^1$ include, for example, $C_{6-14}$ aryl groups (e.g., phenyl, naphthyl, anthranyl, phenanthryl, acenaphthylenyl, etc.), and the like.

The aralkyl groups for $R^1$ include, for example, $C_{7-14}$ aralkyl groups (e.g., benzyl, phenethyl, 3-phenylpropyl, 4-phenylpropyl, 4-phenylbutyl, 2-naphthylmethyl, etc.), and the like.

The substituent which the hydrocarbon groups of $R^1$ may have, includes, for example, halogen atoms, nitro group, cyano group, imino group, amino group which may have substituent(s), hydroxy group which may have substituent(s), carboxyl group which may be esterified, carbamoyl group which may have substituent(s), thiocarbamoyl group which may have substituent(s), cycloalkyl groups, cycloalkenyl groups, heterocyclic groups which may have substituent(s), and the like. Among the "hydrocarbon groups", the groups containing an aromatic ring may further have alkyl group(s), halogenoalkyl group(s), and aryl group(s) which may have substituent(s) in addition to the above substituents. On the above "hydrocarbon groups" may be present 1 to 5 (preferably, 1 to 3) of these substituents.

The "halogen atoms" which are substituents of the "hydrocarbon groups" for $R^1$ includes, for example, fluorine, chlorine, bromine, iodine, and the like.

The "amino group which may have substituent(s)" which is a substituent of the "hydrocarbon groups" for $R^1$, D and G includes, for example, (1)amino group which may have 1 or 2 substituents selected from (i) $C_{1-6}$ alkyl groups (e.g., methyl, ethyl, propyl, isopropyl, trifluoromethyl, etc.), $C_{6-14}$ aryl groups (e.g., phenyl group, etc.), $C_{7-14}$ aralkyl groups (e.g., benzyl group, etc.) which may be substituted by 1 to 5 of the halogen atoms or $C_{1-6}$ alkoxy groups, (ii) formyl group, $C_{1-6}$ alkyl-carbonyl groups (e.g., acetyl, propionyl, butyryl, etc.), $C_{6-14}$ aryl-carbonyl groups (e.g., phenylcarbonyl, naphthylcarbonyl, anthranylcarbonyl, phenanthrylcarbonyl, acenaphthylenylcarbonyl, etc.), $C_{6-14}$ aryl-carbonyl groups (e.g., benzoyl, etc.), (iii) $C_{1-6}$ alkoxy-carbonyl groups (e.g., methoxycarbonyl, ethoxycarbonyl, propoxycarbonyl, sec-propoxycarbonyl, butoxycarbonyl, etc.), $C_{7-14}$ aralkyloxy-carbonyl groups (e.g., benzyloxycarbonyl group, etc.), (iv) sulfo group, $C_{1-6}$ alkyl-sulfonyl groups (e.g., methylsulfonyl, ethylsulfonyl, propylsulfonyl, sec-propylsulfonyl, butylsulfonyl, t-butylsulfonyl, etc.), $C_{6-14}$ aryl-sulfonyl groups (e.g., phenylsulfonyl, naphthylsulfonyl, anthranylsulfonyl, phenanthrylsulfonyl, acenaphthylenylsulfonyl, etc.), and (v) $C_{1-6}$ alkylamino-carbonyl groups (e.g., methylaminocarbonyl, ethylaminocarbonyl, propylaminocarbonyl, butylaminocarbonyl, dimetylaminocarbonyl, etc.) and the like, and (2) 5- or 6-membered cyclic amino groups which may have substituent(s) such as pyrrolidinyl group, piperidyl group, morpholinyl group, thiomorpholinyl group, 4-methylpiperidyl group, 4-phenylpiperidyl group and the like.

The substituent which the "hydroxy group which may have substituent", i.e., a substituent of the "hydrocarbon groups" for $R^1$, D and G, may have, includes, for example, (i) $C_{1-6}$ alkyl groups which may have substituent(s), (ii) $C_{6-10}$ aryl groups which may have substituent(s), (iii) $C_{7-14}$ aralkyl groups which may have substituent(s), and (iv) acyl groups, etc. Examples of the "$C_{1-6}$ alkyl groups" of the "$C_{1-6}$ alkyl groups which may have substituent(s)" include methyl, ethyl, propyl, isopropyl, butyl, pentyl, and the like. The "$C_{1-6}$ alkyl groups" may have 1 to 3 substituents selected from, for example, halogen atoms (e.g., fluorine, chlorine, bromine, iodine, etc.), hydroxy group, $C_{1-6}$ alkoxy groups (e.g., methoxy, ethoxy, propoxy, isopropoxy, etc.), formyl group, $C_{1-6}$ alkyl-carbonyl groups (e.g., acetyl, propionyl, butyryl, etc.), carboxyl group, $C_{1-6}$ alkoxy-carbonyl groups (e.g., methoxycarbonyl, ethoxycarbonyl, propoxycarbonyl, sec-propoxycarbonyl, butoxycarbonyl, etc.), amino group, mono- or di-$C_{1-6}$ alkylamino groups (e.g., methylamino, ethylamino, dimethylamino, diethylamino, etc.), pyrrolidyl group, piperidyl group, morpholinyl group, thiomorpholinyl group, 4-methylpiperidyl group, 4-phenylpiperidyl group, carbamoyl group, thiocarbamoyl group, mono- or di-$C_{1-6}$ alkyl-carbamoyl groups (e.g., N-methylcarbamoyl, N-ethylcarbamoyl, N,N-dimethylcarbamoyl, N,N-diethylcarbamoyl, etc.), mono- or di-$C_{1-6}$ alkyl-thiocarbamoyl groups (e.g., N-methylthiocarbamoyl, N-ethylthiocarbamoyl, N,N-dimethylthiocarbamoyl, N,N-diethylthiocarbamoyl, etc.), phenoxy group, mono- or di-$C_{1-6}$ alkyl-carbamoyloxy groups (e.g., N-methylcarbamoyloxy, N-ethylcarbamoyloxy, N,N-dimethylcarbamoyloxy, N,N-diethylcarbamoyloxy, etc.), mono- or di-$C_{1-6}$ alkylthiocarbamoyloxy groups (e.g., N-methylthiocarbamoyloxy, N-ethylthiocarbamoyloxy, N,N-dimethylthiocarbamoyloxy, N,N-diethylthiocarbamoyloxy, etc.), formylamino group, $C_{1-6}$ alkyl-carbonylamino groups (e,g., acetylamino, propionylamino, butyrylamino, etc.), formyloxy group, and $C_{1-6}$ alkyl-carbonyloxy groups (e.g., acetoxy, etc.), and the like.

The "$C_{6-10}$ aryl groups" of the above "$C_{6-10}$ aryl groups which may have substituent(s)" include, for example, phenyl, naphthyl, and the like. The "$C_{6-10}$ aryl groups" may have 1 to 5 substituents selected from $C_{1-6}$ alkyl groups (e.g., methyl, ethyl, propyl, isopropyl, etc.) and halogeno-$C_{1-6}$ alkyl groups (e.g., $C_{1-6}$ alkyl groups substituted by 1 to 5 of the "halogen atoms" such as trifluoromethyl, etc.), and the like, in addition to the substituents which the above "$C_{1-6}$ alkyl groups" may have. The "$C_{7-14}$ aralkyl groups" of the above "$C_{7-14}$ aralkyl groups which may have substituent (s)" include, for example, benzyl, phenethyl, and the like. The substituents which the "$C_{7-14}$ aralkyl groups" may have, may be those similar to the substituents which the "$C_{6-10}$ aryl groups" may have, and the number of substituent is from 1 to 5. Examples of the "acyl groups" include formyl group, $C_{1-6}$ alkyl-carbonyl groups (e.g., acetyl, propionyl, butyryl, t-butylcarbonyl, etc.), benzoyl group, $C_{1-6}$ alkoxy-carbonyl groups (e.g., methoxycarbonyl, ethoxycarbonyl, propoxycarbonyl, sec-propoxycarbonyl, butoxycarbonyl, t-butoxycarbonyl, etc.), benzyloxycarbonyl group, $C_{1-6}$ alkylsulfonyl groups (e.g., methylsulfonyl, ethylsulfonyl, propylsulfonyl, sec-propylsulfonyl, butylsulfonyl, t-butylsulfonyl, etc.), carbamoyl group, thiocarbamoyl group, mono- or di-$C_{1-6}$ alkyl-carbamoyl groups (e.g., N-methylcarbamoyl, N-ethylcarbamoyl, N,N-dimethylcarbamoyl, N,N-diethylcarbamoyl, etc.), mono- or di-$C_{1-6}$ alkyl-thiocarbamoyl groups (e.g., N-methylthiocarbamoyl, N-ethylthiocarbamoyl, N,N-dimethylthiocarbamoyl, N,N-diethylthiocarbamoyl, etc.), and the like. These may further have 1 to 3 substituents selected from, for example, halogen atoms (e.g., fluorine, chlorine, bromine, iodine, etc.), hydroxy group, $C_{1-6}$ alkoxy groups (e.g., methoxy, ethoxy, propoxy, isopropoxy, etc.), formyl group, $C_{1-6}$ alkyl-carbonyl groups (e.g., acetyl, propionyl, butyryl, etc.), carboxyl group, $C_{1-6}$ alkoxy-carbonyl groups (e.g., methoxycarbonyl, ethoxycarbonyl, propoxycarbonyl, sec-propoxycarbonyl, butoxycarbonyl, etc.), amino group, mono- or di-$C_{1-6}$ alkylamino groups (e.g., methylamino, ethylamino, dimethylamino, diethylamino, etc.), pyrrolidyl group, piperidyl group, morpholinyl group, thiomorpholinyl group, 4-methylpiperidyl group, 4-phenylpiperidyl group, 4-benzyloxycarbonylpiperidyl group, carbamoyl group, thiocarbamoyl group, mono- or di-$C_{1-6}$ alkyl-carbamoyl groups (e.g., methylcarbamoyl, ethylcarbamoyl, dimethylcarbamoyl, diethylcarbamoyl, etc.), mono- or di-$C_{1-6}$ alkyl-thiocarbamoyl groups (e.g., methylthiocarbamoyl, ethylthiocarbamoyl, dimethylthiocarbamoyl, diethylthiocarbamoyl, etc.), phenoxy group, mono- or di-$C_{1-6}$ alkyl-carbamoyloxy groups (e.g., methylcarbamoyloxy, ethylcarbamoyloxy, dimethylcarbamoyloxy, diethylcarbamoyloxy, etc.), mono- or di-$C_{1-6}$ alkyl-thiocarbamoyloxy groups (e.g., methylthiocarbamoyloxy, ethylthiocarbamoyloxy, dimethylthiocarbamoyloxy, diethylthiocarbamoyloxy, etc.), formylamino group, $C_{1-6}$ alkyl-carbonylamino

groups (e.g., acetylamino, propionylamino, butyrylamino, etc.), formyloxy group, and $C_{1-6}$ alkoxy-carbonyloxy groups (e.g., acetoxy, etc.), etc.

The above "carboxyl group which may be esterified" which is a substituent of the "hydrocarbon group" for $R^1$ includes, for example, groups represented by the formula -COOR$^c$ (wherein R$^c$ represents hydrogen atom, a $C_{1-6}$ alkyl group (e.g., methyl, ethyl, propyl, isopropyl, butyl, t-butyl, etc.), benzyl group, etc.), and the like.

The substituent which the above "carbamoyl groups which may have substituent(s)", i.e., a substituent of the "hydrocarbon group" for $R^1$, D and G, may have, includes, for example, $C_{1-6}$ alkyl groups (e.g., methyl, ethyl, propyl, isopropyl, butyl, t-butyl, etc.), benzyl group, phenyl group which may have substituent(s) (e.g., phenyl group which may have substituent(s) similar to the substituent which the "aryl group which may have substituent(s)", i.e., a substituent of the "hydrocarbon group" for $R^1$, may have, and the like), heterocyclic groups which may have substituent(s) (e.g., heterocyclic groups which may have substituent(s) similar to the substituent which the "heterocyclic groups which may have substituent(s)", i.e., a substituent of the "hydrocarbon group" for $R^1$, may have, and the like), and the like.

The substituent which the above "thiocarbamoyl groups which may have substituent(s)" which are substituents of the "hydrocarbon group" for $R^1$, D and G may have, includes substituents similar to those of the above "carbamoyl groups which may have substituent(s)" may have.

The above "cycloalkyl group" which is a substituent of the "hydrocarbon group" for $R^1$ includes, for example, $C_{3-6}$ cycloalkyl groups such as cyclopropyl, cyclobutyl, cyclopentyl, cyclohexyl, etc., and the like.

The above "cycloalkenyl group" which is a substituent of the "hydrocarbon group" for $R^1$ includes, for example, $C_{3-6}$ cycloalkenyl groups such as 1-cyclobuten-1-yl, 1-cyclopenten-1-yl, 2-cyclopenten-1-yl, 3-cyclopenten-1-yl, 2-cyclohexen-1-yl, 3-cyclohexen-1-yl, etc., and the like.

The "heterocyclic group" of the above "heterocyclic group which may have substituent(s)" which is a substituent of the "hydrocarbon group" for $R^1$ includes, for example, 5 or 6-membered monocyclic heterocyclic groups having 1 to 4 hetero atoms selected from nitrogen, oxygen and sulfur atoms other than carbon atom(s) (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridadinyl, pyrimidinyl, triazinyl, oxiranyl, azetizinyl, oxetanyl, thietanyl, pyrrolidinyl, tetrahydrofuryl, thiolanyl, piperidyl, tetrahydropyranyl, morpholinyl, thiomorpholinyl, piperazinyl, etc.), and bior tricyclic condensed heterocyclic groups which are formed by condensing the above "5- or 6-membered monocyclic heterocyclic rings" or bi- or tricyclic aromatic condensed heterocyclic groups which are formed by condensing the above "5- or 6-membered monocyclic heterocyclic ring(s)" and benzene ring (preferably, bi- or tricyclic condensed heterocyclic groups containing benzene ring) (e.g., benzofuryl, isobenzofuryl, benzo[b]thienyl, indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolyl, quinazolinyl, quinoxalinyl, phthalazinyl, naphthylidinyl, purinyl, pteridinyl, carbazolyl, α-carbolinyl, β-carbolinyl, γ-carbolinyl, acrydinyl, phenoxazinyl, phenothiazinyl, phenazinyl, phenoxathinyl, thianthrenyl, phenantridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-a]pyridazinyl, imidazo[1,2-a]pyrimidinyl, 1,2,4-triazolo[4,3-a]pyridyl, 1,2,4-triazolo[4,3-b]pyridazinyl, isochromanyl, chromanyl, indolyl, isoindolyl, etc.), and the like. The substituent which the "heterocyclic group" may have includes, for example, oxy group and pyrrolidinyl group other than those similar to the substituents which the "cyclic hydrocarbon group" as ring B may have. The "heterocyclic group" may have 1 to 5 substituents selected from these substituents.

The above "alkyl group" which is a substituent of the "hydrocarbon group" for $R^1$ includes, for example, $C_{1-6}$ alkyl groups such as methyl, ethyl, propyl, isopropyl, butyl, isobutyl, sec-butyl, t-butyl, etc., and the like.

The above "halogenoalkyl group" which is a substituent of the "hydrocarbon group" for $R^1$ includes, for example, $C_{1-6}$ alkyl groups substituted by 1 to 5 halogen atoms (e.g., fluorine, chlorine, bromine, iodine, etc.), (e.g., trifluoromethyl, trichloromethyl, etc.), and the like.

The "aryl group" of the above "aryl group which may have substituent(s)" which is a substituent of the "hydrocarbon group" for $R^1$ includes, for example, $C_{6-14}$ aryl groups such as phenyl, naphthyl, 2-biphenyl, 3-biphenyl, 4-biphenyl, anthranyl, phenanthryl, acenaphthylenyl, etc., and the like. The "aryl group" may have 1 to 5 substituents selected from, for example, halogen atoms (e.g., fluorine, chlorine, bromine, iodine, etc.), $C_{1-6}$ alkyl groups (e.g., methyl, ethyl, propyl, isopropyl, butyl, t-butyl, etc.), halogeno-$C_{1-6}$ alkyl groups (e.g., $C_{1-6}$ alkyl groups substituted by 1 to 5 of the "halogen atoms"; for example, trifluoromethyl, etc.), $C_{1-6}$ alkoxy groups (e.g., methoxy, ethoxy, propoxy, isopropoxy, t-butoxy, etc.), $C_{7-14}$ aralkyloxy groups (e.g., benzyloxy, etc.), hydroxy group, amino group, mono- or di-$C_{1-6}$ alkylamino groups (e.g., methylamino, ethylamino, dimethylamino, diethylamino, etc.), carboxyl group, $C_{1-6}$ alkyl-carbonyl groups (e.g., acetyl, propionyl, butyryl, etc.), $C_{1-6}$ alkoxy-carbonyl groups (e.g., methoxycarbonyl, ethoxycarbonyl, propoxycarbonyl, secpropoxycarbonyl, butoxycarbonyl, etc.), nitro group and cyano group.

As the "heterocyclic group which may have substituent(s)" represented by $R^1$, there may be used those similar to the examples of the "heterocyclic group which may have substituent(s)" mentioned as the substituent on the above "hydrocarbon group" represented by $R^1$.

As the acyl group for $R^1$, there may be mentioned those similar to the examples of the acyl group which the

"hydroxy group which may have substituent(s)" mentioned as the substituents on the above "hydrocarbon group which may have substituent(s)" represented by $R^1$ may have.

$R^1$ is preferably, for example, hydrogen atom, a $C_{1-6}$ alkyl group, a $C_{2-6}$ alkenyl group, a $C_{6-14}$ aryl group or a $C_{7-14}$ aralkyl group which each may be substituted by substituent(s) selected from (1) a halogen, (2) nitro, (3) amino which may have 1 or 2 substituents selected from a $C_{1-6}$ alkyl which may be substituted by a $C_{1-6}$ alkyl-carbonyl, benzoyloxycarbonyl and a $C_{1-6}$ alkylsulfonyl, (4) hydroxy which may be substituted by (i) a $C_{1-6}$ alkyl which may be substituted by hydroxy, a $C_{1-6}$ alkyl-carbonyl, carboxy or a $C_{1-6}$ alkoxy-carbonyl, (ii) phenyl which may be substituted by hydroxy, (iii) benzoyl or (iv) a mono- or di- $C_{1-6}$ alkylamino-carbonyl, (5) a $C_{3-6}$ cycloalkyl, (6) phenyl which may be substituted by hydroxy or a halogeno-$C_{1-6}$ alkyl and (7) thienyl, furyl, thiazolyl, indanyl, indolyl or benzyloxycarbonylpiperidyl. Among them, preferred is a $C_{1-6}$ alkyl group or a $C_{7-14}$ aralkyl group which each may be substituted by a substituent selected from (1) hydroxy, (2) phenyl, and (3) amino which may be substituted by a $C_{1-6}$ alkyl-carbonyl or a $C_{1-6}$ alkyl-sulfonyl. The substituting position of the substituent on the aralkyl group represented by $R^1$ is preferably para position.

In the above formula, $R^2$ represents amino group which may have substituent(s). The "amino group which may have substituent(s)" includes, for example, (i) unsubstituted amino group, (ii) an amino group having 1 or 2 substituents selected from hydrocarbon groups which may have substituent(s), heterocyclic groups which may have substituent(s) and acyl groups, and (iii) nitrogen-containing heterocyclic groups which may have substituent(s), and the like.

As the "hydrocarbon group which may have substituent(s)" for $R^2$, there may be used those similar to the "hydrocarbon group which may have substituent(s)" represented by $R^1$.

As the "heterocyclic group which may have substituent(s)" for $R^2$, there may be used those similar to the "heterocyclic group which may have substituent(s)" represented by $R^1$.

Examples of the "acyl group" for $R^2$ include formyl group, $C_{1-6}$ alkyl-carbonyl groups (e.g., acetyl, propionyl, butyryl, etc.), benzoyl group, $C_{1-6}$ alkoxy-carbonyl groups (e.g., methoxycarbonyl, ethoxycarbonyl, propoxycarbonyl, sec-propoxycarbonyl, butoxycarbonyl, t-butoxycarbonyl, etc.), $C_{7-14}$ aralkyloxy-carbonyl groups (e.g., benzyloxycarbonyl group, etc.), piperidin-4-ylcarbonyl group, $C_{1-6}$ alkylsulfonyl groups (e.g., methylsulfonyl, ethylsulfonyl, propylsulfonyl, sec-propylsulfonyl, butylsulfonyl, t-butylsulfonyl, etc.), carbamoyl group, thiocarbamoyl group, mono- or di-$C_{1-6}$ alkyl-carbamoyl groups (e.g., methylcarbamoyl, ethylcarbamoyl, dimethylcarbamoyl, diethylcarbamoyl, etc.), mono- or di-$C_{1-6}$ alkyl-thiocarbamoyl groups (e.g., methylthiocarbamoyl, ethylthiocarbamoyl, dimethylthiocarbamoyl, diethylthiocarbamoyl, etc.), and the like. These may further have 1 to 3 substituents selected from, for example, halogen atoms (e.g., fluorine, chlorine, bromine, iodine, etc.), hydroxy group, $C_{1-6}$ alkoxy groups (e.g., methoxy, ethoxy, propoxy, isopropoxy, etc.), formyl group, $C_{1-6}$ alkyl-carbonyl groups (e.g., acetyl, propionyl, butyryl, etc.), carboxyl group, $C_{1-6}$ alkoxy-carbonyl groups (e.g., methoxycarbonyl, ethoxycarbonyl, propoxycarbonyl, sec-propoxycarbonyl, butoxycarbonyl, etc.), amino group, mono- or di-$C_{1-6}$ alkylamino groups (e.g., methylamino, ethylamino, dimethylamino, diethylamino, etc.), pyrrolidinyl group, piperidyl group, morpholinyl group, thiomorpholinyl group, 4-methylpiperidyl group, 4-phenylpiperidyl group, carbamoyl group, thiocarbamoyl group, mono- or di-$C_{1-6}$ alkyl-carbamoyl groups (e.g., methylcarbamoyl, ethylcarbamoyl, dimethylcarbamoyl, diethylcarbamoyl, etc.), mono- or di-$C_{1-6}$ alkyl-thiocarbamoyl groups (e.g., methylthiocarbamoyl, ethylthiocarbamoyl, dimethylthiocarbamoyl, diethylthiocarbamoyl, etc.), phenoxy group, mono- or di-$C_{1-6}$ alkyl-carbamoyloxy groups (e.g., methylcarbamoyloxy, ethylcarbamoyloxy, dimethylcarbamoyloxy, diethylcarbamoyloxy, etc.), mono- or di-$C_{1-6}$ alkylthiocarbamoyloxy groups (e.g., methylthiocarbamoyloxy, ethylthiocarbamoyloxy, dimethylthiocarbamoyloxy, diethylthiocarbamoyloxy, etc.), formylamino group, $C_{1-6}$ alkyl-carbonylamino groups (e.g., acetylamino, propionylamino, butyrylamino, etc.), formyloxy group, and $C_{1-6}$ alkyl-carbonyloxy groups (e.g., acetoxy, etc.), etc.

The "nitrogen-containing heterocyclic group" of the "nitrogen-containing heterocyclic group which may have substituent(s)" for $R^2$ includes, for example, 5 to 7-membered nitrogen-containing heterocyclic groups which may have 1 to 4 hetero atoms selected from nitrogen, oxygen and sulfur atoms other than the nitrogen atom(s) having a bond (e.g., 1-imidazolyl, 1-pyrazolyl, 1-pyrrolyl, 1-pyrrolidinyl, 1-piperidyl, morpholinyl, thiomorpholinyl, etc.) or rings formed by condensing the 5 to 7-membered nitrogen-containing heterocyclic group with benzene, pyridine, etc. (e.g., 1-benzimidazolyl, 1,2,3,4-tetrahydroisoquinolin-2-yl, 1,2,3,4-tetrahydroquinolin-1-yl, 1-indolyl, etc.), and the like.

As the substituent for $R^2$ which the "nitrogen-containing heterocyclic group" may have, there may be used those similar to the substituents which the above "cyclic hydrocarbon" in ring B may have, for example. Preferred are halogen atoms (e.g., fluorine, chlorine, bromine, iodine, etc.), $C_{1-6}$ alkyl groups (e.g., methyl, ethyl, propyl, butyl, sec-butyl, t-butyl, isopropyl, etc.), and $C_{1-6}$ alkoxy groups (e.g., methoxy, ethoxy, propoxy, butoxy, sec-butoxy, t-butoxy, isopropoxy, etc.), and the number of the substituents is from 1 to 5.

$R^2$ is preferably, for example, (1) unsubstituted amino group, (2) piperidyl group or (3) amino group which may have 1 or 2 substituents selected from (i) benzyl, (ii) a $C_{1-6}$ alkyl which may be substituted by amino or phenyl, (iii) a mono- or di-$C_{1-6}$ alkyl-carbamoyl, (iv) a mono- or di-$C_{1-6}$ alkyl-thiocarbamoyl, (v) a $C_{1-6}$ alkoxy-carbonyl, (vi) a $C_{1-6}$ alkyl-sulfonyl, (vii) piperidylcarbonyl and (viii) a $C_{1-6}$ alkyl-carbonyl which may be substituted by a halogen or amino. Particularly preferred is unsubstituted amino group.

E represents a bond, -CO-, -CON($R^a$)-, -COO-,-N($R^a$)CON($R^b$)-, -N($R^a$)COO-, -N($R^a$)$SO_2$-, -N($R^a$)-, -O-, -S-, -SO- or -$SO_2$- ($R^a$ and $R^b$ each independently represents hydrogen atom or a hydrocarbon group which may have substituent(s)). Preferably, -CON($R^a$)- and -N($R^a$)CON($R^b$)-are used, and $R^a$ and $R^b$ each is preferably hydrogen atom. Particularly preferred is -CONH-.

As the "hydrocarbon group which may have substituent(s)" for $R^a$ or $R^b$, there may be used, for example, those similar to the above "hydrocarbon group which may have substituent(s)" represented by $R^1$.

In the above formula, L represents a bond or a divalent group. The divalent group includes, for example, divalent hydrocarbon groups which may have substituent(s) and may be bonded through -O- or -S-.

L is preferably, for example, a divalent hydrocarbon group which may have substituent(s), and particularly preferred is a $C_{1-6}$ alkylene group which may have substituent(s).

As the "divalent hydrocarbon group which may have substituent(s)" for L, there may be used, for example, those similar to the above "divalent group" represented by D. The "$C_{1-6}$ alkylene group" of the "$C_{1-6}$ alkylene group which may have substituent(s)" includes, for example, methylene, ethylene, propylene, butylene, and the like. The "$C_{1-6}$ alkylene group" may have, for example, 1 to 5 of $C_{1-6}$ alkyl groups (e.g., methyl, ethyl, propyl, isopropyl, butyl, etc.), and the like.

L is preferably $C_{1-6}$ alkylene group which may be substituted by $C_{1-6}$ alkyl and may be bonded through -O-, and particularly preferred is a $C_{1-6}$ alkylene group (preferably methylene, etc.) or the like.

X and Y each represents hydrogen atom or an independent substituent. The independent substituent for X or Y include those similar to the "substituent" represented by above A.

The compound represented by the formula (I) is preferably the compound wherein X and Y each independently is , a halogen, hydroxy, a $C_{1-6}$ alkoxy, a halogeno-$C_{1-6}$ alkoxy, a $C_{7-14}$ aralkyloxy, a benzoyl-$C_{1-6}$ alkoxy, a hydroxy-$C_{1-6}$ alkoxy, a $C_{1-6}$ alkoxy-carbonyl-$C_{1-6}$ alkoxy, a $C_{3-14}$ cycloalkyl-$C_{1-6}$ alkoxy, an imidazol-1-yl-$C_{1-6}$ alkoxy, a $C_{7-14}$ aralkyloxycarbonyl-$C_{1-6}$ alkoxy, or a hydroxyphenyl-$C_{1-6}$ alkoxy;

ring B is benzene ring which may be substituted by a $C_{1-6}$ alkoxy, or tetrahydroisoquinoline ring or isoindoline ring which is formed by combination with $R^2$;
Z is a $C_{6-14}$ aryl group, a $C_{3-10}$ cycloalkyl group, piperidyl group, thienyl group, furyl group, pyridyl group, thiazolyl group, indanyl group or indolyl group which may have 1 to 3 substituents selected from a halogen, formyl, a halogeno-$C_{1-6}$ alkyl, a $C_{1-6}$ alkoxy, a $C_{1-6}$ alkyl-carbonyl, oxo and pyrrolidinyl;
A is hydrogen atom;
D is a $C_{1-6}$ alkylene group;
G is a bond, or a $C_{1-6}$ alkylene group which may contain phenylene and may substituted by phenyl;
$R^1$ is hydrogen atom, a $C_{1-6}$ alkyl group, a $C_{2-6}$ alkenyl group, a $C_{6-14}$ aryl group or a $C_{7-14}$ aralkyl group which each may be substituted by substituent(s) selected from (1) a halogen, (2) nitro, (3) amino which may have 1 or 2 substituents selected from a $C_{1-6}$ alkyl which may be substituted by a $C_{1-6}$ alkyl-carbonyl, benzoyloxycarbonyl and a $C_{1-6}$ alkylsulfonyl, (4) hydroxy which may be substituted by (i) a $C_{1-6}$ alkyl which may be substituted by hydroxy, a $C_{1-6}$ alkyl-carbonyl, carboxy or a $C_{1-6}$ alkoxy-carbonyl, (ii) phenyl which may be substituted by hydroxy, (iii) benzoyl or (iv) a mono- or di- $C_{1-6}$ alkylamino-carbonyl, (5) a $C_{3-6}$ cycloalkyl, (6) phenyl which may be substituted by hydroxy or a halogeno-$C_{1-6}$ alkyl and (7) thienyl, furyl, thiazolyl, indolyl or benzyloxycarbonylpiperidyl;
$R^2$ is (1) unsubstituted amino group, (2) piperidyl group or (3) amino which may have 1 or 2 substituents selected from (i) benzyl, (ii) a $C_{1-6}$ alkyl which may be substituted by amino or phenyl, (iii) a mono- or di-$C_{1-6}$ alkyl-carbamoyl, or a mono- or di-$C_{1-6}$ alkyl-thiocarbamoyl, (iv) a $C_{1-6}$ alkoxy-carbonyl, (v) a $C_{1-6}$ alkyl sulfonyl, (vi) piperidylcarbonyl and (vii) a $C_{1-5}$ alkyl-carbonyl which may be substituted by a halogen or amino;
E is a bond, -CON($R^a$)-, -N($R^a$)CO-, -N($R^a$)CON($R^b$)- ($R^a$ and $R^b$ each represents hydrogen atom or a $C_{1-6}$ alkyl group);
L is preferably a $C_{1-6}$ alkylene group which may be bonded through -O- and may be substituted by a $C_{1-6}$ alkyl.

Particularly preferred is the compound wherein X and Y each independently is a halogen, hydroxy or a $C_{1-6}$ alkoxy;

ring B is benzene ring, or, by combination with $R^2$, tetrahydroisoquinoline ring or isoindoline ring;
Z is phenyl group which may be substituted by a halogen, D is a $C_{1-6}$ alkylene group, G is a $C_{1-6}$ alkylene group;
$R^1$ is a $C_{1-6}$ alkyl group or a $C_{7-14}$ aralkyl group which each may be substituted by substituent(s) selected from (1) hydroxy, (2) phenyl and (3) amino which may be substituted by a $C_{1-6}$ alkyl-carbonyl or a $C_{1-6}$ alkylsulfonyl;
$R^2$ is unsubstituted amino group, E is -CONH-, L is a $C_{1-6}$ alkylene group.

Among the compounds represented by the formula (I), a compound represented by the formula (Ia-a) or a salt thereof can be produced, for example, by the method of, using a compound represented by the formula (IIa), a

reactive derivative thereof or a salt thereof as the intermediate, reacting the compounds with a compound represented by the formula (III) or a salt thereof as exemplified by following Scheme 1.

Scheme 1

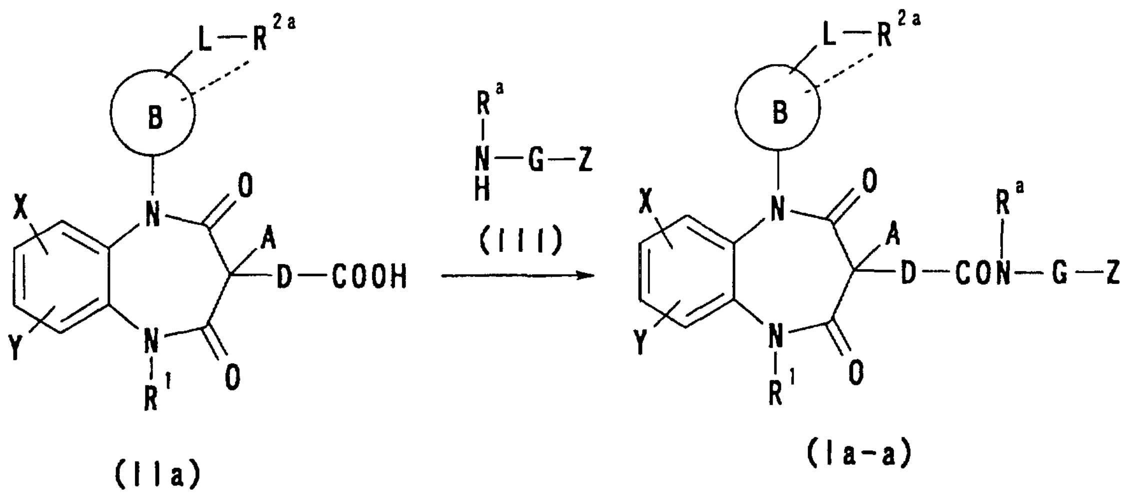


(IIa)

(Ia-a)

[in Scheme 1, $R^{2a}$ represents a group which may have a protective group (e.g., t-butoxycarbonyl, benzyloxycarbonyl, trityl, etc.) in the above $R^2$, and other symbols have the same meanings as described above.]

The compound represented by the formula (Ia-a) or the salt thereof can be produced by reacting the compound represented by the formula (IIa), the reactive derivative thereof or the salt thereof with the compound represented by the formula (III) or the salt thereof in a solvent, optionally in the presence of a base using a condensing agent. The reactive derivatives of the compound represented by the formula (IIa) include, for example, acid anhydrides, acid halides (acid chlorides, acid bromides), imidazolides, or mixed acid anhydrides (e.g., anhydrides with methyl carbonic acid, ethyl carbonic acid, etc.), and the concrete examples include the compounds wherein COOH in the compound represented by the formula (IIa) is changed into COQ [wherein Q represents a leaving group: a halogen atom {fluorine, chlorine, bromine, iodine, etc.), methanesulfonyloxy, benzenesulfonyloxy, p-toluenesulfonyloxy, and the like]. The solvents to be used in the reaction of Scheme 1 include, for example, ether-type solvents (e.g., diethyl ether, tetrahydrofuran, dioxane, etc.), hydrocarbon-type solvents (e.g., benzene, toluene, hexane, heptane, etc.), halogen-type solvents (e.g., dichloromethane, dichloroethane, chloroform, carbon tetrachloride, etc.), acetonitrile, dimethylformamide, and the like. The bases to be used include triethylamine, 4-dimethylaminopyridine, triethylenediamine, tetramethylethylenediamine, and the like. The condensing agents to be used include, for example, condensing agents which are used for peptide synthesis, concretely, dicyclohexylcarbodiimide, diethyl cyanophosphate, 1-ethyl-3-(3-dimethylaminopropyl)carbodiimide, and the like. At that time, relative to 1 mol of the compound represented by the formula (IIa) or the salt thereof, the compound represented by the formula (III) or the salt thereof is used in an amount of 0.5 to 2 molar equivalents, preferably 1 to 1.2 molar equivalents, and the condensing agent is used in an amount of 0.5 to 5 molar equivalents, preferably 1 to 2 molar equivalents. The reaction temperature is from 0 to 100°C, preferably 20 to 50°C, and the reaction time is from 0.5 to 24 hours, preferably 1 to 5 hours.

The compound represented by the formula (IIa) in above Scheme 1 or the salt thereof can be produced by the method shown in following Scheme 2.

Scheme 2

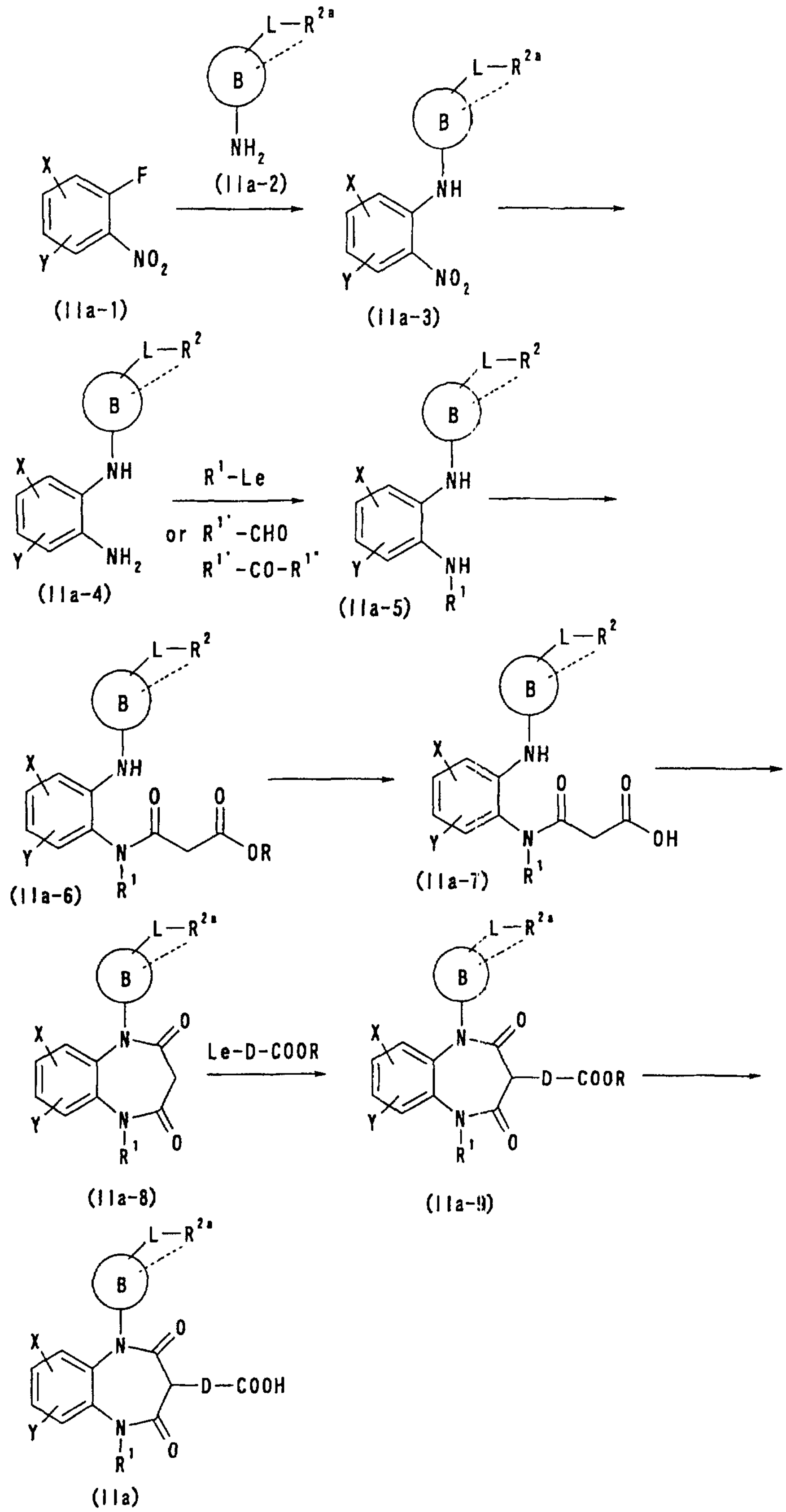


(IIa-1) (IIa-2) (IIa-3) (IIa-4) (IIa-5) (IIa-6) (IIa-7) (IIa-8) (IIa-9) (IIa)

[in Scheme 2, Le represents a leaving group (e.g., chlorine, bromine, iodine, methanesulfonyloxy, toluenesulfonyloxy, etc.); $R^{1'}$ or $R^{1''}$ represents a group selected from the hydrocarbon groups which may have substituent(s) represented by $R^1$ except methylene chain; R represents a $C_{1-6}$ alkyl group, a $C_{7-14}$ aralkyl group and phenyl group which each may be substituted by a halogen atom or a $C_{1-6}$ alkoxy; other symbols have the same meanings as described above.]

The compound represented by the formula (IIa-3) or the salt thereof in above Scheme 2 can be produced by reacting the compound represented by the formula (IIa-1) or the salt thereof with the compound represented by the formula (IIa-2) or the salt thereof. The reaction can be carried out without solvent or in a solvent of an ether-type solvent (e.g., diethyl ether, tetrahydrofuran, dioxane, etc.), a halogen-type solvent (e.g., dichloromethane, dichloroethane, chloroform, carbon tetrachloride, etc.), a hydrocarbon-type solvent (e.g., benzene, toluene, hexane, heptane, etc.), dimethylformamide, dimethylsulfoxide, an ester-type solvent (e.g., ethyl acetate, methyl acetate, etc.) or the like, optionally in the presence of a base (e.g., sodium hydrogen carbonate, potassium hydrogen carbonate, sodium carbonate, potassium carbonate, sodium hydride, potassium hydride, etc.). Relative to 1 mol of the compound represented by the formula (IIa-1) or the salt thereof, the compound represented by the formula (IIa-2) or the salt thereof is used in an amount of 0.5 to 5 molar equivalents, preferably 0.8 to 2 molar equivalents. At that time, the reaction temperature is from 0 to 200°C, preferably 80 to 150°C. The base is used in an amount of 0.5 to 5 molar equivalents, preferably 1 to 1.5 molar equivalents relative to 1 mol of the compound represented by the formula (IIa-2). The reaction time is from 0.5 to 48 hours, preferably 0.5 to 24 hours.

The reaction from the compound represented by the formula (IIa-3) or the salt thereof to the compound represented by the formula (IIa-4) or the salt thereof can be carried out in a solvent of an ether-type solvent (e.g., diethyl ether, tetrahydrofuran, dioxane, etc.), a hydrocarbon-type solvent (e.g., benzene, toluene, hexane, heptane, etc.), an alcohol-type solvent (e.g., methanol, ethanol, propanol, etc.), acetone, dimethylformamide, or the like, by subjecting to a catalytic reduction using hydrogen and a metal catalyst such as a palladium-type catalyst (e.g., metal palladium, palladium supported on carbon, etc.), Raney-Ni, platinum or the like, or a reduction reaction using a metal or a metal salt such as iron chloride, tin chloride, or like. The hydrogen pressure is from 1 to 100 atm, preferably 1 to 10 atm, and the reaction temperature is from 0 to 200°C, preferably 10 to 50°C. (The reaction time is from 0.5 to 48 hours, preferably 0.5 to 12 hours.)

In the reaction from the compound represented by the formula (IIa-4) or the salt thereof to the compound represented by the formula (IIa-5) or the salt thereof, it can be produced by a nitrogen-carbon bond forming reaction between the compound represented by the formula (IIa-4) or the salt thereof and a halogenated hydrocarbon, a sulfonic acid ester or the like, or a reductive alkylation with an aldehyde or a ketone. The nitrogen-carbon bond forming reaction is carried out in a solvent of an ether-type solvent (e.g., diethyl ether, tetrahydrofuran, dioxane, etc.), a halogen-type solvent (e.g., dichloromethane, dichloroethane, chloroform, carbon tetrachloride, etc.), a hydrocarbon-type solvent (e. g., benzene, toluene, hexane, heptane, etc.), an alcohol-type solvent (e.g., methanol, ethanol, propanol, butanol, etc.), acetonitrile, dimethylformamide, dimethylsulfoxide, an ester-type solvent (e.g., ethyl acetate, methyl acetate, etc.) or the like or a mixed solvent thereof, optionally in the presence of a phase transfer catalyst (e.g., quaternary ammonium salts such as tetrabutylammonium bromide, benzyltriethylammonium chloride, etc. and crown ethers such as 18-Crown-6, etc., and the like) or a base (e.g., sodium hydrogen carbonate, potassium hydrogen carbonate, sodium carbonate, potassium carbonate, sodium hydride, potassium hydride, etc.), optionally in the presence of a phase transfer catalyst and a base. Relative to 1 mol of the compound represented by the formula (IIa-4) or the salt thereof, the compound represented by the formula $R^1$-Le or the salt thereof is used in an amount of 0.5 to 5 molar equivalents, preferably 0.8 to 2 molar equivalents. At that time, the reaction temperature is from 0 to 200°C, preferably 20 to 80°C. The base is used in an amount of 0.5 to 5 molar equivalents, preferably 1 to 1.5 molar equivalents relative to 1 mol of the compound represented by the formula (IIa-4). The reaction time is from 0.5 to 48 hours, preferably 0.5 to 24 hours. The reductive alkylation is carried out in a solvent of an ether-type solvent (e.g., diethyl ether, tetrahydrofuran, dioxane, etc.), a halogen-type solvent (e.g., dichloromethane, dichloroethane, chloroform, carbon tetrachloride, etc.), a hydrocarbon-type solvent (e.g., benzene, toluene, hexane, heptane, etc.), an alcohol-type solvent (e.g., methanol, ethanol, propanol, butanol, etc.) or the like or a mixed solvent thereof, for example, by reacting the compound represented by the formula (IIa-4) or the salt thereof and the compound represented by the formula ($R^{1'}$-CHO) or ($R^{1'}$-CO-$R^{1''}$) or the salt thereof, with catalytic reduction or in the presence of a metal hydrogen complex compound (e.g., sodium borohydride, sodium cyanoborohydride, etc.). Relative to 1 mol of the compound represented by the formula (IIa-4) or the salt thereof, the compound represented by the formula ($R^{1'}$-CHO) or ($R^{1'}$-CO-$R^{1'}$) or the salt thereof is used in an amount of 1 to 10 molar equivalents, preferably 1 to 2 molar equivalents, and the reducing agent is used in an amount of 0.3 to 5 molar equivalents, preferably 0.5 to 1.5 molar equivalents. At that time, the reaction temperature is from 0 to 100°C, preferably 10 to 70°C and the reaction time is from 1 to 24 hours, preferably 3 to 15 hours.

In the reaction from the compound represented by the formula (IIa-5) or the salt thereof to the compound represented by the formula (IIa-6) or the salt thereof in above Scheme 2, it can be produced, for example, by reacting with an acid chloride of a malonic acid monoester (e.g., ethyl malonyl chloride, etc.) in a solvent of an ether-type solvent (e.g., diethyl ether, tetrahydrofuran, dioxane, etc.), a halogen-type solvent (e.g., dichloromethane, dichloroethane, chloroform, carbon tetrachloride, etc.), a hydrocarbon-type solvent (e.g., benzene, toluene, hexane, heptane, etc.), dimethylformamide, dimethylsulfoxide, an ester-type solvent (e.g., ethyl acetate, methyl acetate, etc.), acetonitrile, water or the like. Relative to 1 mol of the compound represented by the formula (IIa-5), the acid chloride of dicarboxylic acid monoester is used in an amount of 1 to 10 molar equivalents, preferably 1 to 2 molar equivalents, the reaction temperature is from -20 to 100°C, preferably 0 to 50°C, and the reaction time is from 0. 5 to 24 hours, preferably 1 to 3 hours.

The compound represented by the formula (IIa-7) or the salt thereof in above Scheme 2 can be produced by treating the compound represented by the formula (IIa-6) or the salt thereof with an acid or a base. Namely, the compound can be produced by treating the compound represented by the formula (IIa-6) or the salt thereof in an aqueous solution of a mineral acid (e.g., nitric acid, hydrochloric acid, hydrobromic acid, iodic acid, sulfuric acid, etc.) or an alkaline metal hydroxide (e.g., sodium hydroxide, potassium hydroxide, barium hydroxide, lithium hydroxide, etc.) or the like, under a condition of 0 to 150°C, preferably 0 to 20°C. At that time, the strength of the acid or base is suitably 1 to 10 N, preferably 1 to 2 N. The reaction time is from 1 to 24 hours, preferably 2 to 10 hours.

The compound represented by the formula (IIa-8) or the salt thereof in above Scheme 2 can be produced by reacting the compound represented by the formula (IIa-7) or the salt thereof in a solvent, optionally in the presence of a base using a condensing agent. The solvents to be used include, for example, ether-type solvents (e.g., diethyl ether, tetrahydrofuran, dioxane, etc.), hydrocarbon-type solvents (e.g., benzene, toluene, hexane, heptane, etc.), halogen-type solvents (e.g., dichloromethane, dichloroethane, chloroform, carbon tetrachloride, etc.), acetonitrile, dimethylformamide, and the like. The bases to be used include triethylamine, 4-dimethylaminopyridine, triethylenediamine, tetramethylethylenediamine, and the like. The condensing agents to be used include, for example, condensing agents which are used for peptide synthesis, concretely, dicyclohexylcarbodiimide, diethyl cyanophosphate, 1-ethyl-3-(3-dimethylaminopropyl)carbodiimide, and the like. At that time, relative to 1 mol of the compound represented by the formula (IIa-7) or the salt thereof, the condensing agent is used in an amount of 0.5 to 5 molar equivalents, preferably 1 to 2 molar equivalents. The reaction temperature is from 0 to 100°C, preferably 20 to 50°C, and the reaction time is from 0.5 to 24 hours, preferably 1 to 5 hours.

In the production of the compound represented by the formula (IIa-9), it can be produced by reacting the compound represented by the formula (IIa-8) with the compound represented by the formula (Le-D-COOR), for example, in the presence of sodium hydride, an alkyllithium or the like. For example, in a solvent of dimethylformamide, acetonitrile, diethyl ether, tetrahydrofuran, dioxane, or the like, the compound represented by the formula (Le-D-COOR) is used in an amount of 0.5 to 5 molar equivalents, preferably 1 to 2 molar equivalents and sodium hydride or an alkyllithium is used in an amount of 0.5 to 3 molar equivalents, preferably 1 to 5 molar equivalent relative to 1 mol of the compound represented by the formula (IIa-8). The reaction temperature is from -20 to 100°C, preferably 0 to 30°C, and the reaction time is from 0.5 to 24 hours, preferably 1 to 3 hours.

In the production of the compound represented by the formula (IIa) or the salt thereof in above Scheme 2, it can be produced by treating the compound represented by the formula (IIa-9) or the salt thereof with an acid or an base. Namely, the compound can be produced by treating the compound represented by the formula (IIa-9) or the salt thereof, for example, in an aqueous solution of a mineral acid (e.g., nitric acid, hydrochloric acid, hydrobromic acid, iodic acid, sulfuric acid. etc.) or an alkaline metal hydroxide (e.g., sodium hydroxide, potassium hydroxide, barium hydroxide, lithium hydroxide, etc.) or the like, under a condition of 0 to 150°C, preferably 20 to 50°C. At that time, the strength of the acid or base is suitably 1 to 10 N, preferably 4 to 10 N. The reaction time is from 1 to 24 hours, preferably 2 to 10 hours.

The compound represented by the formula (IIa-8) or the salt thereof in above Scheme 2 can be also produced by the methods shown in Schemes 3 and 4.

Scheme 3

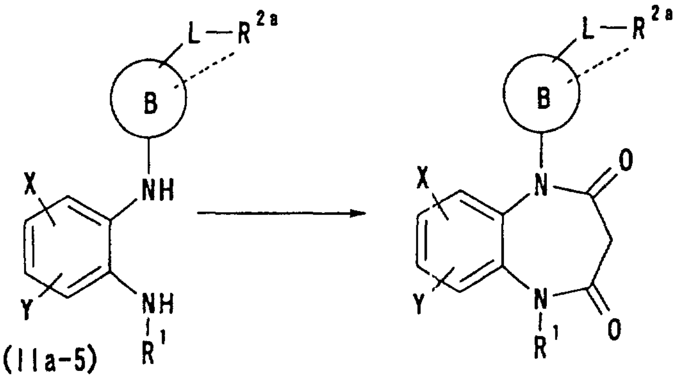

(IIa-5)

(IIa-8)

Scheme 4

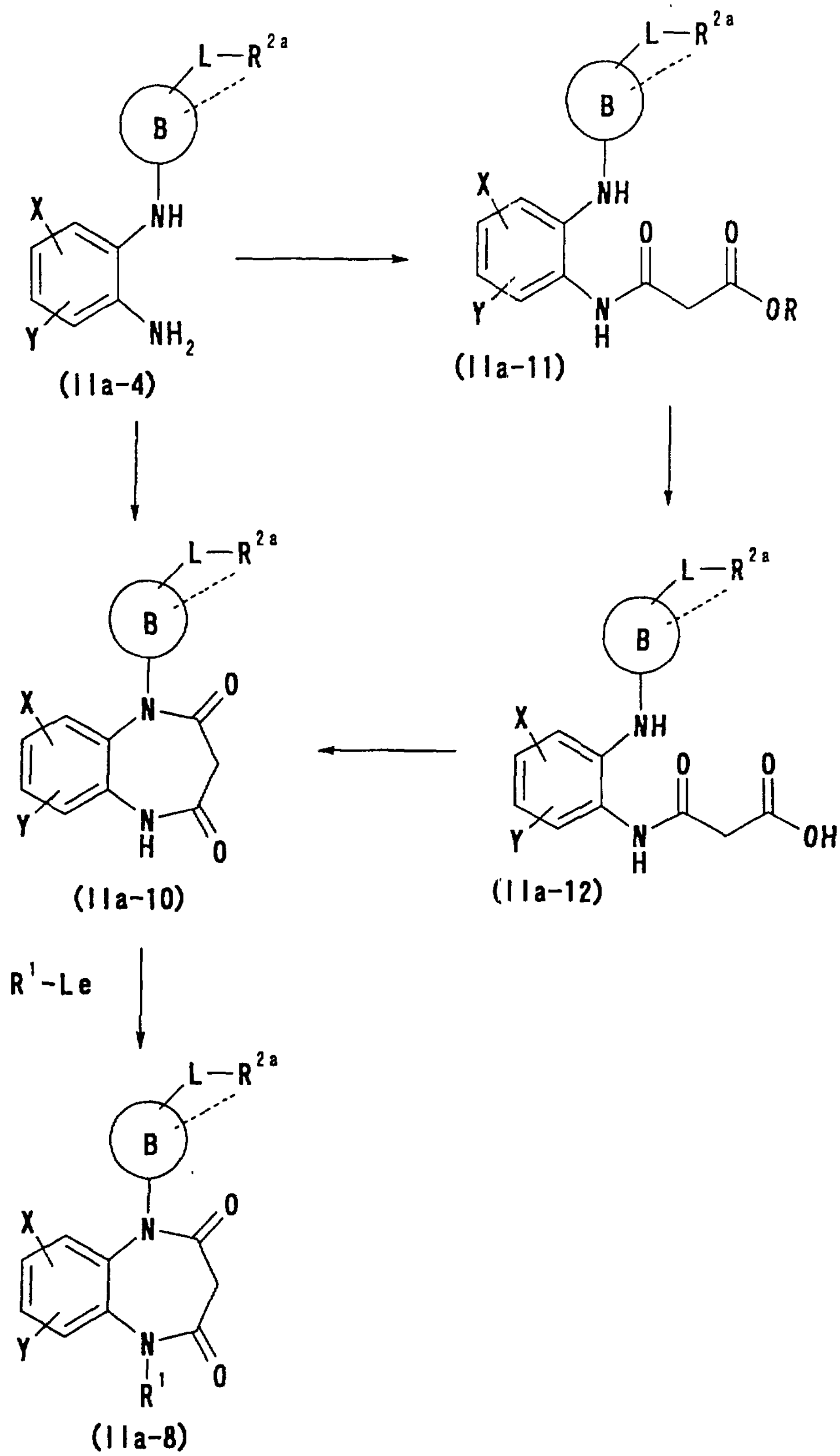


(IIa-4) (IIa-11) (IIa-10) (IIa-12) (IIa-8)

[in Schemes 3 and 4, the symbols have the same meanings as described above.]

Namely, the compound represented by the formula (IIa-8) or the salt thereof can be produced, for example, by reacting the compound represented by the formula (IIa-5) or the salt thereof with malonyl dichloride in a solvent of

an ether-type solvent (e.g., diethyl ether, tetrahydrofuran, dioxane, etc.), a halogen-type solvent (e.g., dichloromethane, dichloroethane, chloroform, carbon tetrachloride, etc.), a hydrocarbon-type solvent (e.g., benzene, toluene, hexane, heptane, etc.), dimethylformamide, dimethylsulfoxide, an ester-type solvent (e.g., ethyl acetate, methyl acetate, etc.), acetonitrile, water or the like. Relative to 1 mol of the compound represented by the formula (IIa-5), malonyl dichloride is used in an amount of 1 to 10 molar equivalents, preferably 1 to 2 molar equivalents, the reaction temperature is from -20 to 100°C, preferably 0 to 70°C, and the reaction time is from 0.5 to 24 hours, preferably 1 to 3 hours. Also, the compound represented by the formula (IIa-8) or the salt thereof in above Scheme 4 can be produced from the compound represented by the formula (IIa-4) or the salt thereof, in a similar manner to the method shown in Scheme 2 or 3 using the compound represented by the formula (IIa-10) or the salt thereof as production intermediate, by reacting it with the formula (Le-$R^1$).

The reaction between the formula (IIa-10) or the and the formula ($R^1$-Le) can be carried out in a solvent of an ether-type solvent (e.g., diethyl ether, tetrahydrofuran, dioxane, etc.), a halogen-type solvent (e.g., dichloromethane, dichloroethane, chloroform, carbon tetrachloride, etc.), a hydrocarbon-type solvent (e.g., benzene, toluene, hexane, heptane, etc.), an alcohol-type solvent (e.g., methanol, ethanol, propanol, butanol, etc.), acetonitrile, dimethylformamide, dimethylsulfoxide, an ester-type solvent (e.g., ethyl acetate, methyl acetate, etc.) or the like or a mixed solvent thereof, optionally in the presence of a phase transfer catalyst (e.g., quaternary ammonium salts such as tetrabutylammonium bromide, benzyltriethylammonium chloride, etc. and crown ethers such as 18-Crown-6, etc., and the like) or a base (e.g., sodium hydrogen carbonate, potassium hydrogen carbonate, sodium carbonate, potassium carbonate, sodium hydride, potassium hydride, etc.). Relative to 1 mol of the compound represented by the formula (IIa-4) or the salt thereof, the compound represented by the formula $R^1$-Le is used in an amount of 0.5 to 5 molar equivalents, preferably 0.8 to 2 molar equivalents. At that time, the reaction temperature is from 0 to 200°C, preferably 20 to 80°C. The base is used in an amount of 0.5 to 5 molar equivalents, preferably 1 to 1.5 molar equivalents relative to 1 mol of the compound represented by the formula (IIa-4). The reaction time is from 0.5 to 24 hours.

Among the compounds represented by the formula (I) or the salts thereof, the compounds represented by the formulae (Ia-b), (Ia-c), (Ia-d) and (Ia-e) or the salts thereof can be produced by the method exemplified in following Scheme 5.

Scheme 5

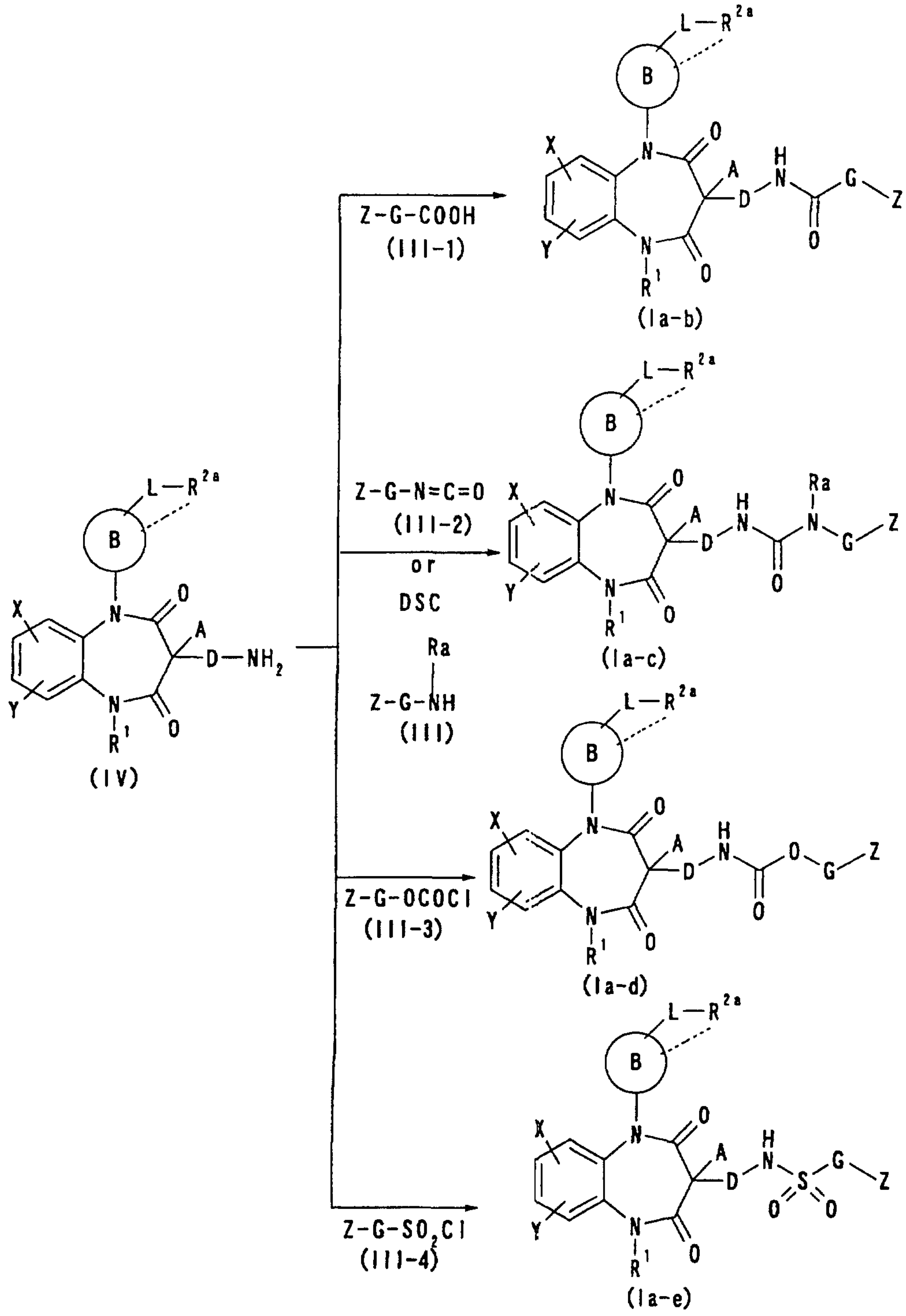


[wherein the symbols have the same meanings as described above.]

The compound represented by the formula (Ia-b) or the salt thereof in above Scheme 5 can be produced by reacting the compound represented by the formula (IV) or the salt thereof with the compound represented by the formula (III-1) or the salt thereof. The reaction is carried out using similar conditions to those in the condensation of the compound represented by the formula (IIa) or the salt thereof with the compound represented by the formula (III) or the salt thereof at the production of the compound represented by the formula (Ia-a) or the salt thereof as exemplified in above Scheme 1.

The compound represented by the formula(Ia-c) or the salt thereof in above Scheme 5 can be produced by reacting the compound represented by the formula (IV) or the salt thereof with the compound represented by the formula (III) and a reagent such as DSC (N,N'-disuccinimidyl carbonate) or the like or the compound represented by the formula (III-2) or the salt thereof. In the reaction, the solvents to be used include, for example, ether-type solvents (e.g., diethyl ether, tetrahydrofuran, dioxane, etc.), halogen-type solvents (e.g., dichloromethane, dichloroethane, chloroform, etc.), acetonitrile, dimethylformamide, and the like. Abase (e.g., triethylamine, 4-dimethylaminopyridine, triethylenediamine, tetramethylethylenediamine, etc.) is used optionally. In the reaction, relative to 1 mol of the compound represented by the formula (IV) or the salt thereof, the compound represented by the formula (III) and a reagent such as DSC(N,N'-disuccinimidyl carbonate) or the like or the compound represented by the formula (III-2) or the salt thereof is used in an amount of 1 to 10 molar equivalents, preferably 1 to 2 molar equivalents. At that time, the reaction temperature is from 0 to 100°C, preferably 20 to 50°C, and the reaction time is from 1 to 24 hours, preferably 3 to 10 hours.

The compound represented by the formula (Ia-d) or the salt thereof in above Scheme 5 can be produced by reacting the compound represented by the formula (IV) or the salt thereof with the compound represented by the formula (III-3) or the salt thereof. In the reaction, the solvents to be used include, for example, ether-type solvents (e.g., diethyl ether, tetrahydrofuran, dioxane, etc.), halogen-type solvents (e.g., dichloromethane, dichloroethane, chloroform, etc.), acetonitrile, dimethylformamide, and the like. Abase (e.g., triethylamine, 4-dimethylaminopyridine, triethylenediamine, tetramethylethylenediamine, etc.) is used optionally. In the reaction, relative to 1 mol of the compound represented by the formula (IV) or the salt thereof, the compound represented by the formula (III-3) or the salt thereof is used in an amount of 1 to 10 molar equivalents, preferably 1 to 2 molar equivalents. At that time, the reaction temperature is from 0 to 100°C, preferably 20 to 50°C, and the reaction time is from 1 to 24 hours, preferably 3 to 10 hours.

The compound represented by the formula (Ia-e) or the salt thereof in above Scheme 5 can be produced by reacting the compound represented by the formula (IV) or the salt thereof with the compound represented by the formula (III-4) or the salt thereof. The reaction can be carried out in a solvent of an ether-type solvent (e.g., diethyl ether, tetrahydrofuran, dioxane, etc.), an alcohol-type solvent (e.g., methanol, ethanol, propanol, butanol, etc.), acetone, dimethylformamide, or the like, optionally in the presence of a base (e.g., sodium hydrogen carbonate, potassium hydrogen carbonate, sodium carbonate, potassium carbonate, sodium hydride, potassium hydride, triethylamine, etc.). In the reaction, relative to 1 mol of the compound represented by the formula (IV) or the salt thereof, the compound represented by the formula (III-4) or the salt thereof is used in an amount of 1 to 10 molar equivalents, preferably 1 to 2 molar equivalents. At that time, the reaction temperature is from 0 to 100°C, preferably 20 to 50°C, and the reaction time is from 1 to 24 hours, preferably 3 to 10 hours.

The compound represented by the formula (IV) or the salt thereof in above Scheme 5 can be produced by the method shown in following Scheme 6. The compound can be produced by reacting the compound represented by the formula (IIa) or the salt thereof with diphenyl phosphorylazide or the like in a solvent in the presence of a base, then subjecting the resulting acylazide product to Curtius rearrangement in a solvent to obtain an isocyanate derivative (V) as a production intermediate, and treating it with an acid.

Alternatively, the compound represented by the formula (IV) or the salt thereof can be produced by the conversion of the isocyanate derivative (V) into a carbamate derivative (VI), followed by further conversion into the compound represented by the formula (IV) or the salt thereof, as shown in following Scheme 7.

Scheme 6

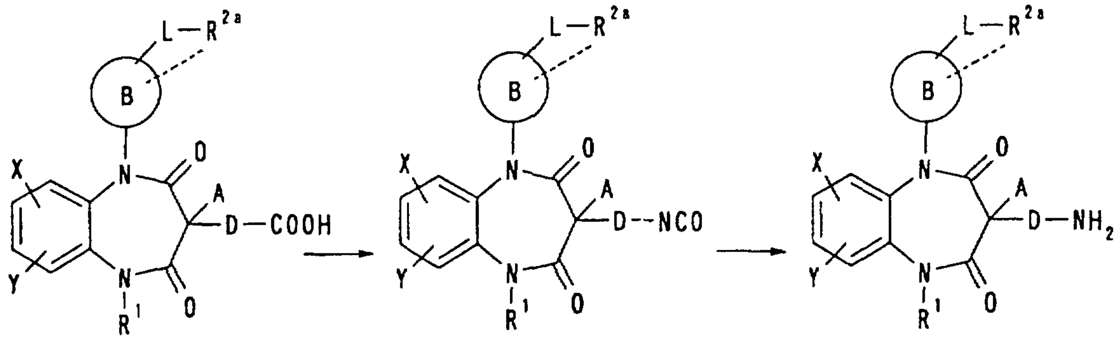

(IIa) (V) (IV)

Scheme 7

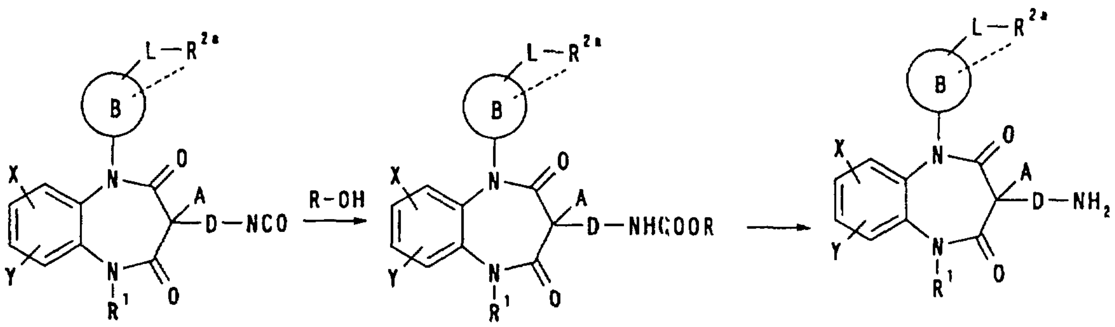


(V) (VI) (IV)

[in Schemes 6 and 7, the symbols have the same meanings as described above.]

In the reaction of the compound represented by the formula (IIa) or the salt thereof with diphenyl phosphorylazide in above Scheme 6, the solvents to be used include, for example, ether-type solvents (e.g., diethyl ether, tetrahydrofuran, dioxane, etc.), halogen-type solvents (e.g., dichloromethane, dichloroethane, chloroform, etc.), dimethylformamide, and the like. The bases to be used include, for example, triethylamine, 4-dimethylaminopyridine, triethylenediamine, tetramethylethylenediamine, and the like. In the reaction, relative to 1 mol of the compound represented by the formula (IIa) or the salt thereof, diphenyl phosphorylazide is used in an amount of 1 to 10 molar equivalents, preferably 1.5 to 3 molar equivalents. At that time, the reaction temperature is from -20 to 50°C, preferably 0 to 20°C, and the reaction time is from 0.5 to 5 hours, preferably 1 to 2 hours.

In the case of subjecting the product obtained in the above reaction to Curtius rearrangement, the solvents to be used include, for example, hydrocarbon-type solvents (e.g., benzene, toluene, xylene, etc.), ether-type solvents (e.g., diethyl ether, tetrahydrofuran, dioxane, etc.), halogen-type solvents (e.g., dichloromethane, dichloroethane, chloroform, etc.), dimethylformamide, and the like. The reaction temperature is from 50 to 200°C, preferably 80 to 150°C, and the reaction time is from 0.5 to 12 hours, preferably 1 to 3 hours.

In the case of treating the product obtained in the above reaction with an acid, the solvents to be used include, for example, water, dioxane, dimethylformamide, and the like. Examples of the acid to be used include mineral acids such as sulfuric acid, hydrochloric acid, nitric acid and hydrobromic acid. At that time, the reaction temperature is from 20 to 200°C, preferably 50 to 100°C, and the reaction time is from 0.5 to 5 hours, preferably 1 to 2 hours.

The compound represented by the formula (Ia-c) or (Ia-d) or the salt thereof in above Scheme 5 can be produced by reacting the compound represented by the formula (V) in Scheme 6 with the compound represented by the formula (III) or (VII) as exemplified in above Scheme 8. The reaction of the compound represented by the formula (V) with the compound represented by the formula (III) or (VII) in this case can be carried out under similar conditions to those in the case of reacting the compound represented by the formula (IV) or the salt thereof with the compound represented by the formula (III-2) in above Scheme 5.

Scheme 8

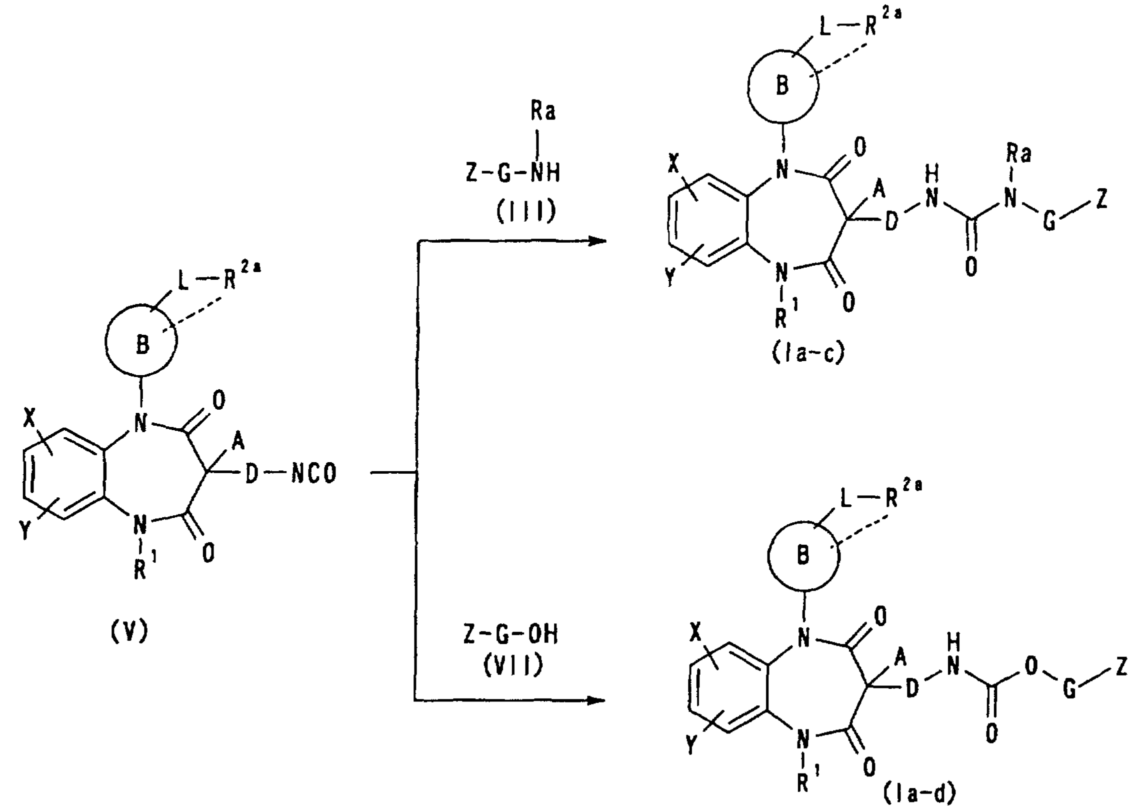


[in Scheme 8, the symbols have the same meanings as described above.]

Among the compounds represented by the formula (I) or the salts thereof, the compound represented by the formula (Ia-f) or the salt thereof can be produced by reacting the compound represented by the formula (IIa) or the salt thereof with the compound represented by the formula (VII) or the salt thereof according to the method exemplified in following Scheme 9.

Scheme 9

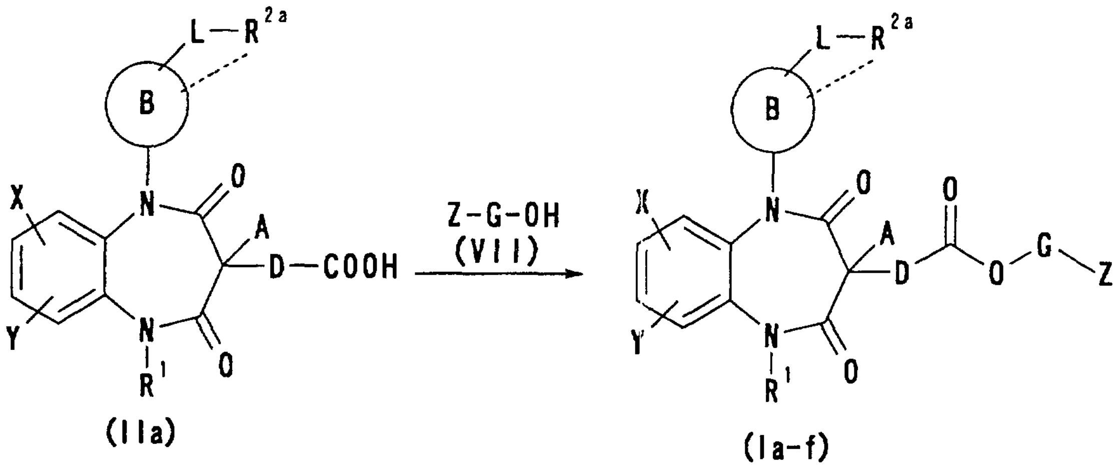


[in Scheme 9, the symbols have the same meanings as described above.]

For example, the compound can be produced by reacting the compound represented by the formula (IIa) or the salt thereof with the compound represented by the formula (VII) or the salt thereof in a solvent, optionally in the presence of a base using a condensing agent. The solvents to be used include, for example, ether-type solvents (e.

g., diethyl ether, tetrahydrofuran, dioxane, etc.), halogen-type solvents (e.g., dichloromethane, dichloroethane, chloroform, carbon tetrachloride, etc.), acetonitrile, dimethylformamide, and the like. The bases to be used include triethylamine, 4-dimethylaminopyridine, triethylenediamine, tetramethylethylenediamine, and the like. The condensing agents to be used include, for example, condensing agents which are used for peptide synthesis, concretely, dicyclohexylcarbodiimide, diethyl cyanophosphate, 1-ethyl-3-(3-dimethylaminopropyl)carbodiimide, and the like. In the reaction, relative to 1 mol of the compound represented by the formula (II) or the salt thereof, the compound represented by the formula (VII) or the salt thereof is used in an amount of 0.5 to 2 molar equivalents, preferably 1 to 1.2 molar equivalents and the condensing agent is used in an amount of 0.5 to 5 molar equivalents, preferably 1 to 2 molar equivalents. At that time, the reaction temperature is from 0 to 100°C, preferably 20 to 50°C, and the reaction time is from 0.5 to 24 hours, preferably 1 to 5 hours.

Among the compounds represented by the formula (I) or the salts thereof, the compound represented by the formula (Ia-g) or the salt thereof can be produced by reacting the compound represented by the formula (VIII) or the salt thereof with the compound represented by the formula (IX) or the salt thereof according to the method exemplified in following Scheme 10.

Scheme 10

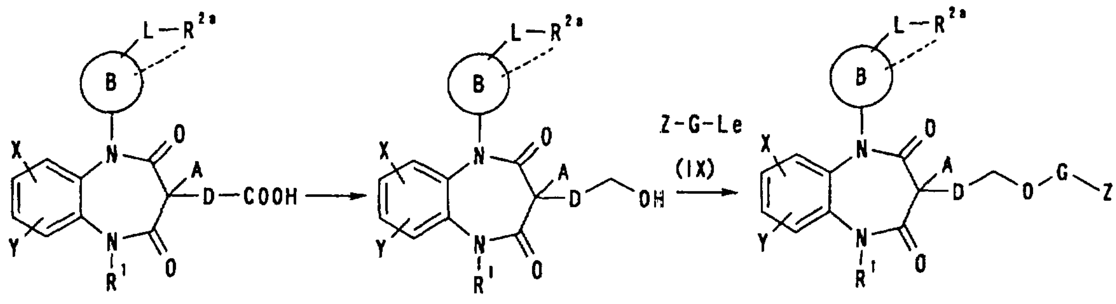


(IIa) (VIII) (Ia-g)

[in Scheme 10, the symbols have the same meanings as described above.]

The compound represented by the formula (VIII) or the salt thereof can be produced by converting the compound represented by the formula (IIa) or the salt thereof with ethyl chlorocarbonate or the like to form a mixed acid anhydride, and treating it with a metal hydrogen complex compound (e.g., lithium aluminum hydride, sodium aluminum hydride, sodium borohydride, etc.) in a solvent, for example, a protic solvent (e.g., methanol, ethanol, propanol, butanol, etc.) or an aprotic solvent (e.g., ethyl ether, tetrahydrofuran, dioxane, etc.). Relative to 1 mol of the compound represented by the formula (IIa) or the salt thereof, the metal hydrogen complex compound is used in an amount of 0.3 to 5 molar equivalents, preferably 0.5 to 2 molar equivalents. At that time, the reaction temperature is from -20 to 100°C, preferably 0 to 20°C and the reaction time is from 0.5 to 10 hours, preferably 1 to 3 hours.

In the reaction of the compound represented by the formula (VIII) or the salt thereof with the compound represented by the formula (IX) or the salt thereof, the solvents to be used include, for example, aprotic solvents (e.g., ethyl ether, tetrahydrofuran, dioxane, acetonitrile, dimethylformamide, etc.). Optionally, for example, an inorganic base (e.g., sodium hydrogen carbonate, potassium hydrogen carbonate, sodium carbonate, potassium carbonate, etc.), an organic base (e.g., triethylamine, 4-dimethylaminopyridine, triethylenediamine, tetramethylethylenediamine, etc.), sodium hydride, cesium fluoride, or the like may be used. In the reaction, relative to 1 mol of the compound represented by the formula (VIII) or the salt thereof, the compound represented by the formula (IX) or the salt thereof is used in an amount of 0.5 to 5 molar equivalents, preferably 1 to 2 molar equivalents. At that time, the reaction temperature is from 0 to 200°C, preferably 20 to 100°C and the reaction time is from 10 minutes to 5 hours, preferably 30 minutes to 2 hours.

Among the compounds represented by the formula (I) or the salts thereof, the compound represented by the formula (Ia-h) or (Ia-i) or the salt thereof can be produced by reacting the compound represented by the formula (X) or the salt thereof with the compound represented by the formula (VII) or (XI) or the salt thereof according to the method exemplified in following Scheme 11.

Scheme 11

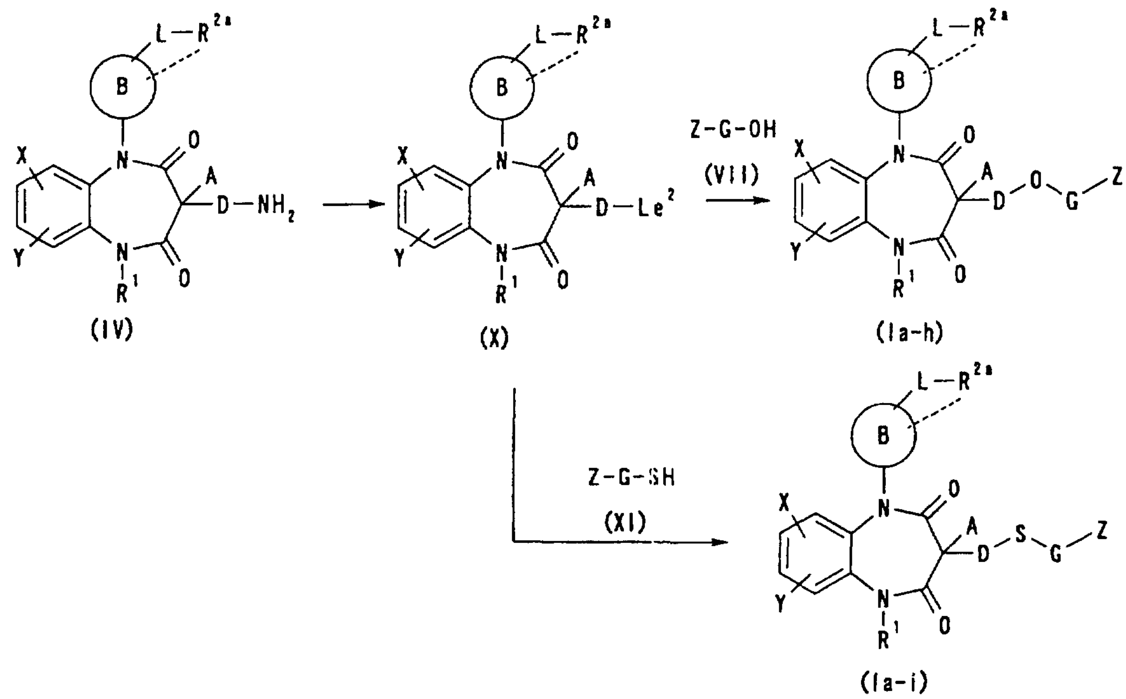


(IV) (X) (Ia-h) (Ia-i)

[in Scheme 11, $Le^2$ represents a halogen (e.g., chlorine, bromine, iodine, etc.) and other symbols have the same meanings as described above.]

The compound represented by the formula (X) or the salt thereof can be produced by diazotizing the compound represented by the formula (IV) or the salt thereof in, for example, hydrochloric acid, hydrobromic acid or hydroiodic acid using sodium nitrite in an amount of 1 to 5 molar equivalents, preferably 1 to 3 molar equivalent relative to the compound represented by the formula (IV) or the salt thereof, followed by heating. At that time, the reaction temperature is from 20 to 200°C, preferably 50 to 100°C and the reaction time is from 5 minutes to 2 hours, preferably 15 to 30 minutes. The reaction of the compound represented by the formula (X) or the salt thereof with the compound represented by the formula (VII) or (XI) or the salt thereof is carried out under similar conditions to those in the reaction of the compound represented by the formula (VIII) or the salt thereof with the compound represented by the formula (IX) or the salt thereof in the case of producing the compound represented by the formula (Ia-g) or the salt thereof.

Among the compounds represented by the formula (I) or the salts thereof, the compound represented by the formula (Ia-j) or the salt thereof can be produced by oxidizing the compound represented by the formula (Ia-i) or the salt thereof as shown in following Scheme 12.

Scheme 12

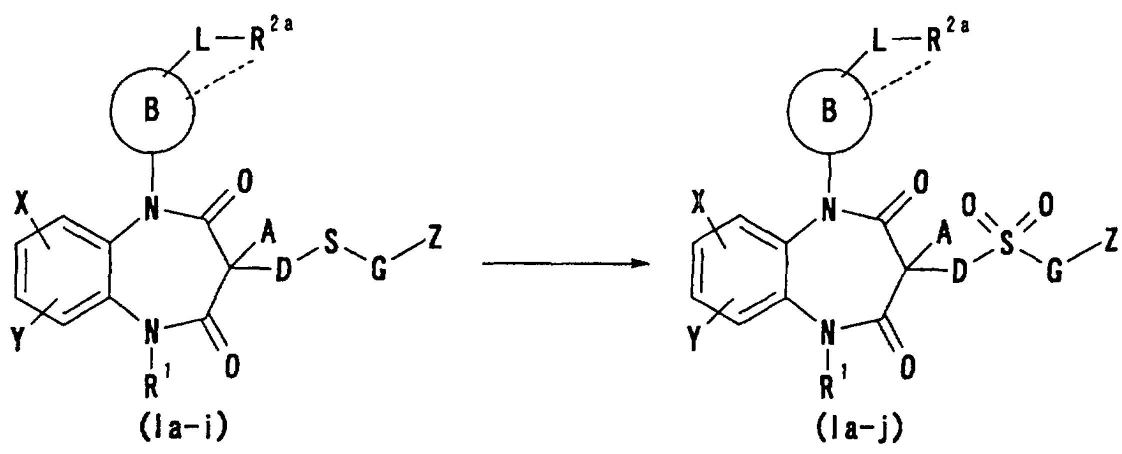

(Ia-i) (Ia-j)

[wherein, the symbols have the same meanings as described above.]

At the reaction, m-chloroperbenzoic acid is used in an amount of 1 to 5 molar equivalents, preferably 2 to 3 molar equivalent relative to 1 mol of the compound represented by the formula (Ia-i) or the salt thereof in a solvent of an ether-type solvent (e.g., diethyl ether, tetrahydrofuran, dioxane, etc.), a hydrocarbon-type solvent (e.g., benzene, toluene, hexane, heptane, etc.), a halogen-type solvent (e.g., dichloromethane, dichloroethane, chloroform, etc.), acetonitrile, dimethylformamide, or the like. At that time, the reaction temperature is from 0 to 100°C, preferably 0 to 30°C and the reaction time is from 1 to 10 hours, preferably 1 to 2 hours.

The compound represented by the formula (I) or the salt thereof and the compound represented by the formula (Ib) or the salt thereof can be produced by removing the protective group of the compound represented by the formula (Ia) or the salt thereof according to the per se known method. Also, the compound represented by the formula (I) or the salt thereof can be produced by reacting the compound represented by the formula (Ib) or the salt thereof with the compound represented by the formula (XII) or (XIII) or the salt thereof.

Scheme 13

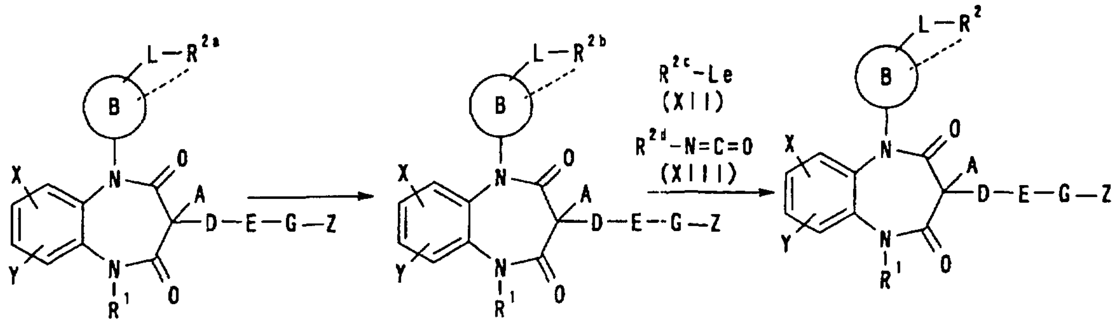


(Ia) (Ib) (I)

[wherein, $R^{2b}$ represents a deprotected $R^{2a}$, $R^{2c}$ and $R^{2d}$ each represent a hydrocarbon group which may have substituent(s), a heterocyclic group which may have substituent(s), hydrogen atom or an acyl group, and other symbols have the same meanings as described above.]

At removing the protective group, in the case that the protective group is t-butoxycarbonyl group, trityl group or benzyloxycarbonyl group, the protective group can be removed by treatment with an acid such as hydrogen chloride, hydrogen bromide, hydrochloric acid, hydrobromic acid, nitric acid, sulfuric acid, trifluoroacetic acid, or the like in a solvent of an ether-type solvent (e.g., diethyl ether, tetrahydrofuran, dioxane, etc.), an alcohol-type solvent (e.g., methanol, ethanol, propanol, etc.), a halogen-type solvent (e.g., dichloromethane, dichloroethane, chloroform, etc.), or the like. In the case that the protective group is benzyloxycarbonyl group, the protective group can be removed by hydrolysis using, for example, a palladium catalyst (e.g., metal palladium, palladium/carbon catalyst, etc.) in a solvent of an ether-type solvent (e.g., diethyl ether, tetrahydrofuran, dioxane, etc.), an alcohol-type solvent (e.g., methanol, ethanol, propanol, etc.), dimethylformamide, ethyl acetate, acetic acid, or the like. In the reaction, in the case of the acid treatment, the reaction temperature is from -20 to 100°C, preferably 0 to 30°C and the reaction time is from 0.1 to 5 hours, preferably about 0.5 to 1 hour. In the reaction, in the case of hydrolysis, the reaction temperature is from -20 to 150°C, preferably 0 to 50°C and the reaction time is from 0.1 to 10 hours, preferably 0.5 to 3 hours, and the hydrogen pressure is from 1 to 100 atm, preferably 1 to 3 atm. The catalyst is used, at that time, in an amount of 0.001 to 0.5 molar equivalent, preferably 0.01 to 0.1 molar equivalent relative to 1 mol of the compound represented by the formula (Ia) or the salt thereof.

The reaction of the compound represented by the formula (Ib) or the salt thereof with the compound represented by the formula (XII) or the salt thereof is carried out under similar conditions to those in the reaction of the compound represented by the formula (IIa-4) or the salt thereof with the compound represented by the formula $R^1$-Le or the salt thereof in above Scheme 2. The reaction of the compound represented by the formula (Ib) or the salt thereof with the compound represented by the formula (XIII) or the salt thereof is carried out under similar conditions to those in the reaction of the compound represented by the formula (IV) or the salt thereof with the compound represented by the formula (III-2) or the salt thereof to produce the compound represented by the formula (Ia-c) or the salt thereof in above Scheme 5.

The compound represented by the formula (IIa-2) or the salt thereof in above Scheme 2 can be, as exemplified in following Scheme 14, produced by combining the compound represented by the formula (IIb-1) or the salt thereof with a protective group according to the method known in the field of organic syntheses, or by converting the substituent $X^a$ into the substituent $NH_2$ according to the method known in the field of organic syntheses. The compound represented

by the formula (IIb-1) or the salt thereof can be produced from the compound represented by the formula (IIb-2) or the salt thereof by converting the substituent $X^a$ into the substituent $NH_2$ according to the method known in the field of organic syntheses. The compound represented by the formula (IIb-2) or the salt thereof can be produced from the compound represented by the formula (IIb-3) or the salt thereof by converting the substituent $R^{2e}$ into the substituent $R^{2b}$ according to the method known in the field of organic syntheses. The compound represented by the formula (IIb-4) or the salt thereof can be produced from the compound represented by the formula (IIb-3) or the salt thereof by converting the substituent $R^{2e}$ into the substituent $R^{2a}$ according to the method known in the field of organic syntheses.

Scheme 14

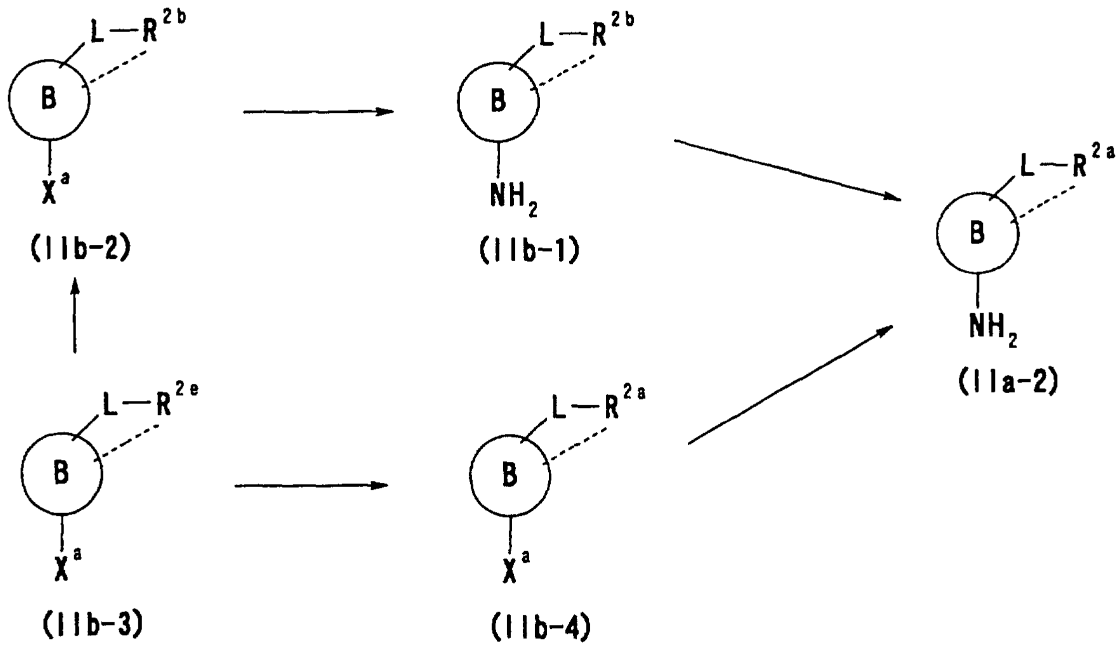

(IIb-2) (IIb-1) (IIa-2) (IIb-3) (IIb-4)

[wherein, $R^{2b}$ represents a deprotected $R^{2a}$, $R^{2e}$ represents a substituent(s) which can be converted into $R^{2a}$ or $R^{2b}$, $X^a$ represents a substituent which can be converted into $NH_2$, and other symbols have the same meanings as described above.]

The starting compounds and production intermediates of the invention may form salts, and they are not particularly limited as long as the reactions proceed. As the salts of these compounds, there may be used, for example, inorganic acid salts (e.g., hydrochlorides, sulfates, hydrobromides, phosphates, etc.), organic acid salts (e.g., acetates, trifluoroacetates, succinates, maleates, fumarates, propionates, citrates, tartarates, malate, lactates, oxalates, methanesulfonates, p-toluenesulfonates, etc.), alkali metal salts (e.g., sodium salts, potassium salts, etc.), alkaline earth metal salts (e.g., calcium salts, magnesium salts, etc.), organic base salts (e.g., trimethylamine salts, triethylamine salts, pyridine salts, piperidine salts, ethanolamine salts, etc.), aluminum salts, ammonium salts, and the like. Furthermore, the starting compounds and production intermediates of the invention can be isolated according to conventional methods, but may be used as starting materials for successive steps without isolation.

In each reaction of the invention described above, when a compound has amino group, carboxyl group or hydroxy group, a protective group may be introduced to each of these groups, and a target compound can be obtained by removing the protective group after the reaction, if necessary.

As the protective group for amino group, there may be used, for example, formyl, $C_{1-6}$ alkyl-carbonyl groups (e.g., acetyl, ethylcarbonyl, etc.), benzyl group, t-butyloxycarbonyl group, benzyloxycarbonyl group, 9-fluorenylmethyloxycarbonyl group, allyloxycarbonyl group, phenylcarbonyl group, $C_{1-6}$ alkyloxy-carbonyl groups (e.g., methoxycarbonyl, ethoxycarbonyl, etc.), $C_{7-10}$ aralkyl-carbonyl groups (e.g., benzylcarbonyl, etc.), trityl group, phthaloyl group, N, N-dimethylaminomethylene group, and the like. These groups may be substituted by 1 to 3 of halogen atoms (e.g., fluorine, chlorine, bromine, etc.), nitro group, and the like.

As the protective group for carboxyl group, there may be used, for example, $C_{1-6}$ alkyl groups (e.g., methyl, ethyl, propyl, isopropyl, butyl, t-butyl, etc.), phenyl group, silyl groups, benzyl group, allyl group, and the like. These groups may be substituted by 1 to 3 of halogen atoms (e.g., fluorine, chlorine, bromine, etc.), nitro group, and the like.

As the protective group for hydroxy group, there may be used, for example, methoxymethyl group, allyl group,

t-butyl group, $C_{7-10}$ aralkyl groups (e.g., benzyl, etc.), formyl group, $C_{1-6}$ alkyl-carbonyl groups (e.g., acetyl, ethylcarbonyl, etc.), benzoyl group, $C_{7-10}$ aralkyl-carbonyl groups (e.g., benzylcarbonyl, etc.), pyranyl groups, furanyl groups, trialkylsilyl groups, and the like. These groups may be substituted by 1 to 3 of halogen atoms (e. g., fluoro, chloro, bromo, etc.), $C_{1-6}$ alkyl groups (e.g., methyl, ethyl, propyl, isopropyl, butyl, t-butyl, etc.), phenyl group, $C_{7-10}$ aralkyl groups (e.g., benzyl, etc.), nitro group, and the like.

These protective groups may be removed by any per se known methods or modified methods thereof. For example, there may be used the methods using acids, bases, reduction, ultraviolet ray, hydrazine, phenylhydrazine, sodium N-methyldithiocarbamate, tetrabutylammonium fluoride, palladium acetate, and the like.

In the case that a compound is obtained in a free state in each reaction of the invention described above, it may be converted into a salt according to a conventional method, and in the case that a compound is obtained as a salt, it may be converted into free compound or other salt.

The compound (I) of the invention or the salt thereof thus obtained can be isolated and purified from the reaction solvent by any known procedures, for example, solvent exchange, concentration, extraction with solvent, fractional distillation, crystallization, recrystallization, chromatography, and the like.

Incidentally, in the case that the compound (I) of the invention or the salt thereof is present as diasteromers, conformers, etc., each of them can be isolated by ordinary separation and purification, if desired. Furthermore, in the case that the compound (I) of the invention or the salt thereof is racemic, it can be separated into d-isomer and l-isomer by ordinary optical resolution.

The compound having a regulating action of somatostatin receptor function or a prodrug thereof to be used in the invention may be the compound per se or a pharmaceutically acceptable salt thereof. In the case that the compound having a regulating action of somatostatin receptor function has an acidic group such as carboxyl group or the like, the examples of such salt include salts with inorganic bases (e.g., alkali metals such as sodium, potassium, etc.; alkaline earth metals such as calcium, magnesium, etc.; transition metals such as zinc, iron, copper, etc.; etc.), organic bases (e.g., organic amines such as trimethylamine, triethylamine, pyridine, picoline, ethanolamine, diethanolamine, triethanolamine, dicyclohexylamine, N, N'-dibenzylethylenediamine, etc.; basic amino acids such as arginine, lysine, ornithine, etc.; etc.), and the like.

In the case that the compound having a regulating action of somatostatin receptor function has a basic group such as amino group or the like, the examples of such salt include salts with inorganic acids, organic acids (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.); acidic amino acids such as aspartic acid, glutamic acid, etc.; and the like.

The prodrug of the compound having a regulating action of somatostatin receptor function to be used in the invention means a compound which is converted into the compound having a regulating action of somatostatin receptor function under physiological conditions through a reaction with an enzyme, a gastric acid, etc. in the living body, that is, a compound which is converted into the compound having a regulating action of somatostatin receptor function with enzymatic oxidation, reduction, hydrolysis, etc.; a compound which is converted into the compound having a regulating action of somatostatin receptor function through hydrolysis with gastric acid, etc. Examples of the prodrug of the compound having a regulating action of somatostatin receptor function include compounds wherein the amino group of the compound having a regulating action of somatostatin receptor function is acylated, alkylated, or phosphorylated (e.g., compounds wherein the amino group of the compound having a regulating action of somatostatin receptor function is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated, or the like); compounds wherein the hydroxy group of the compound having a regulating action of somatostatin receptor function is acylated, alkylated, phosphorylated, or borylated (e.g., compounds wherein the hydroxy group of the compound having a regulating action of somatostatin receptor function is acetylated, palmitoylated, propanoylated, pivaloylated, succinylated, fumarylated, alanylated, dimethylaminomethylcarbonylated, or the like); compounds wherein the carboxyl group of the compound having a regulating action of somatostatin receptor function is esterified or amidated (e.g., compounds wherein the carboxyl group of the compound having a regulating action of somatostatin receptor function is converted into ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); and the like. These compounds can be produced from the compound having a regulating action of somatostatin receptor function by per se known methods.

The prodrug of the compound having a regulating action of somatostatin receptor function may be a compound which is converted into the compound having a regulating action of somatostatin receptor function under physiological conditions as described in "Iyakuhin no Kaihatsu (Pharmaceutical Research and Development)", Vol. 7, Drug Design, pp. 163-198, published in 1990 by Hirokawa Publishing Co.

The compound having a regulating action of somatostatin receptor function may be in the form of either hydrate

or non-hydrate. Furthermore, the compound having a regulating action of somatostatin receptor function may be labeled with an isotope (e.g. , $^{3}H$, $^{14}C$, $^{35}S$. $^{125}I$, etc.) and the like.

The above regulation of somatostatin receptor function means an activation or inhibition of somatostatin receptor function, and the activation of somatostatin receptor function means an activation of transduction system of a somatostatin receptor. A substance having such action is capable of application as a somatostatin receptor ligand, a somatostatin receptor ligand agonist, a somatostatin receptor agonist, a co-activator agonist of a somatostatin receptor, and the like. The substance may be anything as long as it affords a response similar to the response caused by the action of a ligand on a somatostatin receptor.

Furthermore, the inhibition of somatostatin receptor function means an inhibition of transduction system of a somatostatin receptor, and a substance having such action is capable of application as a somatostatin receptor antagonist, and the like. The substance may be anything as long as it can inhibit the response caused by the action of a ligand on a somatostatin receptor.

Among the above regulating action of somatostatin receptor function, preferred is a somatostatin receptor agonistic action.

The compounds (I) of the invention or the salts thereof have low toxicity, and exhibit little adverse side effects, so that they can be used as prophylactics, diagnostic agents, or remedies for mammals (e.g., human, cattle, horse, dog, cat, monkey, mouse and rat, especially, human). The compounds (I) of the invention or the salts thereof inhibit or regulate production or secretion of a variety of hormones, growth factors and physiologically active substances. The "hormones" include, for example, growth hormone (GH), thyroid stimulating hormone (TSH), prolactin, insulin, glucagon, and the like. The "growth factors" include, for example, IGF-1 and the like. The "physiologically active substances" include, for example, vasoactive intestinal polypeptide (VIP), gastrin, glucagon-like peptide-1, amylin, substance-P, CGRP, CCK (cholecystokinin), amylase, and the like. Also, "physiologically active substances" include interleukins and cytokines such as TNF-$\alpha$, etc., and the like. Furthermore, these compounds function through various intracellular signal transduction systems with which somatostatin participates. The intracellular signal transduction systems include intracellular signal transduction systems that involves adenylate cyclase, $K^+$ channels, $Ca^{2+}$ channels, protein dephosphorylation, phospholipase C/inositol trisphosphate production systems, MAP kinase, $Na^+/H^+$ exchanger systems, phospholipase A2, transcription factors such as NF-$\kappa$B, etc. The compounds (I) of the invention or the salts thereof regulate a direct or indirect cell proliferation inhibitory action or apoptosis in which somatostatins participate. Therefore, the compounds (I) of the invention or the salts thereof are useful in regulating diseases associated with disorders of production or secretion of such hormones, growth factors, physiologically active substances and etc.; diseases associated with disorders of the above intracellular signal transduction systems (e.g., diseases associated with excess enhancement or inhibition, etc.); disorders of regulation of cell proliferation. Concretely, they can be used (1) as agents for treatment of tumors such as acromegaly, TSH-producing tumors, nonsecretory (afunctional) hypophysial tumors, ectopic ACTH (adrenocorticotrophin)-producing tumors, medullar thyroid carcinoma, VIP-producing tumors, glucagon-producing tumors, gastrin-producing tumors, insulinoma and carotinoid tumor, etc., (2) as agents for treatment of insulin-dependent or non-insulin dependent diabetes or a variety of diseases associated with them, for example, diabetic retinopathy, diabetic nephropathy, diabetic neuropathy, Down's syndrome and orthostatic hypotension, etc., (3) as agents for improvement of hyperinsulinemia or for treatment of obesity and overeating through inhibition of appetite, etc., (4) as agents for treatment of acute pancreatitis, chronic pancreatitis, pancreal/intestinal fistula, hemorrhagic ulcer, peptic ulcer, gastritis, hyperacidity, through inhibition or regulation of external secretion at digestive tracts, etc., (5) as agents for improvement of various symptoms associated with the Helicobacter pylori infection, for example, inhibitors of gastrin hypersecretion, etc., (6) as agents for inhibition of amylase secretion associated with endoscopic cholangiopancreatography, and agents for prognostic treatment of surgical operation of pancreas, etc., (7) as agents for treatment of diarrhea caused by small intestinal malabsorption, promotion of secretion or dyskinesia of the digestive tracts (for example, short bowel syndrome), diarrhea caused by the drugs for cancer chemotherapy, diarrhea caused by congenital small intestine atrophy, diarrhea caused by neuroendocrine tumors such as VIP-producing tumors, etc., diarrhea caused by AIDS, diarrhea caused by graft versus host reaction associated with bone marrow transplantation, diarrhea caused by diabetes, diarrhea caused by celiac plexus blocking, diarrhea caused by systemic sclerosis and diarrhea caused by eosinophilia, etc., (8) as agents for treatment of dumping syndrome, irritable colitis, Crohn disease and inflammatory bowel disease, etc., (9) as agents for treatment of various cancers having growth-dependency on insulin, IGF-1 or other growth factors, or tumors or cancers due to the disorders of inhibiting cell growth caused by other reasons (e.g., thyroid cancer, large bowel cancer, breast cancer, prostatic cancer, small cell lung cancer, non-small cell cancer, pancreatic cancer, stomach cancer, cholangiocarcinoma, hepatic cancer, vesical cancer, ovarian cancer, uterine cancer, melanoma, osteosarcoma, chondrosarcoma, malignant pheochromocytoma, neuro-blastoma, brain tumors, thymoma, renal cancers), leukemia (e.g., leukemia of basophilic leukocyte, chronic lymphocytic leukemia, chronic myeloid leukemia, Hodgkin disease, and non-Hodgkin lymphoma) (agents for treatment of these cancers can be used solely or in combination with other anticancer agents such as Tamoxifen, LHRH agonists, LHRH antagonists, interferon-$\alpha$, interferon-$\beta$, interferon-$\gamma$, interleukin-2, etc.), (10) as agents for prevention and treatment of hypertrophic

cardiomyopathy, arteriosclerosis, valvular disease, myocardiac infarction (especially, myocardiac infarction post percutaneous transluminal coronary arterioplasty) and reangioplasty, etc., (11) as agents for treatment of hemorrhage of esophageal varicosis, cirrhosis and peripheral blood vessel disorders, etc., (12) as agents for treatment of diseases associated with general or local inflammation, for example, polyarteritis, rheumatoid arthritis, psoriasis, sunburn, eczema and allergy (e.g., asthma, atopic dermatitis, allergic rhinitis, etc.) because they inhibit or regulate the secretion of physiologically active substances acting on the immune system (e.g., Substance P, tachykinin, cytokines, etc.), (13) as agents for treatment of dementia (e.g., Alzheimer disease, Alzheimer-type senile dementia, vascular/multiinfarct dementia, etc.), headache, migraine, schizophrenia, epilepsy, depression, generalized anxiety disorder, sleep disorder, and multiple sclerosis, etc., because they influence the production and secretion of nerve regulating factors, (14) as analgesics, (15) as agents for treatment of acute bacterial meningitis, acute virus encephalitis, adult respiratory distress syndrome, bacterial pneumonia, severe systemic mycotic infection, tuberculosis, spinal damage, bone fracture, hepatic failure, pneumonia, alcoholic hepatitis, virus A hepatitis, virus B hepatitis, virus C hepatitis, AIDS infection, human papilloma virus infection, influenza infection, metastasis of cancer, multiple myeloma, osteomalacia, osteoporosis, bone Paget disease, oesophagitis, nephritis, renal failure, sepsis, septic shock, hypercalcemia, hypercholesterolemia, hyperglyceridemia, hyperlipemia, systemic lupus erythematosus, transient ischemic attach, alcoholic hepatitis, etc., (16) for cure of organ transplantation, burns, trauma, alopecia, etc., (17) for oculopathy (e.g., glaucoma, etc.), (18) for imaging of tumors having somatostatin receptors after incorporating a radioactive substance (e.g., $^{125}I$, $^{111}In$, etc.) to the present compound either directly or through a suitable spacer, and (19) for targeting of tumors having a somatostatin receptor by incorporating an anti-cancer drug to the present compound directly or through a suitable spacer.

The compounds (I) of the invention or the salts thereof may be used as bulk itself but usually be formulated into pharmaceutical preparations together with a suitable amount of carrier for pharmaceutical preparation according to ordinary methods. The "carrier for pharmaceutical preparation" includes, for example, excipients (e.g., calcium carbonate, kaolin, sodium hydrogen carbonate, lactose, D-mannitol, starches, crystalline cellulose, talc, granulated sugar, porous substances, etc.), binders (e.g., dextrin, gums, α-starch, gelatin, hydroxypropyl cellulose, hydroxypropylmethyl cellulose, pullulan, etc.), thickening agents (e.g., natural gums, cellulose derivatives, acrylic acid derivatives, etc.), disintegrators (e.g., carboxymethyl cellulose, croscarmellose sodium, crospovidone, low-substitution hydroxypropyl cellulose, partial α-starch, etc.), solvents (e.g., water for injections, alcohol, propylene glycol, macrogol, sesame oil, corn oil, etc.), dispersants (e.g., Tween 80, HCO60, polyethylene glycol, carboxymethyl cellulose, sodium alginate, etc.), solubilizers (e.g., polyethylene glycol, propylene glycol, D-mannitol, benzyl benzoate, ethanol, trisaminomethane, triethanolamine, sodium carbonate, sodium citrate, etc.), suspending agents (e.g., stearyl triethanolamine, sodium lauryl sulfate, benzalkonium chloride, polyvinyl alcohol, polyvinylpyrrolidone, hydroxyethyl cellulose, etc.), pain-reducing agents (e.g., benzyl alcohol, etc.), isotonizing agents (e.g., sodium chloride, glycerin, etc.), buffers (e.g., phosphates, acetates, carbonates, citrates, etc.), lubricants (e.g., magnesium stearate, calcium stearate, talc, starch, sodium benzoate, etc.), colorants (e.g., tar pigments, caramel, iron sesquioxide, titanium oxide, riboflavins, etc.), tasting agent (e.g., sweeteners, flavors, etc.), stabilizers (e.g., sodium sulfite, ascorbic acid, etc.), preservatives (e.g., parabens, sorbic acid, etc.), and the like. The preventing or treating agent for medical use of the invention which may contain the above carrier for pharmaceutical preparation contains a necessary amount of the compound (I) of the invention or pharmaceutically acceptable salt thereof for preventing or treating various diseases. The content of the compound (I) of the invention or pharmaceutically acceptable salt thereof in the preparation of the invention usually ranges from 0.1 to 100% by weight based on the total amount of the preparation. Concrete examples of the formulation include, for example, tablets (including sugar-coated tablets, film-coated tablets), pills, capsules (including microcapsules), granules, fine granules, powders, drip injections, syrups, emulsions, suspensions, injections, inhalants, ointments, suppositories, troches, poultices, and the like. These preparations are prepared according to ordinary methods (e.g., the methods described in the Japanese Pharmacopoeia, 12th Correction).

The following will illustrate methods for preparing major preparations, but the methods are, needless to say, not limited to them.

(1) Tablets

The compound of the invention is homogeneously mixed as such or together with an excipient, a binder, an disintegrator, or other suitable additive(s), and is shaped into granules by an appropriate method. Thereafter, the granules are mixed with a lubricant, etc. and compressed to form tablets. Then, for the purpose of masking the taste, enteric dissolution, or sustained release, the tablets may be optionally coated with a suitable coating agent.

(2) Injections

A determined amount of the compound of the invention is dissolved, suspended or emusified into water for injection or the like optionally together with a stabilizer, a solubilizer, a suspending agent, an emulsifier, a buffer, a

preservative, etc. to make the volume a predetermined one.

(3) Suppositories

Using an oil and fat base material, a water-soluble base material, or other suitable material as the base material, the compound of the invention is added thereto after optional addition of an emulsifier, a suspending agent, etc. The mixture is homogeneously mixed, and then it is shaped into a suitable form.

(4) Capsules

The one obtained by mixing homogeneously the compound of the case and suitable additive(s) such as an excipient, etc., granulating according to an appropriate method, or coating the resulting granules with a suitable coating agent is filled in capsule shells as such or lightly.

The pharmaceutical preparations of the invention exhibit low toxicity and high safety, and have an excellent regulating action of somatostatin receptor function, so that they are useful as agents for preventing or treating the above-described diseases.

The amount of the compound of the invention to be used in the above pharmaceutical preparations varies depending on the compound to be selected, the animal species selected as subject for administration, the administration times, etc., but the compound exhibits effectiveness over a wide range. For example, for treating acromegaly, diabetic complication, intractable diarrhea, diabetes or obesity of adults, the dose in the case of oral administration of the pharmaceutical preparations of the invention may be usually 0.001 to 20 mg/kg-body weight, preferably 0.2 to 3 mg/kg-body weight per one day, in terms of the active amount of the compound (I) of the invention. In the case of parenteral administration, combined preparation with other active ingredient(s) or administration in combination with other pharmaceutical preparation(s), the dose may be less than the above amount. However, actual dose of the compound varies depending on the situations such as the selected compound, various preparation forms, the age, body weight and sex of the patient, the degree of the disease, the administration route employed, the term and interval of the administration, etc., and can be changed at any time according to doctor's decision.

The administration route of the above pharmaceutical preparations depends on various situations and is not particularly limited. For example, they can be administered either orally or parenterally. The "parenterally" used herein includes intravenous, intramuscular, subcutaneous, nasal, rectal, vaginal and intraperitoneal administration, and the like.

Although the administering term and interval of the above pharmaceutical preparation varies depending on various situations and decided by doctor at any time, there may be mentioned divided administration, continuous administration, intermittent administration, large amount administration during short period of time, repeated administration, and the like. For example, in the case of oral administration, it is desirable to administer the preparation once to several times a day (particularly, once to three times a day), dividedly. In addition, it is also possible to administer an intravenous drip injection over a long period of time.

The present invention will be explained in more detail with reference to following Examples and Experimental Examples. These are mere examples and are not intended to restrict the present invention, and may be modified within the range of not deviating from the scope of the invention. The meanings of the abbreviations used in Reference Examples and Examples are as follows:

s: singlet, d: doublet, t: triplet, q: quartet, dd: double doublet, dt: double triplet, m: multiplet, bs: broad singlet, J: coupling constant, room temperature: 0 to 30°C.

Example 1

N-(2-Fluorobenzyl)-1-(4-aminomethylphenyl)-5-(4-biphenylmethyl)-2,4-dioxo-2,3,4,5-tetrahydro-1H-1,5-benzodiazepine-3-acetamide hydrochloride

(1) tert-Butyl (4-aminobenzyl)carbamate

Di-tert-butyl dicarbonate (43.9 g, 199 mmol) was added dropwise to an ice-cooled stirred tetrahydrofuran (400 ml) solution of 4-aminobenzylamine (24.3 g, 199 mmol). The resulting reaction mixture was stirred at 0°C for 1 hour. The reaction solution was concentrated under reduced pressure and the residue was diluted with ethyl acetate. After washing with water, the solution was dried over anhydrous magnesium sulfate. After the removal of the solvent by evaporation under reduced pressure, the residue was crystallized from hexane and the crystals were collected by filtration to give tert-butyl (4-aminobenzyl)carbamate (41.9 g, 94.8%) as crystals.
Melting point 69-70°C.

$^1$H-NMR($CDCl_3$)δ: 1.47(9H, s), 3.62(2H, bs), 4.19(2H, d, J=5.8Hz), 4.73(1H, bs), 6.65(2H, d, J=8.6Hz), 7.08(2H, d, J=8.6Hz).

(2) tert-Butyl [4-(2-nitrophenylamino)benzyl]carbamate

A mixture of tert-butyl (4-aminobenzyl)carbamate (89.2 g, 401 mmol), o-fluoronitrobenzene (56.7 g, 401 mmol) and potassium carbonate (55.4 g, 401 mmol) was stirred at 140°C for 2 hours under nitrogen atmosphere. After cooling, the reaction mixture was diluted with ethyl acetate, washed with water, and after drying over anhydrous magnesium sulfate, concentrated under reduced pressure. The residue was purified by a silica gel column chromatography to give tert-butyl [4-(2-nitrophenylamino)benzyl]carbamate (36 g, 26%) as crystals.

Melting point 121-123°C.
Elemental analysis for $C_{18}H_{21}N_3O_4$;
Calcd.: C, 62.96; H, 6.16; N, 12.24.
Found: C, 62.71; H, 6.05; N, 12.12.
$^1$H-NMR($CDCl_3$)δ: 1.49(9H, s), 4.34(2H, d, J=6Hz), 4.92(1H, bs), 6.78(1H, t, J=7.2Hz), 7.18-7.37(6H, m), 8.21 (1H, d, J=8. 6Hz), 9.47(1H, bs).

(3) tert-Butyl [4-(2-aminophenylamino)benzyl]carbamate

Palladium supported on carbon (10%, 4 g) was added to an ethanol solution of tert-butyl [4-(2-nitrophenylamino)benzyl]carbamate. (36 g, 105 mmol). The resulting mixture was subjected to hydrogenation for 4 hours under the conditions of ambient temperature and normal pressure. The catalyst was removed by filtration and the filtrate was concentrated under reduced pressure. The residual solid was recrystallized from hexane-ethyl acetate to give tert-butyl [4-(2-aminophenylamino)benzyl]carbamate (29.5 g, 89.9%) as crystals.

Melting point 117-119°C.
Elemental analysis for $C_{18}H_{23}N_3O_2$;
Calcd.: C, 68.88; H, 7.40; N, 13.41.
Found: C, 69.09; H, 7.55; N, 13.48.
$^1$H-NMR($CDCl_3$)δ: 1.46(9H, s). 3.78(2H, bs), 4.21(2H, d, J=5.4Hz), 4.73(1H, bs), 5.19(1H, bs), 6.69-7.15(8H, m).

(4) tert-Butyl [4-[2-(4-biphenylmethylamino)phenylamino]benzyl]carbamate

Acetic acid (5.4 ml, 94 mmol) was added to an ethanol solution (500 ml) of tert-butyl [4-(2-aminophenylamino) benzyl]carbamate (29.5 g, 94.1 mmol) and 4-phenylbenzaldehyde. The resulting mixture was stirred at 0°C for 30 minutes and then sodium cyanoborohydride (7.1 g, 117 mmol) was added thereto. Thereafter, the mixture was stirred at 0°C for 1 hour and at room temperature for 30 minutes. Then, the reaction solution was poured into water and extracted with ethyl acetate. After washing with water, the extract solution was dried over anhydrous magnesium sulfate and concentrated under reduced pressure. The residue was purified by a silica gel column chromatography to give tert-butyl [4-[2-(4-biphenylmethylamino)phenylamino]benzyl]carbamate (40.5 g, 90%) as an oily substance.
$^1$H-NMR($CDCl_3$)δ: 1.45(9H, s), 4.21(2H, d, J=5.4Hz), 4.39(2H, s), 4.75(1H, bs), 5.12(1H, bs), 6.68-7.59(16H, m).

(5) 5-(4-Biphenylmethyl)-1-(4-tert-butoxycarbonylaminomethylphenyl)-2,4-dioxo-2,3,4,5-tetrahydro-1H-1,5-benzodiazepine

Potassium carbonate (14.0 g, 101 mmol) was added to an ice-cooled stirred tetrahydrofuran (500 ml) solution of tert-butyl [4-[2-(4-biphenylmethylamino)phenylamino]benzyl]carbamate (40.5 g, 84.4 mmol). Then, a tetrahydrofuran solution (50 ml) of malonyl dichloride (14.1 g, 101 mmol) was added dropwise. The resulting mixture was stirred at 0°C for 1 hour and then at room temperature for 1 hour. The reaction solution was diluted with ethyl acetate, and after washing with water, dried over anhydrous magnesium sulfate. The solution was concentrated under reduced pressure and the residue was purified by a silica gel column chromatography to give 5-(4-biphenylmethyl)-1-(4-tert-butoxycarbonylaminomethylphenyl)-2,4-dioxo-2,3,4,5-tetrahydro-1H-1,5-benzodiazepine (5.9 g, 13%) as crystals. $^1$H-NMR ($CDCl_3$)δ: 1.46(9H, s), 3.54(2H, s), 4.32(2H, d, J=5.8Hz), 4.95(1H, bs), 6.89-7.34(8H, m), 9.27(1H, bs).

(6) Methyl 5-(4-biphenylmethyl)-1-(4-tert-butoxycarbonylaminomethylphenyl)-2,4-dioxo-2,3,4,5-tetrahydro-lH-1,5-benzodiazepine-3-acetate

To a stirred N,N-dimethylformamide (120 ml) solution of 5-(4-biphenylmethyl)-1-(4-tert-butoxycarbonylaminomethylphenyl)-2,4-dioxo-2,3,4,5-tetrahydro-1H-1,5-benzodiazepine (5.9 g, 10.8 mmol) was added 60% oily sodium hydride (1.3 g, 32.4 mmol). After stirring at room temperature for 5 minutes, methyl bromoacetate (2.0 ml, 21.6 mmol) was added thereto. The resulting mixture was stirred at room temperature for 1 hour and then, 60% oily sodium hydride (1.3 g, 32.4 mmol) and methyl bromoacetate (3.1 ml, 32.4 mmol) were added portionwise thereto over a period of 2 hours. The reaction solution was diluted with ethyl acetate and, after washing with water, was dried over anhydrous magnesium sulfate. After concentrating under reduced pressure, the residue was purified by a silica gel column chromatography to give methyl 5-(4-biphenylmethyl)-1-(4-tert-butoxycarbonylaminomethylphenyl)-2,4-dioxo-2,3,4,5-tetrahydro-1H-1,5-benzodiazepine-3-acetate (5.6 g, 84%) as amorphous solid.
$^1$H-NMR($CDCl_3$)δ: 1.44 (9H, s), 3.18 (2H, dd, J=1.6Hz,7Hz), 3.71 (3H, s), 3.96 (1H, t, J=7Hz), 4.22 (2H, d, J=5.6Hz), 4.74 (1H, bs), 4.78 (1H, d, J=14.8Hz), 5.84 (1H, d, J=14.8Hz), 6.60-7.59 (17H, m).

(7) 5-(4-Biphenylmethyl)-1-(4-tert-butoxycarbonylaminomethylphenyl)-2,4-dioxo-2,3,4,5-tetrahydro-1H-1,5-benzodiazepine-3-acetic acid

To a stirred tetrahydrofuran (50 ml) and methanol (150 ml) solution of methyl 5-(4-biphenylmethyl)-1-(4-tert-butoxycarbonylaminomethylphenyl)-2,4-dioxo-2,3,4,5-tetrahydro-1H-1,5-benzodiazepine-3-acetate (5.6 g, 9.0 mmol) was added IN sodium hydroxide aqueous solution (40 ml, 40 mmol). The resulting mixture was stirred at 60°C for 2 hours. After cooling of the reaction solution, water and potassium hydrogen sulfate (5.4 g, 40 mmol) were added thereto. The mixture was extracted with ethyl acetate and, after washing with water, dried over anhydrous magnesium sulfate. The extract was concentrated under reduced pressure to give 5-(4-biphenylmethyl)-1-(4-tert-butoxycarbonylaminomethylphenyl)-2,4-dioxo-2,3,4,5-tetrahydro-1H-1,5-benzodiazepine-3-acetic acid (3.5 g, 64%) as amorphous solid.
$^1$H-NMR($CDCl_3$)δ: 1.44(9H, s), 3.21(2H, d, J=7Hz), 3.92(2H, t, J=7Hz), 4.21(2H, d, J=5.6Hz), 4.76(1H, bs), 4.78(1H, d, J=14.6Hz), 5.82(1H, d, J=14.6Hz), 6.60-7.59(17H, m).

(8) N-(2-Fluorobenzyl)-5-(4-biphenylmethyl)-1-(4-tert-butoxycarbonylaminomethylphenyl)-2,4-dioxo-2,3,4,5-tetrahydro-1H-1,5-benzodiazepine-3-acetamide

To an ice-cooled stirred N,N-dimethylformamide (1 ml) solution of 5-(4-biphenylmethyl)-1-(4-tert-butoxycarbonylaminomethylphenyl)-2,4-dioxo-2,3,4,5-tetrahydro-1H-1,5-benzodiazepine-3-acetic acid (0.13 g, 0.22 mmol) were added 2-fluorobenzylamine (0.028 ml, 0.24 mmol), diethyl cyanophosphate (0.04 ml, 0.26 mmol) and triethylamine (0.037 ml, 0.26 mmol). The resulting mixture was stirred at room temperature for 48 hours. The reaction solution was poured into water and extracted with ethyl acetate. The extract solution was washed with water and dried over anhydrous magnesium sulfate, followed by concentration under reduced pressure. The residue was purified by a silica gel column chromatography to give N-(2-fluorobenzyl)-5-(4-biphenylmethyl)-1-(4-tert-butoxycarbonylaminomethylphenyl)-2,4-dioxo-2,3,4,5-tetrahydro-lH-1,5-benzodiazepine-3-acetamide (135 mg, 86%) as amorphous solid.
$^1$H-NMR($CDCl_3$)δ: 1.44(9H, s), 3.03(2H, d, J=7,0Hz), 4.07(2H, d, J=7.0Hz), 4.22(2H, d, J=5.8Hz), 4.40-4.61(2H, m), 4.71(1H, bs), 4.77(1H, d, J=14.6Hz), 5.82(1H, d, J=14.6Hz), 6.31(1H, t, J=5.8Hz), 6.59-7.59(21H, m).

(9) N-(2-Fluorobenzyl)-1-(4-aminomethylphenyl)-5-(4-biphenylmethyl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-5-(4-biphenylmethyl)-1-(4-tert-butoxycarbonylaminomethylphenyl)-2,4-dioxo-2,3,4,5-tetrahydro-1H-1,5-benzodiazepine-3-acetamide (165 mg, 0.23 mmol). The resulting mixture was stirred at room temperature for 30 minutes and then concentrated under reduced pressure to give N-(2-fluorobenzyl)-5-(4-biphenylmethyl)-1-(4-aminomethylphenyl)-2,4-dioxo-2,3,4,5-tetrahydro-1H-1,5-benzodiazepine-3-acetamide hydrochloride (140 mg, 93%) as amorphous solid.

Elemental analysis for $C_{38}H_{34}N_4O_3ClF \cdot 2H_2O$;
Calcd.: C, 66.61; H, 5.59; N, 8.18.
Found: C, 66.37; H, 5.66; N, 7.93.
$^1$H-NMR(DMSO-$d_6$)δ: 2.914(2H, t, J=7.0Hz), 3.92-3.99(3H, m), 4.30(2H, d, J=5.4Hz), 5.04(1H, d, J=15.2Hz), 5.66 (1H, d, J=15.2Hz), 6.78-7.86(21H, m), 8.41(3H, bs), 8.63(1H, t, J=6.2Hz).

Syntheses in Examples 2 and 3 were carried out in a similar manner to (7) of Example 1.

Example 2

N-(2-Chlorobenzyl)-5-(4-biphenylmethyl)-1-(4-aminomethylphenyl)-2,4-dioxo-2,3,4,5-tetrahydro-1H-1,5-benzodiazepine-3-acetamide hydrochloride

(1) N-(2-Chlorobenzyl)-5-(4-biphenylmethyl)-1-(4-tert-butoxycarbonylaminomethylphenyl)-2,4-dioxo-2,3,4,5-tetrahydro-1H-1,5-benzodiazepine-3-acetamide

To an ice-cooled stirred N,N-dimethylformamide (2 ml) solution of 5-(4-biphenylmethyl)-1-(4-tert-butoxycarbonylaminomethylphenyl)-2,4-dioxo-2,3,4,5-tetrahydro-1H-1,5-benzodiazepine-3-acetic acid (0.2 g, 0.33 mmol) were added o-chlorobenzylamine (51 mg, 0.36 mmol), diethyl cyanophosphate (0.060 ml, 0.4 mmol) and triethylamine (0.055ml, 0.4 mmol). The resulting mixture was stirred at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-chlorobenzyl)-5-(4-biphenylmethyl)-1-(4-tert-butoxycarbonylaminomethylphenyl)-2,4-dioxo-2,3,4,5-tetrahydro-1H-1,5-benzodiazepine-3-acetamide (150 mg, 65%) as amorphous solid.
$^1$H-NMR($CDCl_3$)δ: 1.44(9H, s), 3.04(2H, d, J=6.6Hz), 4.07(1H, t, J=6.6Hz), 4.21(2H, d, J=5.8Hz), 4.45-4.56(2H, m), 4.73(1H. bs), 4.77(1H, d, J=14.6Hz), 5.81(1H, d, J=14.6Hz), 6.41(1H. t, J=5Hz), 6.57(2H, d, J=8.4Hz), 6.80(1H, d, J=8.4Hz), 7.04-7.55(18H, m).

(2) N-(2-Chlorobenzyl)-1-(4-aminomethylphenyl)-5-(4-biphenylmethyl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-chlorobenzyl)-5-(4-biphenylmethyl)-1-(4-tert-butoxycarbonylaminomethylphenyl)-2,4-dioxo-2,3,4,5-tetrahydro-1H-1,5-benzodiazepine-3-acetamide (160 mg, 0.22 mmol). The resulting mixture was stirred at room temperature for 1 hour and then concentrated under reduced pressure. The residue was solidified from ethyl ether to give N-(2-chlorobenzyl)-1-(4-aminomethylphenyl)-5-(4-biphenylmethyl)-2,4-dioxo-2,3,4,5-tetrahydro-1H-1,5-benzodiazepine-3-acetamide hydrochloride (140 mg, 97%) as amorphous solid.
$^1$H-NMR(DMSO-$d_6$)δ: 2.65-2.84(2H, m), 3.71-3.80(2H, m), 4.11(2H, d, J=5.8Hz), 4.84(1H. d, J=15.4Hz), 5.44(1H, d, J=15.4Hz), 6.56-7.44(20H, m), 7.62(1H, d, J=7.8Hz), 8.14(3H, bs), 8.47(1H, t, J=6.2Hz).

Example 3

N-(2-Methoxybenzyl)-5-(4-biphenylmethyl)-2,4-dioxo-2,3,4,5-tetrahydro-1H-1,5-benzodiazepine-3-acetamide hydrochloride

(1) N-(2-Methoxybenzyl)-1-(4-tert-butoxycarbonylaminomethylphenyl)-5-(4-biphenylmethyl)-1-(4-tert-butoxycarbonylaminomethylphenyl)-2,4-dioxo-2,3,4,5-tetrahydro-1H-1,5-benzodiazepine-3-acetamide

To an ice-cooled stirred N,N-dimethylformamide (2 ml) solution of 5-(4-biphenylmethyl)-1-(4-tert-butoxycarbonylaminomethylphenyl)-2,4-dioxo-2,3,5-tetrahydro-1H-1,5-benzodiazepine-3-acetic acid (0.2 g, 0.33 mmol) were added o-methoxybenzylamine (50 mg, 0.36 mmol), diethyl cyanophosphate (0.060 ml, 0.4 mmol) and triethylamine (0.055 ml, 0.4 mmol). The resulting mixture was stirred at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-methoxybenzyl)-5-(4-biphenylmethyl)-1-(4-tert-butoxycarbonylaminomethylphenyl)-2,4-dioxo-2,3,5-tetrahydro-lH-1,5-benzodiazepine-3-acetamide (145 mg, 61%) as amorphous solid.
$^1$H-NMR($CDCl_3$)δ: 1.44(9H, s), 2.99(2H, d, J=6.6Hz), 3.86(3H, s), 4.06(1H, t, J=6.6Hz), 4.21(2H, d, J=6.6Hz), 4.39-4.47 (2H, m), 4.71(1H, bs), 4.74(1H, d, J=15.2Hz), 5.82(1H, d, J=15.2Hz), 6.41(1H, t, J=5Hz), 6.56(2H, d, J=8.4Hz), 6.77-7.40(19H, m).

(2) N-(2-Methoxybenzyl)-1-(4-aminomethylphenyl)-5-(4-biphenylmethyl)-2,4-dioxo-2,3,4,5-tetrahydro-lH-1,5-benzodiazepine-3-acetamide hydrochloride

A 4N ethyl acetate solution (1 ml) of hydrogen chloride was added to a stirred ethyl acetate (1 ml) solution of

N-(2-methoxybenzyl)-5-(4-biphenylmethyl)-1-(4-tert-butoxycarbonylaminomethylphenyl)-2,4-dioxo-2,3,4,5-tetrahydro-1H-1,5-benzodiazepine-3-acetamide (145 mg, 0.21 mmol). The resulting mixture was stirred at room temperature for 1 hour and then concentrated under reduced pressure. The residue was solidified from ethyl ether to give N-(2-methoxybenzyl)-5-(4-biphenylmethyl)-1-(4-aminomethylphenyl)-2,4-dioxo-2,3,4,5-tetrahydro-1H-1,5-benzodiazepine-3-acetamide hydrochloride (130 mg, 98%) as amorphous solid.
$^1$H-NMR(DMSO-$d_6$)δ: 2.89-2.96(2H, m), 3.81(3H, s), 3.96-4.06(3H, m), 4.13-4.31(3H, m), 5.05(1H, d, J=15Hz), 5.67 (1H, d, J=15Hz), 6.77-7.68(20H, m), 7.85(1H, d, J=8.4Hz), 8.30(3H, bs), 8.46(1H, t, J=5.0Hz).

Example 4

N-(2-Fluorobenzyl)-1-(4-aminomethylphenyl)-5-(phenylcarbamoylmethyl)-2,4-dioxo-2,3,4,5-tetrahydro-1H1,5-benzodiazepine-3-acetamide hydrochloride

(1) Ethyl N-[2-[4-(tert-butoxycarbonylaminomethyl)phenylamino]phenyl]malonamidat e

To an ice-cooled stirred tetrahydrofuran (100 ml) solution of tert-butyl [4-(2-aminophenylamino)benzyl]carbamate (5.00 g, 16.0 mmol) were added triethylamine (2.45 ml, 17.6 mmol) and ethyl malonyl chloride (2.25 ml, 17.6 mmol). The reaction mixture was stirred at 0°C for 1 hour and then, a tetrahydrofuran (1 ml) solution of triethylamine (0.446 ml, 3.20 mmol) and ethyl malonyl chloride (0.410 ml, 3.20 mmol) was added thereto. After stirring at 0°C for 10 minutes, the reaction mixture was poured into water and extracted with ethyl acetate. The extract solution was washed with water and then dried over anhydrous magnesium sulfate. After concentration under reduced pressure, the residue was purified by a silica gel column chromatography to give ethyl N-[2-(4-tert-butoxycarbonylaminomethyl)phenylamino] phenylmalonamate (3.70 g, 98.0%) as an oil.
$^1$H-NMR($CDCl_3$)δ: 1.27(3H, t, J=6.8Hz), 1.46(9H, s), 3.45(2H, s), 4.17(2H, q, J=6.8Hz), 4.21(2H, d, J=4.4Hz), 4.75(1H, bs), 5.84(1H, bs), 6.81(2H, d, J=8.4Hz), 7.05-7.31(5H, m), 7.77(1H, d, J=7.0Hz), 9.20(1H, bs).

(2) N-[2-(4-tert-butoxycarbonylaminomethyl)phenylamino]phenylmalonamic acid

To an ice-cooled stirred tetrahydrofuran (30 ml) and methanol (90 ml) solution of ethyl N-[2-(4-tert-butoxycarbonylaminomethyl)phenylamino]phenylmalonamate (6.60 g, 15.4 mmol) was added 1N sodium hydroxide aqueous solution (33 ml, 33 mmol). The resulting mixture was stirred at 0°C for 10 minutes and at room temperature for 3 hours. Water was added to the reaction solution and the mixture was washed with diisopropyl ether. Potassium hydrogen sulfate (4.49 g, 33 mmol) was added thereto and then the mixture was extracted with ethyl acetate. After washing with water, the extract solution was dried over anhydrous magnesium sulfate. The solution was concentrated under reduced pressure to give N-[2-(4-tert-butoxycarbonylaminomethyl)phenylamino]phenylmalonamic acid (5.98 g, 97.2%) as an oil.
$^1$H-NMR($CDCl_3$)δ: 1.45(9H, s), 3.36(2H, s), 4.16(2H, d, J=5.8Hz), 5.03(1H, bs), 6.60(1H, bs), 6.70(2H, d, J=8.4Hz), 7.01-7.25(5H, m), 7.78(1H, d, J=7.4Hz), 9.04(1H, bs).

(3) 1-(4-tert-butoxycarbonylaminomethylphenyl)-2,4-dioxo-2,3,4,5-tetrahydro-lH-1,5-benzodiazepine

To an ice-cooled stirred N,N-dimethylformamide (250 ml) solution of N-[2-(4-tert-butoxycarbonylaminomethyl) phenylamino]phenylmalonamic acid (5.88 g, 14.7 mmol) were added 4-dimethylaminopyridine (1.80 g, 14.7 mmol) and 1-ethyl-3-(3-dimethylaminopropyl)carbodiimide hydrochloride (8.47 g, 44.2 mmol). The resulting mixture was stirred at 0°C for 15 minutes and at room temperature for 18 hours. The reaction solution was poured into water and extracted with ethyl acetate. The extract solution was washed with water, a saturated sodium hydrogen carbonate aqueous solution, and water, successively, and then dried over anhydrous magnesium sulfate. After concentration under reduced pressure, the residue was purified by a silica gel column chromatography to give 1-(4-tert-butoxycarbonylaminomethylphenyl)-2,4-dioxo-2,3,4,5-tetrahydro-lH-1,5-benzodiazepine (6.11 g, 58.2%) as an oil.
$^1$H-NMR($CDCl_3$)δ: 1.46(9H, s), 3.54(2H, s), 4.33(2H, d, J=6.2Hz), 4.93(1H, t, J=6.2Hz), 6.90-7.52(8H, m), 8.93(1H, bs).

(4) 1-(4-tert-butoxycarbonylaminomethylphenyl)-5-(methoxycarbonylmethyl)-2,4-dioxo-2,3,4,5-tetrahydro-1H-1,5-benzodiazepine

To a stirred acetonitrile (100 ml) suspension of 1-(4-tert-butoxycarbonylaminomethylphenyl)-2,4-dioxo-2,3,4,5-tetrahydro-1H-1,5-benzodiazepine (2.0 g, 5.2 mmol) were added benzyltriethylammonium chloride (0.56 g, 2.4 mmol), powdery potassium carbonate (1.8 g, 13 mmol) and methyl bromoacetate (0.83 ml, 8.7 mmol). The reaction mixture was stirred at 90°C for 1.5 hours. Then, the reaction solution was cooled and the insoluble matter was removed

by filtration. The filtrate was concentrated under reduced pressure and water was added to the residue. Thereafter, the mixture was extracted with ethyl acetate and the extract solution was washed with water and dried over anhydrous magnesium sulfate, followed by concentration under reduced pressure. The residue was purified by a silica gel column chromatography to give 1-(4-tert-butoxycarbonylaminomethylphenyl)-5-(methoxycarbonylmethyl)-2,4-dioxo-2,3,4,5-tetrahydro-1H-1,5-benzodiazepine (1.4 g, 62%) as an oil.
$^1$H-NMR($CDCl_3$)δ: 1.46(9H, s), 3.53(1H, d, J=12.0Hz), 3.61(1H, d, J=12.0Hz), 3.78(3H, s), 4.32(2H, d, J=6.0Hz). 4.61 (1H, d, J=17.2Hz), 4.80(1H, d, J=17.2Hz), 4.86(1H, bs), 6.95(1H, d, J=8.2Hz), 7.11-7.36(7H, m).

(5) Benzyl 1-(4-tert-butoxycarbonylaminomethylphenyl)-5-(methoxycarbonylmethyl)-2,4-dioxo-2,3,4,5-tetrahydro-1H-1,5-benzodiazepine-3-acetate

To a stirred N,N-dimethylformamide (20 ml) solution of 1-(4-tert-butoxycarbonylaminomethylphenyl)-5-(methoxycarbonylmethyl)-2,4-dioxo-2,3,4,5-tetrahydro-1H-1,5-benzodiazepine (2.0 g, 4.7 mmol) was added 60% oily sodium hydride (410 mg, 10 mmol). After stirring at room temperature for 10 minutes, benzyl bromoacetate (0.89 ml, 5.6 mmol) was added thereto. The resulting mixture was stirred at room temperature for 10 minutes. Thereafter, the reaction solution was poured into ice-water and extracted with ethyl acetate. After washing with water, the extract solution was dried over anhydrous magnesium sulfate. After concentration under reduced pressure, the residue was purified by a silica gel column chromatography to give benzyl 1-(4-tert-butoxycarbonylaminomethylphenyl)-5-(methoxycarbonylmethyl)-2,4-dioxo-2,3,4,5-tetrahydro-1H-1,5-benzodiazepine-3-acetate (1.3 g, 46%) as an oil.
$^1$H-NMR($CDCl_3$)δ: 1.46(9H, s), 3.05-3.33(2H, m), 3.74(3H, s), 3.99(1H, t, J=7.4Hz), 4.32(1H, d, J=5.8Hz), 4.53(2H, d, J=17.6Hz), 4.84(1H, bs), 4.88(1H, d, J=17.6Hz), 5.09(1H, d, J=12.6Hz), 5.16(1H, d, J=12.6Hz), 6.97(1H, d, J=8.0Hz). 7.11-7.33(12H, m).

(6) 1-(4-tert-Butoxycarbonylaminomethylphenyl)-5-(methoxycarbonylmethyl)-2,4-dioxo-2,3,4,5-tetrahydro-1H-1,5-benzodiazepine-3-acetic acid

Palladium supported on carbon (5%, 0.13 g) was added to a methanol (30 ml) solution of benzyl 1-(4-tert-butoxycarbonylaminomethylphenyl)-5-(methoxycarbonylmethyl)-2,4-dioxo-2,3,4,5-tetrahydro-1H-1,5-benzodiazepine-3-acetate (1.3 g, 2.1 mmol). The resulting mixture was subjected to hydrogenation for 1 hour under the conditions of ambient temperature and normal pressure. The catalyst was removed by filtration and the filtrate was concentrated under reduced pressure to give 1-(4-tert-butoxycarbonylaminomethylphenyl)-5-(methoxycarbonylmethyl)-2,4-dioxo-2,3,4,5-tetrahydro-1H-1,5-benzodiazepine-3-acetic acid (1.1 g, 98%) as oil.
$^1$H-NMR($CDCl_3$)δ: 1.45(9H, s), 3.01-3.32(2H, m), 3.74(3H, s), 3.93(1H, t, J=7.0Hz). 4.31(2H, bs), 4.56(1H, d, J=17.2Hz), 4.88(1H, d, J=17.2Hz), 4.93(1H, bs), 6.99(1H, d, J=8.0Hz), 7.13-7.36(7H, m).

(7) N-(2-Fluorobenzyl)-1-(4-tert-butoxycarbonylaminomethylphenyl)-5-(methoxycarbonylmethyl)-2,4-dioxo-2,3,4,5-tetrahydro-1H-1,5-benzodiazepine-acetamide

To an ice-cooled stirred N,N-dimethylformamide (20 ml) solution of 1-(4-tert-butoxycarbonylaminomethylphenyl)-5-(methoxycarbonylmethyl)-2,4-dioxo-2,3,4,5-tetrahydro-1H-1,5-benzodiazepine-3-acetic acid (1.1 g, 2.11 mmol) were added 2-fluorobenzylamine (0.29 ml, 2.5 mmol), diethyl cyanophosphate (0.41 ml, 2.7 mmol) and triethylamine (0.382 ml, 2.74 mmol). The resulting mixture was stirred at 0°C for 1 hour and at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-fluorobenzyl)-1-(4-tert-butoxycarbonylaminomethylphenyl)-5-(methoxycarbonylmethyl)-2,4-dioxo-2,3,4,5-tetrahydro-1H-1,5-benzodiazepine-3-acetamide (960 mg, 73.3%) as amorphous solid.
$^1$H-NMR($CDCl_3$)δ: 1.46(9H, s), 2.86-3.11(2H, m), 3.73(3H, s), 4.07(1H, t, J=7.0Hz), 4.32(2H, d, J=5.6Hz), 4.74(2H, t, J=5.2Hz), 4.72(1H, d, J=17.2Hz), 4.87(1H, bs), 4.90(1H, d, J=17.2Hz), 6.29(1H, bs), 6.94-7.33(12H, m).

(8) N-(2-Fluorobenzyl)-1-(4-tert-butoxycarbonylaminomethylphenyl)-5-(carboxymethyl)-2,4-dioxo-2,3,4,5-tetrahydro-lH-1,5-benzodiazepine-3-acetamide

To a stirred tetrahydrofuran (10 ml) and methanol (10 ml) solution of N-(2-fluorobenzyl)-1-(4-tert-butoxycarbonylaminomethylphenyl)-5-(methoxycarbonylmethyl)-2,4-dioxo-2,3,4,5-tetrahydro-1H-1,5-benzodiazepine-3-acetamide (0.71 g, 1.15 mmol) was added IN sodium hydroxide aqueous solution (2.3 ml, 2.3 mmol). The resulting mixture was stirred at 60°C for 2 hours. After cooling of the reaction solution, water and potassium hydrogen sulfate (0.313 g, 2.30 mmol) were added thereto. The mixture was extracted with ethyl acetate and, after washing with water, the extract solution was dried over anhydrous magnesium sulfate. The solution was concentrated under reduced pressure to give

N-(2-fluorobenzyl)-1-(4-tert-butoxycarbonylaminomethylphenyl)-5-(carboxymethyl)-2,4-dioxo-2,3,4,5-tetrahydro-1H-1,5-benzodiazepine-3-acetamide (0.69 g, 99.3%) as an oil.
$^1$H-NMR($CDCl_3$)δ: 1.44(9H, s), 2.84(1H, dd, J=5.8,15.0Hz), 3.05(1H, dd, J=7.6,15.0Hz), 4.04(2H, dd, J=5.8,7.6Hz), 4.18(2H, bs), 4.31-4.57(3H, m), 4.97(1H, d, J=17.2Hz), 5.39(1H, bs), 6.55(1H, bs), 6.74-7.42(17H, m).

(9) N-(2-Fluorobenzyl)-5-(phenylcarbamoylmethyl)-1-(4-tert-butoxycarbonylaminomethylphenyl)-2,4-dioxo-2,3,4,5-tetrahydro-1H-1,5-benzodiazepine-3-acetamide

To a stirred N,N-dimethylformamide (2 ml) solution of N-(2-fluorobenzyl)-5-(carboxymethyl)-1-(4-tert-butoxycarbonylaminomethylphenyl)-2,4-dioxo-2,3,4,5-tetrahydro-1H-1,5-benzodiazepine-3-acetamide (200 mg, 0.331 mmol) were added aniline (0.0603 ml, 0.662 mmol), 4-dimethylaminopyridine (4.0 mg, 0.0331 mmol) and 1-ethyl-3-(3-dimethylaminopropyl)carbodiimide hydrochloride (95.2 mg, 0.497 mmol). The resulting mixture was stirred at room temperature for 24 hours. The reaction solution was poured into water and extracted with ethyl acetate. The extract solution was washed with water, a saturated sodium hydrogen carbonate aqueous solution, and water, successively, and then dried over anhydrous magnesium sulfate. After concentration under reduced pressure, the residue was solidified from diethyl ether to give N-(2-fluorobenzyl)-1-(4-tert-butoxycarbonylaminomethylphenyl)-5-(phenylcarbamoylmethyl)-2,4-dioxo-2,3,4,5-tetrahydro-1H-1,5-benzodiazepine-3-acetamide (168 mg, 74.7%) as amorphous solid.
$^1$H-NMR($CDCl_3$)δ: 1.46(9H, bs), 2.93-3.19(2H, m), 4.12(1H, t, J=7.2Hz), 4.23(2H, d, J=5.8Hz), 4.37(1H, d, J=15.4Hz), 4.41-4.58(2H, m), 4.75(1H, bs), 4.85(1H, d, J=15.4Hz), 6.35(1H, t, J=6.6Hz), 6.92-7.55(17H, m).

(10) N-(2-Fluorobenzyl)-1-(4-aminomethylphenyl)-5-(phenylcarbamoylmethyl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-1-(4-tert-butoxycarbonylaminomethylphenyl)-5-(phenylcarbamoylmethyl)-2,4-dioxo-2,3,4,5-tetrahydro-1H-1,5-benzodiazepine-3-acetamide (128 mg, 0.29 mmol). The resulting mixture was stirred at room temperature for 1 hour and then concentrated under reduced pressure. The residue was solidified from ethyl ether to give N-(2-Fluorobenzyl)-1-(4-aminomethylphenyl)-5-(phenylcarbamoylmethyl)-2,4-dioxo-2,3,4,5-tetrahydro-1H-1,5-benzodiazepine-3-acetamide hydrochloride (110 mg, 94%) as crystals.

Melting point 212-215°C.
Elemental analysis for $C_{33}H_{31}N_5O_4ClF \cdot 2H_2O$;
Calcd.: C, 60.78; H, 5.41; N, 10.74.
Found: C, 60.73; H, 5.48; N, 10.79.
$^1$H-NMR(DMSO-$d_6$)δ: 2.86(2H, d, J=6.8Hz), 3.94(1H, t, J=6.8Hz), 4.07(2H, bs), 4.28(2H, d, J=5.2Hz), 4.72(1H, d, J=16.2Hz), 5.07(1H, d, J=16.2Hz), 6.90(1H, d, J=8.0Hz), 7.02-7.68(16H, m), 8.39(3H, bs), 8.60(1H, t, J=5.2Hz).

Example 5

N-(2-Fluorobenzyl)-1-(4-aminomethylphenyl)-5-(4-benzoylaminobenzyl)-2,4-dioxo-2,3,4,5-tetrahydro-1H-1,5-benzodiazepine-3-acetamide hydrochloride

(1) 5-(4-Nitrobenzyl)-1-(4-tert-butoxycarbonylaminomethylphenyl)-2,4-dioxo-2,3,4,5-tetrahydro-1H-1,5-benzodiazepine

To a stirred acetonitrile (100 ml) suspension of 1-(4-tert-butoxycarbonylaminomethylphenyl)-2,4-dioxo-2,3,4,5-tetrahydro-1H-1,5-benzodiazepine (1.8 g, 4.7 mmol) were added benzyltriethylammonium chloride (0.5 g, 2.2 mmol), powdery potassium carbonate (1.6 g, 11.8 mmol) and 4-nitrobenzyl chloride (1.4 g, 7.9 mmol). The reaction mixture was stirred at 90°C for 2 hours. Then, the reaction solution was cooled and the insoluble matter was removed by filtration. The filtrate was concentrated under reduced pressure and water was added to the residue. Thereafter, the mixture was extracted with ethyl acetate, and the extract solution was washed with water and dried over anhydrous magnesium sulfate, followed by concentration under reduced pressure. The residue was purified by a silica gel column chromatography to give 1-(4-tert-butoxycarbonylaminomethylphenyl)-5-(4-nitrobenzyl)-2,4-dioxo-2,3,4,5-tetrahydro-1H-1,5-benzodiazepine (1.9 g, 79%) as an oil.
$^1$H-NMR($CDCl_3$)δ: 1.47(9H, s), 3.60(2H, s), 4.30(2H, d, J=6.2Hz), 4.89(1H, bs), 4.93(1H, d, J=15.4Hz), 5.77(1H, d, J=15.4Hz), 6.82(2H, d, J=8.4Hz), 6.89(2H, d, J=8.2Hz), 7.08-7.42(7H, m), 8.14(2H, d, J=8.8Hz).

(2) 5-(4-Aminobenzyl)-1-(4-tert-butoxycarbonylaminomethylphenyl)-2,4-dioxo-2,3,4,5-tetrahydro-1H-1,5-benzodiazepine

Palladium supported on carbon (5%, 0.2 g) was added to a methanol (50 ml) solution of 1-(4-tert-butoxycarbonylaminomethylphenyl)-5-(4-nitrobenzyl)-2,4-dioxo-2,3,4,5-tetrahydro-lH-1,5-benzodiazepine (1.7 g, 3.2 mmol). The resulting mixture was subjected to hydrogenation for 1 hour under the conditions of ambient temperature and normal pressure. The catalyst was removed by filtration and the filtrate was concentrated under reduced pressure to give 5-(4-aminobenzyl)-1-(4-tert-butoxycarbonylaminomethylphenyl)-2,4-dioxo-2,3,4,5-tetrahydro-1H-1,5-benzodiazepine (1.2 g, 79%) as an oil.
$^1$H-NMR($CDCl_3$)δ: 1.46(9H, s), 3.50(2H, d, J=5.6Hz), 3.70(2H, bs), 4.26(2H, d, J=5.8Hz), 4.53(1H, d, J=14.6Hz), 4.83 (1H, bs), 5.75(1H, d, J=14.6Hz), 6.51(2H, d, J=8.4Hz), 6.67(2H, d, J=8.4Hz), 6.81(1H, d, J=8.0Hz). 6.94(2H, d, J=8.4Hz), 7.06(1H, t, J=8.0Hz), 7.17-7.28(3H, m), 7.48(1H, d, J=8.2Hz).

(3) 5-[4-(Benzylideneamino)benzyl]-1-(4-tert-butoxycarbonylaminomethylphenyl)-2,4-dioxo-2,3,4,5-tetrahydro-1H-1,5-benzodiazepine

Benzaldehyde (0.46 ml, 4.5 mmol) and acetic acid (0.013 ml, 0.23 mmol) were added to a stirred methanol (20 ml) solution of 5-(4-aminobenzyl)-1-(4-tert-butoxycarbonylaminomethylphenyl)-2,4-dioxo-2,3,4,5-tetrahydro-1H-1,5-benzodiazepine (1.1 g, 2.3 mmol). The resulting mixture was stirred at room temperature for 1 hour. Diethyl ether was added to the reaction solution and the precipitate formed was removed by filtration to give 5-[4-(benzylideneamino) benzyl]-1-(4-tert-butoxycarbonylaminomethylphenyl)-2,4-dioxo-2,3,4,5-tetrahydro-1H-1,5-benzodiazepine (1.1 g, 81%).
$^1$H-NMR($CDCl_3$)δ: 1.43(9H, s), 3.57(2H, s), 4.25(2H, d, J=6.2Hz), 4.76(1H, d, J=15.2Hz), 4.79(1H, bs), 5.82(1H, d, J=15.2Hz), 6.77-7.92(17H, m), 8.41(1H, bs).

(4) Methyl 5-[4-(benzylideneamino)benzyl]-1-(4-tert-butoxycarbonylaminomethylphenyl)-2,4-dioxo-2,3,4,5-tetrahydro-1H-1,5-benzodiazepine-3-acetate

To a stirred dimethylformamide (10 ml) solution of 5-[4-(benzylideneamino)benzyl]-1-(4-tert-butoxycarbonylaminomethylphenyl)-2,4-dioxo-2,3,4,5-tetrahydro-1H-1,5-benzodiazepine (1.1 g, 1.8 mmol) was added 60% oily sodium hydride (160 mg, 4.0 mmol). After stirring at room temperature for 10 minutes, methyl bromoacetate (0.21 ml, 2.2 mmol) was added thereto. The resulting mixture was stirred at room temperature for 10 minutes. Thereafter, the reaction solution was poured into ice-water and extracted with ethyl acetate. After washing with water, the extract solution was dried over anhydrous magnesium sulfate. After concentration under reduced pressure, the residue was solidified from ethyl acetate-diethyl ether-diisopropyl ether to give methyl 5-[4-(benzylideneamino)benzyl]-1-(4-tert-butoxycarbonylaminomethylphenyl)-2,4-dioxo-2,3,4,5-tetrahydro-1H-1,5-benzodiazepine-3-acetate (0.57 g, 48%) as amorphous solid.
$^1$H-NMR($CDCl_3$)δ: 1.42(9H, s), 3.17(2H, d, J=7.2Hz), 3.70(3H, s), 3.96(1H, t, J=7.2Hz), 4.24(2H, d, J=5.8Hz), 4.79(1H, d, J=14.6Hz), 4.83(1H, bs), 5.78(1H, d, J=14.6Hz), 6.73(2H, d, J=8.4Hz), 6.85(1H, d, J=8.0Hz), 7.07-7.91(14H, m), 8.40(1H, bs).

(5) Methyl 5-(4-aminobenzyl)-1-(4-tert-butoxycarbonylaminomethylphenyl)-2,4-dioxo-2,3,4,5-tetrahydro-1H-1,5-benzodiazepine-3-acetate

To a stirred methanol (8 ml) solution of 5-[4-(benzylideneamino)benzyl]-1-(4-tert-butoxycarbonylaminomethylphenyl)-2,4-dioxo-2,3,4,5-tetrahydro-1H-1,5-benzodiazepine-3-acetate (0.46 g, 0.71 mmol) was added IN hydrochloric acid (0.71 ml, 0.71 mmol). After stirring at room temperature for 1 hour, IN sodium hydroxide aqueous solution (0.71 ml, 0.71 mmol) was added thereto. The reaction solution was extracted with ethyl acetate. After washing with water, the extract solution was dried over anhydrous magnesium sulfate. After concentration under reduced pressure, the residue was solidified from diisopropyl ether to give methyl 5-(4-aminobenzyl)-1-(4-tert-butoxycarbonylaminomethylphenyl)-2,4-dioxo-2,3,4,5-tetrahydro-1H-1,5-benzodiazepine-3-acetate (0.34 g, 86%) as amorphous solid.
$^1$H-NMR($CDCl_3$)δ: 1.46(9H, s), 3.14(2H, d, J=7.0Hz), 3.64(2H, bs), 3.69(3H, s), 3.91(1H, t, J=7.0Hz), 4.26(2H, d, J=5.4Hz), 4.56(1H, d, J=14.4Hz), 4.83(1H, bs), 5.74(1H, d, J=14.4Hz), 6.50(2H, d, J=8.4Hz), 6.62(1H, d, J=8.4Hz), 6.84(1H, d, J=6.6Hz), 6.91(2H, d, J=8.4Hz), 7.06-7.31(4H, m), 7.52(1H, d, J=8.4Hz).

(6) Methyl 5-(4-benzoylaminobenzyl)-1-(4-tert-butoxycarbonylaminomethylphenyl)-2,4-dioxo-2,3,4,5-tetrahydro-1H-1,5-benzodiazepine-3-acetate

To a stirred tetrahydrofuran (3 ml) solution of methyl 5-(4-aminobenzyl)-1-(4-tert-butoxycarbonylaminomethylphenyl)-2,4-dioxo-2,3,4,5-tetrahydro-1H-1,5-benzodiazepine-3-acetate (0.15 mg, 0.27 mmol) was added triethylamine (0.049 ml, 0.35 mmol) and benzoyl chloride (0.041 ml, 0.35 mmol). After stirring at room temperature for 30 minutes, the reaction solution was poured into water and extracted with ethyl acetate. After washing with water, the extract solution was dried over anhydrous magnesium sulfate and then concentrated under reduced pressure. The residue was solidified from diisopropyl ether to give methyl 5-(4-benzoylaminobenzyl)-1-(4-tert-butoxycarbonylaminomethylphenyl)-2,4-dioxo-2,3,4,5-tetrahydro-1H-1,5-benzodiazepine-3-acetate(170mg, 96%) as amorphous solid.
$^1$H-NMR($CDCl_3$)δ: 1.41(9H, s), 3.15(2H, d, J=6.6Hz), 3.69(3H, s), 3.95(1H, t, J=6.6Hz), 4.20(2H, d, J=5.6Hz), 4.73(1H, d, J=15.0Hz), 4.93(1H, bs), 5.75(1H, d, J=15.0Hz), 6.69(2H, d, J=8.4Hz), 6.86(1H, d, J=8.2Hz), 7.08-7.52(12H, m), 7.89(2H, d, J=6.2Hz), 8.07(1H, bs).

(7) 5-(4-Benzoylaminobenzyl)-1-(4-tert-butoxycarbonylaminomethylphenyl)-2,4-dioxo-2,3,4,5-tetrahydro-1H-1,5-benzodiazepine-3-acetic acid

To a stirred tetrahydrofuran (3 ml) and methanol (3 ml) solution of methyl 5-(4-benzoylaminobenzyl)-1-(4-tert-butoxycarbonylaminomethylphenyl)-2,4-dioxo-2,3,4,5-tetrahydro-1H-1,5-benzodiazepine-3-acetate (170 mg, 0.26 mmol) was added IN sodium hydroxide aqueous solution (0.51 ml, 0.51 mmol). The resulting mixture was stirred at 60°C for 2.5 hours. After cooling of the reaction solution, water and potassium hydrogen sulfate (70 mg, 0.51 mmol) were added thereto. The mixture was extracted with ethyl acetate and, after washing with water, the extract solution was dried over anhydrous magnesium sulfate. The solution was concentrated under reduced pressure to give 5-(4-benzoylaminobenzyl)-1-(4-tert-butoxycarbonylaminomethylphenyl)-2,4-dioxo-2,3,4,5-tetrahydro-1H-1,5-benzodiazepine-3-acetic acid (160 mg, 96%) as amorphous solid.
$^1$H-NMR($CDCl_3$)δ: 1.45(9H, s), 3.10(1H, dd, J=7.8,17.4Hz), 3.28(1H, dd, J=7.8,17.4Hz), 3.97(1H, t, J=7.8Hz), 4.24 (1H, bs), 4.86(1H, d, J=15.4Hz), 5.02(1H. bs), 5.39(1H, d, J=15.4Hz), 6.78(2H, d, J=8.4Hz), 6.89(1H, d, J=8.2Hz), 7.03-7.54(12H, m), 7.87(2H, d, J=6.6Hz), 8.42(1H, bs).

(8) N-(2-Fluorobenzyl)-5-(4-benzoylaminobenzyl)-1-(4-tert-butoxycarbonylaminomethylphenyl)-2,4-dioxo-2,3,4,5-tetrahydro-lH-1,5-benzodiazepine-3-acetamide

To an ice-cooled stirred N,N-dimethylformamide (2 ml) solution of 5-(4-benzoylaminobenzyl)-1-(4-tert-butoxycarbonylaminomethylphenyl)-2,4-dioxo-2,3,4,5-tetrahydro-1H-1,5-benzodiazepine-3-acetic acid (160 mg, 0.25 mmol) were added 2-fluorobenzylamine (0.044 ml, 0.30 mmol), diethyl cyanophosphate (0.48 ml, 0.32 mmol) and triethylamine (0.045 ml, 0.32 mmol). The resulting mixture was stirred at 0°C for 30 minutes and at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-fluorobenzyl)-5-(4-benzoylaminobenzyl)-1-(4-tert-butoxycarbonylaminomethylphenyl)-2,4-dioxo-2,3,4,5-tetrahydro-1H-1,5-benzodiazepine-3-acetamide (133 mg, 71%) as amorphous solid.
$^1$H-NMR($CDCl_3$)δ: 1.40(9H, s), 2.97(2H, d, J=7.0Hz), 4.04(1H, t, J=7.0Hz). 4.21(2H, bs), 4.40-4.59(2H, m), 4.68(1H, d, J=14.0Hz), 4.97(1H, bs), 5.75(1H, d, J=14.0Hz), 6.42(1H, bs), 6.61(2H, d, J=7.8Hz), 6.82(1H, d, J=8.2Hz), 7.00-7.58 (16H, m), 7.87(2H, d, J=7.8Hz), 8.11(1H, bs).

(9) N-(2-fluorobenzyl)-1-(4-aminomethylphenyl)-5-(4-benzoylaminobenzyl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-5-(4-benzoylaminobenzyl)-1-(4-tert-butoxycarbonylaminomethylphenyl)-2,4-dioxo-2,3,4,5-tetrahydro-1H-1,5-benzodiazepine-3-acetamide (130 mg, 0.17 mmol). The resulting mixture was stirred at room temperature for 1 hour and then concentrated under reduced pressure. The residue was solidified from ethyl ether to give N-(2-fluorobenzyl)-1-(4-aminomethylphenyl)-5-(4-benzoylaminobenzyl)-2,4-dioxo-2,3,4,5-tetrahydro-1H-1,5-benzodiazepine-3-acetamide hydrochloride (110 mg, 97%) as amorphous solid.

Elemental analysis for $C_{39}H_{35}N_5O_4ClF\cdot H_2O$;
Calcd.: C, 65.96; H, 5.25; N, 9.86.
Found: C, 65.71; H, 5.36; N, 9.66.

$^1$H-NMR(DMSO-$d_6$)δ: 2.78-3.02(2H, m), 3.93(1H, t, J=6.8Hz), 3.99(2H, d, J=5.6Hz), 4.30(2H, d, J=5.8Hz), 4.94 (1H, d, J=15.4Hz), 5.59(1H, d, J=15.4Hz), 6.78-7.80(19H, m), 7.98(2H, d, J=6.2Hz), 8.37(3H, bs), 8.62(1H, t, J=5.8Hz), 10.34(1H, bs).

Example 6

N-(2-Fluorobenzyl)-1-(4-aminomethylphenyl)-5-(4-methanesulfonylaminobenzyl)-2,4-dioxo-2,3,4,5-tetrahydro-1H-1,5-benzodiazepine-3-acetamide hydrochloride

(1) Methyl 1-(4-tert-butoxycarbonylaminomethylphenyl)-5-(4-methanesulfonylaminobenzyl)-2,4-dioxo-2,3,4,5-tetrahydro-1H-1,5-benzodiazepine-3-acetate

To a stirred tetrahydrofuran (3 ml) solution of methyl 5-(4-aminobenzyl)-1-(4-tert-butoxycarbonylaminomethylphenyl)-2,4-dioxo-2,3,4,5-tetrahydro-1H-1,5-benzodiazepine-3-acetate (0.15 mg, 0.27 mmol) was added triethylamine (0.049 ml, 0.35 mmol) and methanesulfonyl chloride (0.027 ml, 0.35 mmol). After stirring of the reaction mixture at room temperature for 1 hour, dimethylaminopyridine (43 mg, 0.35 mmol), triethylamine (0.049 ml, 0.35 mmol) and methanesulfonyl chloride (0.027 ml, 0.35 mmol) were added thereto. The resulting mixture was further stirred at room temperature for 1 hour. The reaction solution was poured into water and extracted with ethyl acetate. After washing with water, the extract solution was dried over anhydrous magnesium sulfate and then concentrated under reduced pressure. The residue was purified by a silica gel column chromatography to give methyl 1-(4-tert-butoxycarbonylaminomethylphenyl)-5-(4-methanesulfonylaminobenzyl)-2,4-dioxo-2,3,4,5-tetrahydro-1H-1,5-benzodiazepine-3-acetate (75 mg, 44%) as an oil.
$^1$H-NMR($CDCl_3$)δ: 1.45(9H, s), 2.91(3H, s), 3.19(2H, d, J=7.0Hz), 3.69(3H, s), 3.94(1H, t, J=7.0Hz). 4.22(2H, d, J=5.8Hz), 4.65(1H, d, J=14.8Hz), 5.38(1H, bs), 5.75(1H, d, J=14.8Hz), 6.57(2H, d, J=8.2Hz), 6.85-7.34(9H, m), 7.52 (1H, d, J=8.2Hz), 7.61(1H, bs).

(2) 1-(4-tert-butoxycarbonylaminomethylphenyl)-5-(4-methanesulfonylaminobenzyl)-2,4-dioxo-2,3,4,5-tetrahydro-1H-1,5-benzodiazepine-3-acetic acid

To a stirred tetrahydrofuran (2 ml) and methanol (2 ml) solution of methyl 1-(4-tert-butoxycarbonylaminomethylphenyl)-5-(4-methanesulfonylaminobenzyl)-2,4-dioxo-2,3,4,5-tetrahydro-1H-1,5-benzodiazepine-3-acetate (75 mg, 0.12 mmol) was added IN sodium hydroxide aqueous solution (0.47 ml, 0.47 mmol). The resulting mixture was stirred at 60°C for 2 hours. After cooling of the reaction solution, water and potassium hydrogen sulfate (64 mg, 0.47 mmol) were added thereto. The mixture was extracted with ethyl acetate and, after washing with water, the extract solution was dried over anhydrous magnesium sulfate. The solution was concentrated under reduced pressure to give 1-(4-tert-butoxycarbonylaminomethylphenyl)-5-(4-methanesulfonylaminobenzyl)-2,4-dioxo-2,3,4,5-tetrahydro-1H-1,5-benzodiazepine-3-acetic acid (63 mg, 98%) as crystals.
$^1$H-NMR($CDCl_3$)δ: 1.40(9H, s), 2.77-2.89(2H, m), 2.94(3H, s), 3.78(1H, t, J=7.0Hz), 4.10(2H, d, J=6.0Hz), 4.92(1H, d, J=15.0Hz), 5.52(1H, d, J=15.0Hz), 6.65-7.38(11H, m), 7.75(1H, d, J=8.2Hz), 9.65(1H, bs), 9.74(1H, bs).

(3) N-(2-Fluorobenzyl)-1-(4-tert-butoxycarbonylaminomethylphenyl)-5-(4-methanesulfonylaminobenzyl)-2,4-dioxo-2,3,4,5-tetrahydro-1H-1,5-benzodiazepine-3-acetamide

To an ice-cooled stirred N,N-dimethylformamide (2 ml) solution of 1-(4-tert-butoxycarbonylaminomethylphenyl)-5-(4-methanesulfonylaminobenzyl)-2,4-dioxo-2,3,4,5-tetrahydro-1H-1,5-benzodiazepine-3-acetic acid (60 mg, 0.096 mmol) were added 2-fluorobenzylamine (0.013 ml, 0.12 mmol), diethyl cyanophosphate (0.019 ml, 0.13 mmol) and triethylamine (0.017 ml, 0.13 mmol). The resulting mixture was stirred at 0°C for 1 hour and at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-fluorobenzyl)-1-(4-tert-butoxycarbonylaminomethylphenyl)-5-(4-methanesulfonylaminobenzyl)-2,4-dioxo-2,3,4,5-tetrahydro-1H-1,5-benzodiazepine-3-acetamide (38 mg, 54%) as amorphous solid.
$^1$H-NMR($CDCl_3$)δ: 1.44(9H, s), 2.82(3H, s), 2.98-3.11(2H, m), 4.10(1H, t, J=6.6Hz), 4.27(2H, bs), 4.38-4.54(4H, m), 5.71(1H, bs), 5.83(1H, d, J=14.4Hz), 5.71(1H, bs), 5.83(1H, d, J=14.4Hz), 6.3(2H, d, J=7.0Hz), 6.78-7.35(13H, m), 7.54(2H, d, J=7.4Hz), 7.84(1H, bs).

(4) N-(2-Fluorobenzyl)-1-(4-aminomethylphenyl)-5-(4-methanesulfonylaminobenzyl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-1-(4-tert-butoxycarbonylaminomethylphenyl)-5-(4-methanesulfonylaminobenzyl)-2,4-dioxo-2,3,4,5-tetrahydro-1H-1,5-benzodiazepine-3-acetamide (38 mg, 0.06 mmol). The resulting mixture was stirred at room temperature for 1 hour and then concentrated under reduced pressure. The residue was solidified from ethyl ether to give N-(2-fluorobenzyl)-1-(4-aminomethylphenyl)-5-(4-methanesulfonylaminobenzyl)-2,4-dioxo-2,3,4,5-tetrahydro-1H-1,5-benzodiazepine-3-acetamide hydrochloride (30 mg, 76%) as amorphous solid. $^1$H-NMR(DMSO-$d_6$)δ: 2.85-2.92 (2H, m), 2.96(3H, s), 3.92(1H, t, J=7.2Hz), 4.04(2H, d, J=7.2Hz), 4.29(1H, d, J=6.0Hz). 4.93(1H, d, J=14.8Hz), 5.54 (1H, d, J=14.8Hz), 6.75-7.48(15H, m), 7.77(1H, d, J=7.8Hz), 8.30(3H, bs), 8.61(1H, t, J=6.0Hz), 9.77(1H, bs).

Example 7

N-(2-Fluorobenzyl)-1-(4-aminomethylphenyl)-5-[2-(4-biphenyl)ethyl]-2,4-dioxo-2,3,4,5-tetrahydro-1H-1,5-benzodiazepine-3-acetamide hydrochloride

(1) N-Methyl-N-methoxy-4-biphenylacetamide

To a N,N-dimethylformamide (140 ml) solution of 4-biphenylacetic acid (4.9 g, 15.6 mmol) were added N,O-dimethylhydroxyamine hydrochloride (6.2 g, 64 mmol), triethylamine (8.9 ml, 64 mmol) and 1-ethyl-3-(3-dimethylaminopropyl)carbodiimide hydrochloride (13.5 g, 70 mmol). The resulting mixture was stirred at room temperature for 3 hours. The reaction solution was poured into water and extracted with ethyl acetate. The extract solution was washed with water, a saturated sodium hydrogen carbonate aqueous solution, and water, successively, and then dried over anhydrous magnesium sulfate. After concentration under reduced pressure, the residue was solidified from diisopropyl ether to give N-methyl-N-methoxy-4-biphenylacetamide (7.6 g, 48%) as amorphous solid.
$^1$H-NMR($CDCl_3$)δ: 3.21(3H, s), 3.65(3H, s), 3.82(2H, s), 7.26-7.61(9H, m). (2)

(2) 4-Biphenylacetaldehyde

To a tetrahydrofuran (100 ml) solution of N-methyl-N-methoxy-4-biphenylacetamide (5.0 g, 20.5 mmol) was added dropwise a toluene solution (25.5 ml, 25.5 mmol) of 1M diisopropylaluminum hydride at -70 to -60°C. After stirring at the temperature for 30 minutes, the reaction solution was poured into water and extracted with ethyl acetate. The extract solution was washed with 1N hydrochloric acid and water, successively, and then dried over anhydrous magnesium sulfate. The solution was concentrated under reduced pressure to give 4-biphenylacetaldehyde (4 g, 100%) as an oil.
$^1$H-NMR($CDCl_3$)δ: 3.74(2H, d, J=2.2Hz), 7.12-7.62(9H, m), 9.80(1H, t, J=2.2Hz).

(3) tert-Butyl [4-[2-[2-(4-biphenyl)ethylamino]phenylamino]benzyl]carbamate

Acetic acid (1.8 ml, 31 mmol) and sodium cyanoborohydride (1.2 g, 19.5 mmol) were added to a methanol solution (125 ml) of tert-butyl [4-(2-aminophenylamino)benzyl]carbamate (4.9 g, 15.6 mmol) and 4-biphenylacetaldehyde. The resulting mixture was stirred at 60°C for 1 hour. The reaction solution was poured into water and extracted with ethyl acetate. After washing with water, the extract solution was dried over anhydrous magnesium sulfate and concentrated under reduced pressure. The residue was solidified from diisopropyl ether to give tert-butyl [4-[2-[2-(4-biphenyl)ethylamino]phenylamino]benzyl]carbamate (3.2 g, 42%) as a solid.
$^1$H-NMR($CDCl_3$)δ: 1.45(9H, s), 2.92(2H, t, J=7.0Hz), 3.43(2H, t, J=7.0Hz), 4.17(2H, d, J=5.6Hz), 4.19(1H, s), 4.77(1H, bs), 4.99(1H, bs), 6.60-7.58(17H, m).

(4) Ethyl N-[2-(4-biphenyl)ethyl]-N-[2-[4-(tert-butoxycarbonylaminomethyl)phenylamino]phenyl]malonamate

To an ice-cooled stirred tetrahydrofuran (50 ml) solution of tert-butyl [3-[2-[2-(4-biphenyl)ethylamino]phenylamino]benzyl]carbamate (3.0 g, 6.1 mmol) were added triethylamine (0.93 ml, 6.7 mmol) and ethyl malonyl chloride (0.86 ml, 6.7 mmol). The resulting mixture was stirred at 0°C for 1 hour and then triethylamine (0.47 ml, 3.3 mmol) and ethyl malonyl chloride (0.43 ml, 3.3 mmol) was further added thereto. After stirring at 0°C for 10 minutes, the reaction solution was poured into water and extracted with ethyl acetate. The extract solution was washed with water and then dried over anhydrous magnesium sulfate. After concentration under reduced pressure, the residue was purified by a silica gel column chromatography to give ethyl N-[2-(4-biphenyl)ethyl]-N-[2-(4-tert-butoxycarbonylaminomethyl)phenylamino]phenylmalonamate (3.4 g, 92%) as an oil.
$^1$H-NMR($CDCl_3$)δ: 1.18(3H, t, J=7.2Hz), 1.46(9H, s), 2.91-3.14(2H, m), 3.22(1H, d, J=15.8Hz), 3.34(1H, d, J=15.8Hz), 3.66-3.80(2H, m), 4.13(2H, q, J=7.2Hz), 4.24(2H, d, J=5.8Hz), 4.76(1H, bs), 6.03(1H, bs), 6.84-7.56(17H, m).

(5) N-[2-(4-biphenyl)ethyl]-N-[2-(4-tert-butoxycarbonylaminomethyl)phenylamino]phenylmalonamic acid

To an ice-cooled stirred tetrahydrofuran (10 ml) and methanol (30 ml) solution of ethyl N-[2-(4-biphenyl)ethyl]-N-[2-(4-tert-butoxycarbonylaminomethyl)phenylamino]phenylmalonamate (3.3 g, 5.4 mmol) was added IN sodium hydroxide aqueous solution (11 ml, 11 mmol). The resulting mixture was stirred at 0°C for 10 minutes and at room temperature for 3 hours. Water was added to the reaction mixture and the mixture was washed with diisopropyl ether. Potassium hydrogen sulfate (1.5 g, 11 mmol) was added thereto and then the mixture was extracted with ethyl acetate. After washing with water, the extract solution was dried over anhydrous magnesium sulfate. The solution was concentrated under reduced pressure to give N-[2-(4-biphenyl)ethyl]-N-[2-(3-tert-butoxycarbonylaminomethyl)phenylamino] phenylmalonamic acid (2.3 g, 74%) as an oil.
$^1$H-NMR($CDCl_3$)δ: 1.45(9H, s), 2.84-3.18(2H, m), 3.23(2H, d, J=2.2Hz), 3.88-4.02(2H, m), 4.22(2H, d, J=6.2Hz), 4.82 (1H, bs), 5.48(1H, bs), 6.88-7.58(17H, m).

(6) 5-[2-(4-biphenyl)ethyl]-1-(4-tert-butoxycarbonylaminomethylphenyl)-2,4-dioxo-2,3,4,5-tetrahydro-1H-1,5-benzodiazepine

To an ice-cooled stirred N,N-dimethylformamide (50 ml) solution of N-[2-(4-biphenyl)ethyl]-N-[2-(3-tert-butoxycarbonylaminomethyl)phenylamino]phenylmalonamic acid (2.25 g, 3.9 mmol) were added 4-dimethylaminopyridine (47 mg, 0.39 mmol) and 1-ethyl-3-(3-dimethylaminopropyl)carbodiimide hydrochloride (0.82 g, 4.3 mmol). The resulting mixture was stirred at room temperature for 12 hours. The reaction solution was poured into water and extracted with ethyl acetate. The extract solution was washed with water, a saturated sodium hydrogen carbonate aqueous solution, and water, successively, and then dried over anhydrous magnesium sulfate. After concentration under reduced pressure, the residue was purified by a silica gel column chromatography to give 5-[2-(4-biphenyl)ethyl]-1-(3-tert-butoxycarbonylaminomethylphenyl)-2,4-dioxo-2,3,4,5-tetrahydro-lH-1,5-benzodiazepine (1.0 g, 47%) as an oil.
$^1$H-NMR($CDCl_3$)δ: 1.46(9H, s), 3.59(2H, s), 2.98-3.08(2H, m), 3.51(2H, s), 3.85-3.99(1H, m), 4.24(2H, d, J=6.0Hz), 4.27-4.87(2H, m), 6.89-7.55(17H, m).

(7) Methyl 5-[2-(4-biphenyl)ethyl]-1-(4-tert-butoxycarbonylaminomethylphenyl)-2,4-dioxo-2,3,4,5-tetrahydro-1H-1,5-benzodiazepine-3-acetate

To a stirred N,N-dimethylformamide (10 ml) solution of 5-[2-(4-biphenyl)ethyl]-1-(4-tert-butoxycarbonylaminomethylphenyl)-2,4-dioxo-2,3,4,5-tetrahydro-1H-1,5-benzodiazepine (0.95 g, 1.7 mmol) was added 60% oily sodium hydride (0.15 g, 3.7 mmol). After stirring at room temperature for 10 minutes, methyl bromoacetate (0.19 ml, 2.0 mmol) was added thereto. The resulting mixture was stirred at room temperature for 10 minutes. Thereafter, the reaction solution was poured into ice-water and extracted with ethyl acetate. After washing with water, the extract solution was dried over anhydrous magnesium sulfate. After concentration under reduced pressure, the residue was purified by a silica gel column chromatography to give methyl 5-[2-(4-biphenyl)ethyl]-1-(4-tert-butoxycarbonylaminomethylphenyl)-2,4-dioxo-2,3,4,5-tetrahydro-1H-1,5-benzodiazepine-3-acetate (0.61 g, 57%) as an oil.
$^1$H-NMR($CDCl_3$)δ: 1.46(9H, s), 3.00(2H, t, J=7.6Hz), 3.90-4.01(1H, m), 3.15(2H, d, J=7.2Hz), 3.70(3H, s), 3.89(1H, t, J=7.2Hz), 4.24(2H, d, J=5.8Hz), 4.69-4.79(2H, m), 6.89-7.55(17H, m).

(8) 5-[2-(4-biphenyl)ethyl]-1-(4-tert-butoxycarbonylaminomethylphenyl)-2,4-dioxo-2,3,4,5-tetrahydro-1H-1,5-benzodiazepine-3-acetic acid

To a stirred tetrahydrofuran (5 ml) and methanol (5 ml) solution of methyl 5-[2-(4-biphenyl)ethyl]-1-(4-tert-butoxycarbonylaminomethylphenyl)-2,4-dioxo-2,3,4,5-tetrahydro-1H-1,5-benzodiazepine-3-acetate (0.5 g, 0.79 mmol) was added IN sodium hydroxide aqueous solution (1.6 ml, 1.6 mmol). The resulting mixture was stirred at 60°C for 2 hours. After cooling of the reaction solution, water and potassium hydrogen sulfate (5.4 g, 40 mmol) were added thereto. The mixture was extracted with ethyl acetate and, after washing with water, the extract solution was dried over anhydrous magnesium sulfate. The solution was concentrated under reduced pressure to give 5-[2-(4-biphenyl)ethyl]-1-(4-tert-butoxycarbonylaminomethylphenyl)-2,4-dioxo-2,3,4,5-tetrahydro-1H-1,5-benzodiazepine-3-acetic acid (0.43 g, 89%) as amorphous solid.
$^1$H-NMR($CDCl_3$)δ: 1.45(9H, s), 3.00(2H, t, J=7.6Hz), 3.13-3.23(2H, m), 3.84(2H, m), 3.84(1H, t, J=6.6Hz), 3.91-4.03 (1H, m), 4.24(2H, d, J=5.8Hz), 4.68-4.82(2H, m), 6.88-7.55(17H, m).

(9) N-(2-Fluorobenzyl)-5-[2-(4-biphenyl)ethyl]-1-(4-tert-butoxycarbonylaminomethylphenyl)-2,4-dioxo-2,3,4,5-tetrahydro-1H-1,5-benzodiazepine-3-acetamide

To an ice-cooled stirred N,N-dimethylformamide (3 ml) solution of 5-[2-(4-biphenyl)ethyl]-1-(4-tert-butoxycarbonylaminomethylphenyl)-2,4-dioxo-2,3,4,5-tetrahydro-1H-1,5-benzodiazepine-3-acetic acid (0.25 g, 0.40 mmol) were added 2-fluorobenzylamine (0.055 ml, 0.48 mmol), diethyl cyanophosphate (0.078 ml, 0.52 mmol) and triethylamine (0.073 ml, 0.52 mmol). The resulting mixture was stirred at room temperature for 12 hours. The reaction solution was poured into water and extracted with ethyl acetate. The extract solution was washed with water and dried over anhydrous magnesium sulfate, followed by concentration under reduced pressure. The residue was purified by a silica gel column chromatography to give N-(2-fluorobenzyl)-5-[2-(4-biphenyl)ethyl]-1-(4-tert-butoxycarbonylaminomethylphenyl)-2,4-dioxo-2,3,4,5-tetrahydro-1H-1,5-benzodiazepine-3-acetamide (200 mg, 68%) as amorphous solid.
$^1$H-NMR($CDCl_3$)δ: 1.46(9H, s), 3.00(2H, d, J=7.4Hz), 3.78-4.02(2H, m), 4.24(2H, d, J=6.2Hz), 4.49(2H, t, J=5.0Hz), 4.66-4.78(2H, m), 6.37(1H, bs), 5,94(1H, d, J=14.8Hz), 6.84-7.54(21H, m).

(10) N-(2-Fluorobenzyl)-1-(4-aminomethylphenyl)-5-[2-(4-biphenyl)ethyl]-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-5-[2-(4-biphenyl)ethyl]-1-(4-tert-butoxycarbonylaminomethylphenyl)-2,4-dioxo-2,3,4,5-tetrahydro-1H-1,5-benzodiazepine-3-acetamide (158 mg, 0.22 mmol). The resulting mixture was stirred at room temperature for 1 hour and then concentrated under reduced pressure. The residue was crystallized from ethyl ether to give N-(2-fluorobenzyl)-1-(4-aminomethylphenyl)-5-[2-(4-biphenyl)ethyl]-2,4-dioxo-2,3,4,5-tetrahydro-lH-1,5-benzodiazepine-3-acetamide hydrochloride (140 mg, 94%) as crystals.
Melting point 165-167°C.

Elemental analysis for $C_{39}H_{36}N_4O_3ClF\cdot H_2O$;
Calcd.: C, 68.76; H, 5.62; N, 8.22.
Found: C, 68.72; H, 5.53; N, 8.02.
$^1$H-NMR(DMSO-$d_6$)δ: 2.79-2.94(4H, m), 3.87(1H, t, J=6.8Hz), 3.96-4.12(3H, m), 4.29(2H, d, J=5.4Hz), 4.46-4.68 (1H, m), 6.88(1H, d, J=8.2Hz), 7.05-7.65(19H, m), 7.79(1H, d, J=8.0Hz), 8.32(3H, bs), 8.60(1H, t, J=5.8Hz).

Example 8

N-(2-Fluorobenzyl)-5-(4-biphenylmethyl)-1-(isoindolin-5-yl)-2,4-dioxo-2,3,4,5-tetrahydro-lH-1,5-benzodiazepine-3-acetamide hydrochloride

(1) 5-Amino-2-tert-butoxycarbonylisoindoline

Bromine (18.2 ml) was slowly added dropwise to 4-nitro-o-xylene (25.5 g) under heating at 120 to 130°C. After the completion of the addition, the reaction solution was cooled and diluted with ethyl acetate (200 ml). The resulting mixture was washed with water and dried over anhydrous magnesium sulfate. After the removal of the solvent by evaporation, the residue was dissolved into ethanol (500 ml). Potassium carbonate (70 g) and α-aminodiphenylmethane (31 g) were added thereto and the mixture was stirred under heating to reflux for 3 hours. After cooling of the reaction solution, insoluble matter was removed by filtration and the filtrate was concentrated under reduced pressure. The residue was dissolved into ethyl acetate (200 ml) and then the solution was washed with 1N sodium hydroxide aqueous solution and water, successively. After drying over anhydrous magnesium sulfate, the solvent was removed by evaporation and the residue was crystallized from diisopropyl ether. The crystals were collected by filtration to give 2-diphenylmethyl-5-nitroisoindoline (12.9 g). [Melting point 154-155°C. $^1$H-NMR($CDCl_3$)δ: 3.91(4H, s), 4.67(1H, s), 7.18-7.38(7H, m), 7.50-7.58(4H, m), 7.95-8.15(2H, m).]
Then, 2-diphenylmethyl-5-nitroisoindoline (12,8 g) was dissolved into methanol (200 ml) and 4N ethyl acetate solution (20 ml) of hydrogen chloride and palladium supported on carbon (10%) were added. The resulting mixture was subjected to hydrogenation under the conditions of ambient temperature and normal pressure. After the completion of the reaction, the catalyst was removed by filtration and the filtrate was concentrated under reduced pressure. The residue was dissolved into a mixed solution of water and tetrahydrofuran (1/1 = v/v)(200 ml) and 1N sodium hydroxide aqueous solution (120 ml) was added thereto. To the resulting mixture was added di-tert-butyl dicarbonate (9.3 g) and the mixture was stirred at room temperature for 2 hours. The reaction solution was extracted with ethyl acetate and then the extract solution was dried over anhydrous magnesium sulfate, followed by removal of the solvent by evaporation. The residue was purified by a silica gel column chromatography to give 5-amino-2-tert-butoxycarbonylisoindoline

(7.7 g) as an oil.
$^1$H-NMR($CDCl_3$)δ: 1.51(9H, s), 3.65(2H, bs), 4.50-4.63(4H, m), 6.52-6.64(2H, m), 6.95-7.08(1H, m).

(2) 2-tert-Butoxycarbonyl-5-(2-nitrophenylamino)isoindoline

A dimethylformamide (10 ml) suspension of 5-amino-2-tert-butoxycarbonylisoindoline (5.3 g, 22.6 mmol), o-fluoronitrobenzene (7.15 ml, 22.6 mmol) and potassium carbonate (3.12 g, 22.6 mmol) was stirred at 145°C for 2 hours under nitrogen atmosphere. After cooling, the reaction mixture was diluted with ethyl acetate and washed with water. After drying over anhydrous magnesium sulfate, the mixture was concentrated under reduced pressure. The residue was purified by a silica gel column chromatography to give 2-tert-butoxycarbonyl-5-(2-nitrophenylamino)isoindoline (4.9 g, 61%) as an oil.
$^1$H-NMR($CDCl_3$)δ: 1.53(9H, s), 4.67(2H, s), 4.70(2H, s), 6.76(1H, t, J=6.8Hz), 7.15-7.41(5H, m), 8.20(1H, d, J=8.8Hz), 9.47(1H, bs).

(3) 5-(2-Aminophenylamino)-2-tert-butoxycarbonylisoindoline

Palladium supported on carbon (5%, 0.5 g) was added to a methanol (200 ml) solution of 2-tert-butoxycarbonyl-5-(2-nitrophenylamino)isoindoline (4.8 g, 13.5 mmol). The resulting mixture was subjected to hydrogenation for 2 hours under the conditions of ambient temperature and normal pressure. The catalyst was removed by filtration and the filtrate was concentrated under reduced pressure to give 5-(2-aminophenylamino)-2-tert-butoxycarbonylisoindoline (4.2 g, 95%) as an oil. $^1$H-NMR($CDCl_3$)δ: 1.50(9H, s), 3.48(2H, bs), 4.55(2H, s), 4.58(2H, s), 5.22(1H, bs), 6.56-7.11 (7H, m).

(4) 5-[2-(4-Biphenylmethylamino)phenylamino]-2-tert-butoxycarbonylisoindoline

Acetic acid (1.4 ml, 25 mmol) and sodium cyanoborohydride (1 g, 16.4 mmol) were added to a methanol solution (125 ml) of 5-(2-aminophenylamino)-2-tert-butoxycarbonylisoindoline (4.1 g, 12.5 mmol) and 4-phenylbenzaldehyde (3.0 g, 16.4 mmol). Then, the mixture was stirred at 60°C for 1 hour. The reaction solution was poured into water and the precipitated solid was collected by filtration to give 5-[2-(4-biphenylmethylamino)phenylamino]-2-tert-butoxycarbonylisoindoline (6 g, 98%) as a solid substance. $^1$H-NMR($CDCl_3$)δ: 1.50(9H, s), 4.39(2H, s), 4.56(2H, s), 4.60(2H, s), 4.75(1H, bs), 6.57-7.59(16H, m).

(5) Ethyl N-(4-biphenylmethyl)-N-[2-(2-(tert-butoxycarbonylisoindolin-5-ylamino)phenyl]malonamate

To an ice-cooled stirred ethyl acetate (500 ml) solution of 5-[2-(4-biphenylmethylamino)phenylamino]-2-tert-butoxycarbonylisoindoline (4.6 g, 9.3 mmol) were added triethylamine (1.4 ml, 10.2 mmol) and ethyl malonyl chloride (1.3 ml, 10.2 mmol). After stirring at 0°C for 1 hour, the reaction solution was poured into ice-water and extracted with ethyl acetate. The extract solution was washed with water and then dried over anhydrous magnesium sulfate. After concentration under reduced pressure, the residue was purified by a silica gel column chromatography to give ethyl N-(4-biphenylmethyl)-N-[2-(2-(tert-butoxycarbonylisoindolin-5-ylamino)phenyl]malonamate (3.8 g, 71%) as an oil.
$^1$H-NMR($CDCl_3$)δ: 1.09(3H, t, J=6.8Hz), 1.52(9H, s), 3.25(1H, d, J=15.8Hz), 3.34(1H, d, J=15.8Hz), 4.09(2H, q, J=6.8Hz), 4.43-4,77(5H, m), 5.08-5.27(1H, m), 5.60(0.5H, s), 5.70(0.5H, s), 6.52-7.51(16H, m).

(6) N-(4-Biphenylmethyl)-N-[2-(2-(tert-butoxycarbonylisoindolin-5-ylamino)phenyl]malonamic acid

To an ice-cooled stirred tetrahydrofuran (10 ml) and methanol (30 ml) solution of ethyl N-(4-biphenylmethyl)-N-[2-(2-(tert-butoxycarbonylisoindolin-5-ylamino)phenyl]malonamate (3.7 g, 6.1 mmol) was added 1N sodium hydroxide aqueous solution (12 ml, 12 mmol). The resulting mixture was stirred at 0°C for 20 minutes and at room temperature for 2 hours. Water was added to the reaction solution and the mixture was washed with diisopropyl ether. Potassium hydrogen sulfate (1.6 g, 12 mmol) was added thereto and then the mixture was extracted with ethyl acetate. After washing with water, the extract solution was dried over anhydrous magnesium sulfate. The solution was concentrated under reduced pressure to give N-(4-biphenylmethyl)-N-[2-(2-(tert-butoxycarbonylisoindolin-5-ylamino)phenyl] malonamic acid (2.5 g, 70%) as an oil.
$^1$H-NMR($CDCl_3$)δ: 1.51(9H, s), 3.26(2H, s), 4.27-4.54(5H, m), 5.54(0.5H, d, J=13.6Hz), 5.65(0.5H, d, J=13.2Hz), 6.31 (0.5H, bs), 6.51(0.5H, bs), 6.91-7.59(16H, m).

(7) 5-(4-Biphenylmethyl)-1-(2-tert-butoxycarbonylisoindolin-5-yl)-2,4-dioxo-2,3,4,5-tetrahydro-1H-1,5-benzodiazepine

To an ice-cooled stirred N,N-dimethylformamide (50 ml) solution of N-(4-biphenylmethyl)-N-[2-(2-(tert-butoxycarbonylisoindolin-5-ylamino)phenyl]malonamic acid (2.4 g, 4.12 mmol) were added 4-dimethylaminopyridine (50 mg, 0.41 mmol) and 1-ethyl-3-(3-dimethylaminopropyl)carbodiimide hydrochloride (0.87 g, 4.5 mmol). The resulting mixture was stirred at room temperature for 12 hours. The reaction solution was poured into water and extracted with ethyl acetate. The extract solution was washed with water, a saturated sodium hydrogen carbonate aqueous solution, and water, successively, and then dried over anhydrous magnesium sulfate. After concentration under reduced pressure, the residue was purified by a silica gel column chromatography to give 5-(4-biphenylmethyl)-1-(2-tert-butoxycarbonylisoindolin-5-yl)-2,4-dioxo-2,3,4,5-tetrahydro-1H-1,5-benzodiazepine (1.2 g, 51%) as amorphous solid.
$^1$H-NMR($CDCl_3$)δ: 1.47(4.5H, s), 1.49(4.5H, s), 3.58(2H, s), 4.47-4.59(4H, m), 4.74(0.5H, d, J=14.6Hz), 4.80(0.5H, d, J=12.4Hz), 5.80(0.5H, d, J=12.4Hz), 5.88(0.5H, d, J=14.6Hz), 6.32(0.5H, d, J=7.8Hz), 6.45(0.5H, d, J=9.2Hz), 6.79-7.55(15H, m).

(8) Methyl 5-(4-biphenylmethyl)-1-(2-tert-butoxycarbonylisoindolin-5-yl)-2,4-dioxo-2,3,4,5-tetrahydro-1H-1,5-benzodiazepine-3-acetate

To a stirred N,N-dimethylformamide (10 ml) solution of 5-(4-biphenylmethyl)-1-(2-tert-butoxycarbonylisoindolin-5-yl)-2,4-dioxo-2,3,4,5-tetrahydro-1H-1,5-benzodiazepine (1.1g, 1.9 mmol) was added 60% oily sodium hydride (92 mg, 2.3 mmol). After stirring at room temperature for 15 minutes, methyl bromoacetate (0.22 ml, 2.3 mmol) was added thereto. The resulting mixture was stirred at room temperature for 15 minutes. Thereafter, the reaction solution was poured into ice-water and extracted with ethyl acetate. After washing with water, the extract solution was dried over anhydrous magnesium sulfate. After concentration under reduced pressure, the precipitated crystals were recrystallized from ethyl acetate to give methyl 5-(4-biphenylmethyl)-1-(2-tert-butoxycarbonylisoindolin-5-yl)-2,4-dioxo-2,3,4,5-tetrahydro-1H-1,5-benzodiazepine-3-acetate (0.56 g, 46%) as amorphous solid.
$^1$H-NMR($CDCl_3$)δ: 1.40(4.5H, s), 1.50(4.5H, s), 3.19(2H, d, J=7.0Hz), 3.71(3H, s), 3.97(1H, t, J=7.0Hz), 4.46(1H, bs), 4.56(1H, bs), 4.58(2H, bs), 4.77(0.5H, d, J=14.8Hz), 4.84(0.5H, d, J=13.4Hz), 5.78(0.5H, d, J=13.4Hz), 5.84(0.5H, d, J=14.8Hz), 6.24(0.5H, d, J=10Hz), 6.39(0.5H, d, J=8.2Hz), 6.84-7.59(15H, m).

(9) 5-(4-biphenylmethyl)-1-(2-tert-butoxycarbonylisoindolin-5-yl)-2,4-dioxo-2,3,4,5-tetrahydro-1H-1,5-benzodiazepine-3-acetic acid

To a stirred tetrahydrofuran (15 ml) and methanol (15 ml) solution of methyl 5-(4-biphenylmethyl)-1-(2-tert-butoxycarbonylisoindolin-5-yl)-2,4-dioxo-2,3,4,5-tetrahydro-lH-1,5-benzodiazepine-3-acetate (0.5 g, 0.8 mmol) was added IN sodium hydroxide aqueous solution. (1.6 ml, 1.6 mmol). The resulting mixture was stirred at 60°C for 2 hours. After cooling of the reaction solution, water and potassium hydrogen sulfate (0.32 g, 2.4 mmol) were added thereto. The mixture was extracted with ethyl acetate and, after washing with water, the extract solution was dried over anhydrous magnesium sulfate. The solution was concentrated under reduced pressure to give 5-(4-biphenylmethyl)-1-(2-tert-butoxycarbonylisoindolin-5-yl)-2,4-dioxo-2,3,4,5-tetrahydro-1H-1,5-benzodiazepine-3-acetic acid (0.46 g, 94%) as an oil.
$^1$H-NMR($CDCl_3$)δ: 1.46(4.5H, s), 1.49(4.5H, s), 3.22(2H, d, J=7.0Hz), 3.93(2H, t, J=7.0Hz), 4.46(1H, bs), 4.55(1H, bs), 4.58(2H, bs), 4.79(0.5H, d, J=14.2Hz), 4.85(0.5H, d, J=13Hz), 5.76(0.5H, d, J=13Hz), 5.83(0.5H, d, J=14.2Hz), 6.24 (0.5H, d, J=9.6Hz), 6.40(0.5H, d, J=9.2Hz), 6.69-7.61(15H, m).

(10) N-(2-Fluorobenzyl)-5-(4-biphenylmethyl)-1-(2-tert-butoxycarbonylisoindolin-5-yl)-2,4-dioxo-2,3,4,5-tetrahydro-1H-1,5-benzodiazepine-3-acetamide

To an ice-cooled stirred N,N-dimethylformamide (3 ml) solution of 5-(4-biphenylmethyl)-1-(2-tert-butoxycarbonylisoindolin-5-yl)-2,4-dioxo-2,3,4,5-tetrahydro-lH-1,5-benzodiazepine-3-acetic acid (0.25 g, 0.4 mmol) were added 2-fluorobenzylamine (0.055 ml, 0.48 mmol), diethyl cyanophosphate (0.078 ml, 0.52 mmol) and triethylamine (0.073 ml, 0.52 mmol). The resulting mixture was stirred at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-fluorobenzyl)-5-(4-biphenylmethyl)-1-(2-tert-butoxycarbonylisoindolin-5-yl)-2,4-dioxo-2,3,4,5-tetrahydro-1H-1,5-benzodiazepine-3-acetamide (180 mg, 62%) as amorphous solid.
$^1$H-NMR($CDCl_3$)δ: 1.45(4.5H, s), 1.49(4.5H, s), 3.04(2H, d, J=7.0Hz), 4.08(2H, d, J=7.0Hz), 4.41-4.58(6H, m), 4.77 (0.5H, d, J=15Hz), 4.82(0.5H, d, J=14.4Hz), 5.76(0.5H, d, J=14.4Hz), 5.83(0.5H, d, J=15.0Hz), 6.21(0.5H, d, J=8.2Hz),

6.35-6.41(1.5H, m), 6.69-7.73(19H, m).

(11) N-(2-Fluorobenzyl)-5-(4-biphenylmethyl)-1-(isoindolin-5-yl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-5-(4-biphenylmethyl)-1-(2-tert-butoxycarbonylisoindolin-5-yl)-2,4-dioxo-2,3,4,5-tetrahydro-1H-1,5-benzodiazepine-3-acetamide (140 mg, 0.19 mmol). The resulting mixture was stirred at room temperature for 1 hour and then concentrated under reduced pressure. The residue was crystallized from ethyl ether to give N-(2-fluorobenzyl)-5-(4-biphenylmethyl)-1-(isoindolin-5-yl)-2,4-dioxo-2,3,4,5-tetrahydro-1H-1,5-benzodiazepine-3-acetamide hydrochloride (140 mg, 86%) as crystals.

Melting point 190-192°C.
Elemental analysis for $C_{39}H_{34}N_4O_3ClF \cdot 2H_2O$;
Calcd.: C, 67.19; H, 5.49; N, 8.04.
Found: C, 67.35; H, 5.20; N, 7.87.
$^1$H-NMR(DMSO-$d_6$)δ: 2.68-3.03(2H, m), 3.95(1H, t, J=7.2Hz), 4.30(2H, d, J=5.8Hz), 4.35(2H, s), 4.45(2H, s), 5.02 (1H, d, J=15Hz), 5.64(1H, d, J=15Hz), 6.60(1H, d, J=8.0Hz). 6.80-7.67(18H, m), 7.83(1H, d, J=8.4Hz), 8.61(1H, t, J=5.8Hz), 9.68(2H, bs).

Example 9

N-(2-Fluorobenzyl)-1-(3-aminomethylphenyl)-5-(4-biphenylmethyl)-2,4-dioxo-2,3,4,5-tetrahydro-1H-1,5-benzodiazepine-3-acetamide hydrochloride

(1) tert-Butyl (3-aminobenzyl)carbamate

Di-tert-butyl dicarbonate (56.5 ml, 246 mmol) was added dropwise to an ice-cooled stirred tetrahydrofuran (500 ml) solution of 3-aminobenzylamine (30 g, 246 mmol). The resulting reaction mixture was stirred at 0°C for 1 hour. The reaction solution was concentrated under reduced pressure and the residue was diluted with ethyl acetate. After washing with water, the solution was dried over anhydrous magnesium sulfate. The solvent was removed by evaporation under reduced pressure to give tert-butyl (3-aminobenzyl)carbamate (54 g, 99%) as an oil.
$^1$H-NMR($CDCl_3$)δ: 1.43(9H, s), 3.67(2H, bs), 4.21(2H, d, J=6Hz), 4.83(1H, bs), 6.56-6.67(3H, m), 7.10(1H, t, J=7.4Hz).

(2) tert-Butyl [3-(2-nitrophenylamino)benzyl]carbamate

A mixture of tert-butyl (3-aminobenzyl)carbamate (54 g, 243 mmol), o-fluoronitrobenzene (51.3 g, 243 mmol) and potassium carbonate (38.6 g, 243 mmol) was stirred at 140°C for 2 hours under nitrogen atmosphere. After cooling, the reaction mixture was diluted with ethyl acetate, washed with water, and, after drying over anhydrous magnesium sulfate, concentrated under reduced pressure. The residue was purified by a silica gel column chromatography to give tert-butyl [3-(2-nitrophenylamino)benzyl]carbamate (34.3 g, 41%) as an oil.
$^1$H-NMR($CDCl_3$)δ: 1.45(9H, s), 4.25-4.34(2H, m), 4.93(1H, bs), 6.78(1H, t, J=8.4Hz), 7.11-7.41(5H, m), 8.19 (1H, d, J=8.4Hz), 9.47(1H, bs).

(3) tert-Butyl [3-(2-aminophenylamino)benzyl]carbamate

Palladium supported on carbon (5%, 3.5 g) was added to an ethanol solution (500 ml) of tert-butyl [3-(2-nitrophenylamino)benzyl]carbamate (34.3 g, 99.8 mmol). The resulting mixture was subjected to hydrogenation for 2 hours under the conditions of ambient temperature and normal pressure. The catalyst was removed by filtration and the filtrate was concentrated under reduced pressure to give tert-butyl [3-(2-aminophenylamino)benzyl]carbamate (27 g, 86%) as an oil.
$^1$H-NMR($CDCl_3$)δ: 1.45(9H, s), 3.74(2H, bs), 4.24(2H, dd, J=6.2Hz,9.2Hz), 4.83(1H, bs), 5.21(1H, bs), 6.51-7.33(8H, m).

(4) tert-Butyl [3-[2-(4-biphenylmethylamino)phenylamino]benzyl]carbamate

Acetic acid (4.8 ml, 83.9 mmol) was added to a methanol solution (500 ml) of tert-butyl [3-(2-aminophenylamino)benzyl]carbamate (26.3 g, 83.9 mmol) and 4-phenylbenzaldehyde (15.3 g, 83.9 mmol). The resulting mixture was

stirred at 0°C for 30 minutes and then sodium cyanoborohydride (6.4 g, 105 mmol) was added thereto. Thereafter, the mixture was stirred at 0°C for 1 hour and at room temperature for 30 minutes. Then, the reaction solution was poured into water and extracted with ethyl acetate. After washing with water, the extract solution was dried over anhydrous magnesium sulfate and concentrated under reduced pressure. The residue was purified by a silica gel column chromatography to give tert-butyl [3-[2-(4-biphenylmethylamino)phenylamino]benzyl]carbamate (22.7 g, 57%) as amorphous solid.

Elemental analysis for $C_{31}H_{33}N_3O_2 \cdot 0.5H_2O$;
Calcd.: C, 76.20; H, 7.01; N, 8.60.
Found: C, 76.01; H, 7.15; N, 8.72.
$^1$H-NMR($CDCl_3$)δ: 1.45(9H, s), 4.22-4.40(4H, m), 4.63(1H, bs), 4.80(1H, bs), 5.16(1H, bs), 6.49-7.60(17H, m).

(5) 5-(4-Biphenylmethyl)-1-(3-tert-butoxycarbonylaminomethylphenyl)-2,4-dioxo-2,3,4,5-tetrahydro-1H-1,5-benzodiazepine

Potassium carbonate (3.45 g, 25 mmol) was added to an ice-cooled stirred tetrahydrofuran (500 ml) solution of tert-butyl [3-[2-(4-biphenylmethylamino)phenylamino]benzyl]carbamate (10 g, 20.8 mmol). Then, a tetrahydrofuran solution (10 ml) of malonyl dichloride (2.43 ml, 25 mmol) was added dropwise. The resulting mixture was stirred at 0°C for 30 minutes and then potassium carbonate (3.45 g, 25 mmol) was further added thereto. Then, a tetrahydrofuran solution (10 ml) of malonyl dichloride (2.43 ml, 25 mmol) was added dropwise, followed by stirring for 1 hour. The mixture was further stirred at room temperature for 1 hour and at 60°C for 1 hour. The reaction solution was poured into water and extracted with ethyl acetate. After washing with water, the extract solution was dried over anhydrous magnesium sulfate. The solution was concentrated under reduced pressure and the residue was purified by a silica gel column chromatography to give 5-(4-biphenylmethyl)-1-(3-tert-butoxycarbonylaminomethylphenyl)-2,4-dioxo-2,3,4,5-tetrahydro-1H-1,5-benzodiazepine (1.1 g, 10%) as amorphous solid.
$^1$H-NMR($CDCl_3$)δ: 1.43(9H, s), 3.58(2H, s), 4.12(2H, d, J=7.4Hz), 4.73(1H, bs), 4.77(1H, d, J=14.8Hz), 5.84(1H, d, J=14.8Hz), 6.38(1H, bs), 6.80-7.57(16H, m).

(6) Methyl 5-(4-biphenylmethyl)-1-(3-tert-butoxycarbonylaminomethylphenyl)-2,4-dioxo-2,3,4,5-tetrahydro-1H-1,5-benzodiazepine-3-acetate

To a stirred N,N-dimethylformamide (20 ml) solution of 5-(4-biphenylmethyl)-1-(3-tert-butoxycarbonylaminomethylphenyl)-2,4-dioxo-2,3,4,5-tetrahydro-1H-1,5-benzodiazepine (1 g, 1.83 mmol) was added 60% oily sodium hydride (0.22 g, 5.5 mmol). After stirring at room temperature for 10 minutes, methyl bromoacetate (0.35 ml, 3.66 mmol) was added thereto. The resulting mixture was stirred at room temperature for 1 hour. The reaction solution was poured into water and extracted with ethyl acetate. After washing with water, the extract solution was dried over anhydrous magnesium sulfate. After concentrating under reduced pressure, the residue was purified by a silica gel column chromatography to give ethyl 5-(4-biphenylmethyl)-1-(3-tert-butoxycarbonylaminomethylphenyl)-2,4-dioxo-2,3,4,5-tetrahydro-1H-1,5-benzodiazepine-3-acetate (0.7 g, 62%) as amorphous solid. $^1$H-NMR($CDCl_3$)δ: 1.42(9H, s), 3.15-3.20(2H, m), 3.71(3H, s), 3.97(1H, t, J=7Hz), 4.12(2H, d, J=7Hz), 4.74(1H, bs), 4.81(1H, d, J=14.8Hz), 5.84(1H, d, J=14.8Hz), 6.32(1H, d, J=6.8Hz), 6.84-7.58(16H, m).

(7) 5-(4-Biphenylmethyl)-1-(3-tert-butoxycarbonylaminomethylphenyl)-2,4-dioxo-2,3,4,5-tetrahydro-1H-1,5-benzodiazepine-3-acetic acid

To a stirred tetrahydrofuran (6 ml) and methanol (18 ml) solution of methyl 5-(4-biphenylmethyl)-1-(3-tert-butoxycarbonylaminomethylphenyl)-2,4-dioxo-2,3,4,5-tetrahydro-1H-1,5-benzodiazepine-3-acetate (0.65 g, 1 mmol) was added IN sodium hydroxide aqueous solution (4.5 ml, 4.5 mmol). The resulting mixture was stirred at 40°C for 1 hour. After cooling of the reaction solution, water and potassium hydrogen sulfate (0.61 g, 4.5 mmol) were added thereto. The mixture was extracted with ethyl acetate and, after washing with water, dried over anhydrous magnesium sulfate. The solution was concentrated under reduced pressure to give 5-(4-biphenylmethyl)-1-(3-tert-butoxycarbonylaminomethylphenyl)-2,4-dioxo-2,3,4,5-tetrahydro-1H-1,5-benzodiazepine-3-acetic acid (0.63 g, 99%) as amorphous solid.
$^1$H-NMR($CDCl_3$)δ: 1.42(9H, s), 3.20(2H, d, J=7.4Hz), 3.93(1H, t, J=7.4Hz), 4.12(2H, d, J=7Hz), 4.78(1H, bs), 4.81(1H, d, J=15Hz), 5.81(1H, d, J=15Hz), 6.29(1H, d, J=6.8Hz), 6.83-7.58(16H, m).

(8) N-(2-Fluorobenzyl)-5-(4-biphenylmethyl)-1-(3-tert-butoxycarbonylaminomethylphenyl)-2,4-dioxo-2,3,4,5-tetrahydro-1H-1,5-benzodiazepine-3-acetamide

To an ice-cooled stirred N,N-dimethylformamide (1 ml) solution of 5-(4-biphenylmethyl)-1-(3-tert-butoxycarbonylaminomethylphenyl)-2,4-dioxo-2,3,4,5-tetrahydro-1H-1,5-benzodiazepine-3-acetic acid (0.1 g, 0.17 mmol) were added o-fluorobenzylamine (0.021 ml, 0.18 mmol), diethyl cyanophosphate (0.06 ml, 0.2 mmol) and triethylamine (0.028 ml, 0.2 mmol). The resulting mixture was stirred at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-fluorobenzyl)-5-(4-biphenylmethyl)-1-(3-tert-butoxycarbonylaminomethylphenyl)-2,4-dioxo-2,3,4,5-tetrahydro-1H-1,5-benzodiazepine-3-acetamide (68 mg, 58%) as amorphous solid.
$^1$H-NMR($CDCl_3$)δ: 1.43(9H, s), 3.02(2H, d, J=7Hz), 4.08(1H, t, J=7Hz), 4.09-4.18(2H, m), 4.50(2H, dd, J=3.2Hz, 5.8Hz), 4.72(1H, bs), 4.80(1H, d, J=15Hz), 5.79(1H, d, J=15Hz), 6.28-6.39(2H, m), 6,79-7.56(20H, m).

(9) N-(2-Fluorobenzyl)-1-(3-aminomethylphenyl)-5-(4-biphenylmethyl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-5-(4-biphenylmethyl)-1-(3-tert-butoxycarbonylaminomethylphenyl)-2,4-dioxo-2,3,4,5-tetrahydro-lH-1,5-benzodiazepine-3-acetamide (68 mg, 0.095 mmol). The resulting mixture was stirred at room temperature for 1 hour and then concentrated under reduced pressure. The residue was solidified from ethyl ether to give N-(2-fluorobenzyl)-1-(3-aminomethylphenyl)-5-(4-biphenylmethyl)-2,4-dioxo-2,3,4,5-tetrahydro-1H-1,5-benzodiazepine-3-acetamide hydrochloride (50 mg, 81%) as amorphous solid.
$^1$H-NMR(DMSO-$d_6$)δ: 2.66-2.73(2H, m), 3.73-3.79(3H, m), 4.07-4.12(2H, m), 4.86(1H, d, J=15.8Hz), 5.38(1H, d, J=15.8Hz), 6.16(1H, d, J=8Hz), 6.66(1H, d, J=6.6Hz), 6.89-7.44(18H, m), 7.58(1H, d, J=7.8Hz), 8.02(3H, bs), 8.40(1H, t, J=5.4Hz).

Example 10

N-(2-Fluorobenzyl)-1-(4-aminomethylphenyl)-5-[4-(4-methoxybenzoylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 4-methoxybenzoyl chloride, N-(2-fluorobenzyl)-1-(4-aminomethylphenyl)-5-[4-(4-methoxybenzoylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{40}H_{37}N_5O_5ClF \cdot H_2O$;
Calcd.: C, 64.90; H, 5.31; N, 9.46.
Found: C, 65.07; H, 5.44; N, 9.27.
$^1$H-NMR(DMSO-$d_6$)δ: 2.38-2.52(2H, m), 3.84(3H, s), 3.93-4.01(2H, m), 4.30(2H, d, J=5.6Hz), 4.93(1H, d, J=15.2Hz), 5.56(1H, d, J=15.2Hz), 6.77-7.47(15H, m), 7.66(2H, d, J=8.6Hz), 7.79(1H, d, J=8.4Hz), 7.98(2H, d, J=8.8Hz), 8.31(3H, bs), 8.62(1H, t, J=5.8Hz), 10.15(1H, bs).

Example 11

N-(2-Fluorobenzyl)-1-(4-aminomethylphenyl)-5-[4-(4-chlorobenzoylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 4-chlorobenzoyl chloride, N-(2-fluorobenzyl)-1-(4-aminomethylphenyl)-5-[4-(4-chlorobenzoylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{39}H_{34}N_5O_4Cl_2F \cdot H_2O$;
Calcd.: C, 62.91; H, 4.87; N, 9.41.
Found: C, 62.81; H, 4.87; N, 9.14.
$^1$H-NMR(DMSO-$d_6$)δ: 2.76-3.03(2H, m), 3.88-4.00(3H, m), 4.30(2H, d, J=5.6Hz), 4.96(1H, d, J=15.7Hz), 5.58(1H,

d, J=15.7Hz), 6.78-7.81(18H, m), 8.02(2H, d, J=8.6Hz), 8.37(3H, bs), 8.62(1H, t, J=5.6Hz), 10.42(1H, bs).

Example 12

N-(2-Fluorobenzyl)-1-(4-aminomethylphenyl)-5-[4-(4-trifluoromethylbenzoylamino)benzyl)-2,4-dioxo-2,3,4,5-tetrahydro-lH-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 4-trifluoromethylbenzoyl chloride, N-(2-fluorobenzyl)-1-(4-aminomethylphenyl)-5-[4-(4-trifluoromethylbenzoylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{40}H_{34}N_5O_4ClF_4 \cdot H_2O$;
Calcd.: C, 61.88; H, 4.65; N, 9.02.
Found: C, 62.10; H, 4.95; N, 8.82.
$^1$H-NMR(DMSO-$d_6$)δ: 2.78-3.03(2H, m), 3.90-4.01(3H, m), 4.30(2H, d, J=5.8Hz), 4.98(1H, d, J=15.2Hz), 5.58(1H, d, J=15.2Hz), 6.77-7.47(13H, m), 7.68(2H, d, J=8.6Hz), 7.78(1H, d, J=8.4Hz), 7.91(2H, d, J=8.4Hz), 8.18(2H, d, J=8,0Hz), 8.32(3H, bs), 8.61(1H, t, J=5.8Hz), 10.55(1H, bs).

Example 13

N-(2-Fluorobenzyl)-1-(4-aminomethylphenyl)-5-[4-(2-furoylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 2-furoyl chloride, N-(2-fluorobenzyl)-1-(4-aminomethylphenyl)-5-[4-(2-furoylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{37}H_{33}N_5O_5ClF \cdot 1.5H_2O$;
Calcd.: C, 62.66; H, 5.12; N, 9.88.
Found: C, 62.75; H, 4.97; N, 9.58.
$^1$H-NMR(DMSO-$d_6$)δ: 2.71-3.03(2H, m), 3.89-4.02(3H, m), 4.30(2H, d, J=5.6Hz), 4.94(1H, d, J=15.8Hz), 5.58(1H, d, J=15.8Hz), 6.68-7.47(15H, m), 7.66(2H, d, J=8.6Hz), 7.79(1H, d, J=7.4Hz), 7.93(1H,s), 8.32(3H, bs), 8.62(1H, t, J=5.6Hz), 10.25(1H, bs).

Example 14

N-(2-Fluorobenzyl)-1-(4-aminomethylphenyl)-5-[4-(2-thenoylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 2-thenoyl chloride, N-(2-fluorobenzyl)-1-(4-aminomethylphenyl)-5-[4-(2-thenoylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{37}H_{33}N_5O_4ClFS \cdot 2H_2O$;
Calcd.: C, 60.52; H, 5.08; N, 9.54.
Found: C, 60.81; H, 4.92; N, 9.42.
$^1$H-NMR(DMSO-$d_6$)δ: 2.76-3.03(2H, m), 3.89-4.00(3H, m), 4.30(2H, d, J=5.2Hz), 4.94(1H, d, J=15.0Hz), 5.59(1H, d, J=15.0Hz), 6.78-7.87(18H, m), 8.16(2H, d, J=2.8Hz), 8.38(3H, bs), 8.62(1H, t, J=5.2Hz), 10.39(1H, bs).

Example 15

N-(2-Fluorobenzyl)-1-(4-aminomethylphenyl)-5-[4-(cyclohexylcarbonylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and cyclohexylcarbonyl chloride, N-(2-fluorobenzyl)-1-(4-aminomethylphenyl)-5-[4-(cyclohexanecarbonylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{39}H_{41}N_5O_4ClF \cdot H_2O$;
Calcd.: C, 65.40; H, 6.05; N, 9.78.
Found: C, 65.32; H, 5.78; N, 9.50.
$^1$H-NMR(DMSO-$d_6$)δ: 1.18-2.43(11H, m), 2.76-3.01(2H, m), 3.90(1H, t, J=7.0Hz), 4.01(2H, bs), 4.29(2H, d, J=5.4Hz), 4.90(1H, d, J=15.0Hz), 5.54(1H, d, J=15.0Hz), 6.71-7.51(15H, m), 7.76(1H, d, J=8.0Hz), 8.35(3H, bs), 8.61(1H, t, J=5.4Hz), 9.91(1H, bs).

Example 16

N-(2-Fluorobenzyl)-1-(4-aminomethylphenyl)-5-[4-(pivaloylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and pivaloyl chloride, N-(2-fluorobenzyl)-1-(4-aminomethylphenyl)-5-[4-(pivaloylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{37}H_{39}N_5O_4ClF \cdot 1.5H_2O$;
Calcd.: C, 63.56; H, 6.05; N, 10.02.
Found: C, 63.58; H, 6.05; N, 9.79.
$^1$H-NMR(DMSO-$d_6$)δ: 1.28(9H, s), 2.73-3.01(2H, m), 3.91(1H, t, J=7.0Hz), 3.98(2H, bs), 4.29(2H, d, J=5.8Hz), 4.90(1H, d, J=15.0Hz), 5.55(1H, d, J=15.0Hz), 6.73-7.43(13H, m), 7.53(2H, d, J=8.4Hz), 7.76(1H, d, J=8.4Hz), 8.36(3H, bs), 8.60(1H, t, J=5.8Hz), 9.27(1H, bs).

Example 17

N-(2-Fluorobenzyl)-1-(4-aminomethylphenyl)-5-[4-(benzyloxycarbonylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and benzyl chloroformate, N-(2-fluorobenzyl)-1-(4-aminomethylphenyl)-5-[4-(benzyloxycarbonylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{40}H_{37}N_5O_5ClF \cdot 1.5H_2O$;
Calcd.: C, 64.12; H, 5.38; N, 9.35.
Found: C, 63.96; H, 5.39; N, 9.49.
$^1$H-NMR(DMSO-$d_6$)δ: 2.75-3.02(2H, m), 3.91(1H, t, J=7.2Hz), 3.98-4.15(2H, m), 4.29(2H, d, J=5.6Hz), 4.91(1H, d, J=14.6Hz), 5.15(2H, s), 5.52(1H, d, J=14.6Hz), 6.75-7.78(21H, m), 8.38(3H, bs), 8.62(1H, t, J=5.6Hz), 9.80(1H, bs).

Example 18

N-(2-Fluorobenzyl)-1-(4-aminomethylphenyl)-5-[4-(4-fluorobenzoylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 4-fluorobenzoyl chloride, N-(2-fluorobenzyl)-1-(4-aminomethylphenyl)-5-[4-(4-fluorobenzoylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{39}H_{34}N_5O_4ClF_2 \cdot 2H_2O$;
Calcd.: C, 62.77; H, 5.13; N, 9-39.
Found: C, 62.94; H, 5.04; N, 9.20.
$^1$H-NMR(DMSO-$d_6$)δ: 2.78-3.03(2H, m), 3.93(1H, t, J=7.8Hz), 4.02(2H, bs), 4.29(2H, d, J=5.2Hz), 4.96(1H, d, J=15.8Hz), 5.58(1H, d, J=15.8Hz), 6.77-8.10(20H, m), 8.32(3H, bs), 8.61(1H, t, J=5.2Hz), 10.33(1H, bs).

Example 19

N-(2-Fluorobenzyl)-1-(4-aminomethylphenyl)-5-[4-(3-chlorobenzoylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 3-chlorobenzoyl chloride, N-(2-fluorobenzyl)-1-(4-aminomethylphenyl)-5-[4-(3-chlorobenzoylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{39}H_{34}N_5O_4Cl_2F \cdot 1.5H_2O$;
Calcd.: C, 62.15; H, 4.95; N, 9.29.
Found: C, 62.23; H, 4.75; N, 9.06.
$^1$H-NMR(DMSO-$d_6$)δ: 2.78-3.03(2H, m), 3.90-4.01(3H, m), 4.29(2H, d, J=5.4Hz), 4.97(1H, d, J=14.6Hz), 5.58(1H, d, J=14.6Hz), 6.77-7.81(18H, m), 7.94(1H, d, J=7.4Hz), 8.03(1H, s), 8.33(3H, bs), 8.62(1H, t, J=5.4Hz), 10.43(1H, bs).

Example 20

N-(2-Fluorobenzyl)-1-(4-aminomethylphenyl)-5-[4-(2-chlorobenzoylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-lH-1,5-benzodiazepine-3-acetate and 2-chlorobenzoyl chloride, N-(2-fluorobenzyl)-1-(4-aminomethylphenyl)-5-[4-(2-chlorobenzoylamino)benzyl]-2,4-dioxo-2,3,4,5-tetrahydro-1H-1,5-benzodiazepine-3-acetamide hydrochloride was obtained as amorphous solid in similar manners to (6), (7), (8) and (9) of Example 5.

Elemental analysis for $C_{39}H_{34}N_5O_4Cl_2F \cdot H_2O$;
Calcd.: C, 62.91; H, 4.87; N, 9.41.
Found: C, 63.18; H, 4.90; N, 9.23.
$^1$H-NMR(DMSO-$d_6$)δ: 2.74-3.03(2H, m), 3.89-4.02(3H, m), 4.29(2H, d, J=6.2Hz), 4.95(1H, d, J=15.2Hz), 5.56(1H, d, J=15.2Hz), 6.76-7.80(20H, m), 8.37(3H, bs), 8.62(1H, t, J=6.2Hz), 10.56(1H, bs).

Example 21

N-(2-Fluorobenzyl)-1-(4-aminomethylphenyl)-5-[4-(benzenesulfonylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and benzenesulfonyl chloride, N-(2-fluorobenzyl)-1-(4-aminomethylphenyl)-5-[4-(benzenesulfonylamino)benzyl]-2,4-dioxo-2,3,4,5-tetrahydro-1H-1,5-benzodiazepine-3-acetamide hydrochloride was obtained as amorphous solid in similar manners to (1), (2), (3) and (4) of Example 6.

Elemental analysis for $C_{38}H_{35}N_5O_5ClFS \cdot 1.5H_2O$;
Calcd.: C, 60.43; H, 5.07; N, 9.27.
Found: C, 60.45; H, 5.09; N, 8.98.
$^1$H-NMR(DMSO-$d_6$)δ: 2.76-2.99(2H, m), 3.97(1H, t, J=7.7Hz), 4.07(2H, bs), 4.28(2H, d, J=5.6Hz), 4.90(1H, d, J=15.8Hz), 5.43(1H, d, J=15.8Hz), 6.74-7.72(21H, m), 8.32(3H, bs), 8.60(1H, t, J=5.6Hz), 10.32(1H, bs).

Example 22

N-(2-Fluorobenzyl)-1-(4-aminomethylphenyl)-5-[4-(4-fluorobenzenesulfonylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 4-fluorobenzenesulfonyl chloride, N-(2-fluorobenzyl)-1-(4-aminomethylphenyl)-5-[4-(4-fluorobenzenesulfonylamino)benzyl]-2,4-dioxo-2,3,4,5-tetrahydro-1H-1,5-benzodiazepine-3-acetamide hydrochloride was obtained as amorphous solid in similar manners to (1), (2), (3) and (4) of Example 6.

Elemental analysis for $C_{38}H_{34}N_5O_5ClF_2S \cdot 2H_2O$;
Calcd.: C, 58.35; H, 4,90; N, 8.95.
Found: C, 58.57; H, 4,57; N, 8.83.
$^1$H-NMR(DMSO-$d_6$)δ: 2.78-2.94(2H, m), 3.89(1H, t, J=6.8Hz), 4.03(2H, d, J=7.4Hz), 4.28(2H, d, J=5.0Hz), 4.90 (1H, d, J=15.4Hz), 5.44(1H, d, J=15.4Hz), 6.77-7.79(20H, m), 8.36(3H, bs), 8.59(1H, t, J=5.0Hz), 10.35(1H, bs).

Example 23

N-(2-Fluorobenzyl)-1-(4-aminomethylphenyl)-5-[4-(4-methylbenzenesulfonylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 4-methylbenzenesulfonyl chloride, N-(2-fluorobenzyl)-1-(4-aminomethylphenyl)-5-[4-(4-methylbenzenesulfonylamino)benzyl]-2,4-dioxo-2,3,4,5-tetrahydro-1H-1,5-benzodiazepine-3-acetamide hydrochloride was obtained as amorphous solid in similar manners to (1), (2), (3) and (4) of Example 6.

Elemental analysis for $C_{39}H_{37}N_5O_5ClFS \cdot 2H_2O$;
Calcd.: C, 60.19; H, 5.31; N, 9.00.
Found: C, 60.38; H, 5.01; N, 8.93.
$^1$H-NMR(DMSO-$d_6$)δ: 2.83-2.89(2H, m), 3.89(1H, t, J=7.4Hz), 4.08-4.15(2H, m), 4.28(2H, d, J=6.0Hz), 4.89(1H, d, J=15.0Hz), 5.42(1H, d, J=15.0Hz), 6.75-7.67(20H, m), 8.36(3H, bs), 8.60(1H, t, J=6.0Hz), 10.24(1H, bs).

Example 24

N-(2-Fluorobenzyl)-1-(4-aminomethylphenyl)-5-[4-(4-trifluoromethyloxybenzenesulfonylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 4-trifluoromethyloxybenzenesulfonyl chloride, N-(2-fluorobenzyl)-1-(4-aminomethylphenyl)-5-[4-(4-trifluoromethyloxybenzenesulfonylamino)benzyl]-2,4-dioxo-2.3,4,5-tetrahydro-1H-1,5-benzodiazepine-3-acetamide hydrochloride was obtained as amorphous solid in similar manners to (1), (2), (3) and (4) of Example 6.

Elemental analysis for $C_{39}H_{34}N_5O_5ClF_4S \cdot 2H_2O$;
Calcd.: C, 56.28; H, 4.60; N, 8.41.
Found: C, 56.21; H, 4.37; N, 8.17.
$^1$H-NMR(DMSO-$d_6$)δ: 2.83-2.89(2H, m), 3.89(1H, t, J=6.6Hz), 4.03(2H, d, J=5.2Hz), 4.28(2H, d, J=4.4Hz), 4.92 (1H, d, J=15.0Hz), 5.43(1H, d, J=15.0Hz), 6.75-7.53(17H, m), 7.65(1H, d, J=8.4Hz), 7.83(2H, d, J=8.8Hz), 8.34 (3H, bs), 8.60(1H, t, J=4.4Hz), 10.48(1H, bs).

Example 25

N-(2-Fluorobenzyl)-1-(4-aminomethylphenyl)-5-[4-(4-nitrobenzenesulfonylamino)benzyl]-2,4-dioxo-2,3,4,5-tetrahydro-1H-1,5-benzodiazepine-3-acetamide hydrochloride

Starting with methyl 5-(4-aminobenzyl)-1-(4-tert-butoxycarbonylaminomethylphenyl)-2,4-dioxo-2,3,4,5-tetrahydro-1H-1,5-benzodiazepine-3-acetate and 4-nitrobenzenesulfonyl chloride, N-(2-fluorobenzyl)-1-(4-aminomethylphenyl)-5-[4-(4-nitrobenzenesulfonylamino)benzyl]-2,4-dioxo-2,3,4,5-tetrahydro-lH-1,5-benzodiazepine-3-acetamide

hydrochloride was obtained as amorphous solid in similar manners to (1), (2), (3) and (4) of Example 6.

Elemental analysis for $C_{38}H_{34}N_6O_7ClFS \cdot 1.5H_2O$;
Calcd.: C, 57.03; H, 4.66; N, 10.50.
Found: C, 56.97; H, 4.46; N, 10.23.
$^1$H-NMR(DMSO-$d_6$)δ: 2.83-2.90(2H, m), 3.89(1H, t, J=6.6Hz), 4.01-4.05(2H, m), 4.27(2H, d, J=5.4Hz), 4.92(1H, d, J=15.4Hz), 5.44(1H, d, J=15.4Hz), 6.75-7.97(18H, m), 8.27-8.34(5H, m), 8.60(1H, t, J=5.4Hz), 10.68(1H, bs).

Example 26

N-(2-Fluorobenzyl)-5-[4-(4-aminobenzenesulfonylamino)benzyl]-1-(4-aminomethylphenyl)-2,4-dioxo-2,3,4,5-tetrahydro-1H-1,5-benzodiazepine-3-acetamide dihydrochloride

(1) Palladium supported on carbon (5%, 0.04 g) was added to an ethanol (8 ml) solution of N-(2-fluorobenzyl)-1-(4-tert-butoxycarbonylaminomethylphenyl)-5-[4-(4-nitrobenzenesulfonylaminobenzyl)-2,4-dioxo-2,3,4,5-tetrahydro-1H-1,5-benzodiazepine-3-acetamide (0.4 g, 0.48 mmol). The resulting mixture was subjected to hydrogenation for 7 hours under the conditions of ambient temperature and normal pressure. The catalyst was removed by filtration and the filtrate was concentrated under reduced pressure to give N-(2-fluorobenzyl)-5-[4-(4-aminobenzenesulfonylaminobenzyl)-1-(4-tert-butoxycarbonylaminomethylphenyl)-2,4-dioxo-2,3,4,5-tetrahydro-lH-1,5-benzodiazepine-3-acetamide (0.33 g, 87%) as an oil.
$^1$H-NMR($CDCl_3$)δ: 1.46(9H, s), 2.88-3.15(2H,m), 4.05-4.46(7H, m), 5.77(1H, d, J=14.4Hz), 5.79(1H, bs), 6.25(1H, bs), 6.42-7.53(20H, m), 8.10(1H, bs).

(2) Starting with N-(2-fluorobenzyl)-5-[4-(4-aminobenzenesulfonylaminobenzyl)-1-(4-tert-butoxycarbonylaminomethylphenyl)-2,4-dioxo-2,3,4,5-tetrahydro-1H-1,5-benzodiazepine-3-acetamide, N-(2-fluorobenzyl)-5-[4-(4-aminobenzenesulfonylamino)benzyl]-1-(4-aminomethylphenyl)-2,4-dioxo-2,3,4,5-tetrahydro-1H-1,5-benzodiazepine-3-acetamide dihydrochloride was synthesized in a similar manner to (3) of Example 6.
$^1$H-NMR(DMSO-$d_6$)δ: 2.80-2.98(2H, m), 3.91-4.22(3H, m), 4.31(2H, bs), 4.93(1H, bs), 5.42(1H, bs). 6.52-7.75 (20H, m), 8.48(4H, bs), 8.61(3H, bs), 9.93(1H, bs).

Example 27

N-(2-Fluorobenzyl)-5-[4-(4-acetamidobenzenesulfonylamino)benzyl]-1-(4-aminomethylphenyl)-2,4-dioxo-2,3,4,5-tetrahydro-1H-1,5-benzodiazepine-3-acetamide hydrochloride

(1) To a tetrahydrofuran (4 ml) solution of N-(2-fluorobenzyl)-5-[4-(4-aminobenzenesulfonylaminobenzyl)-1-(4-tert-butoxycarbonylaminomethylphenyl)-2,4-dioxo-2,3,4,5-tetrahydro-1H-1,5-benzodiazepine-3-acetamide (0.24 g, 0.30 mmol) were added 4-dimethylaminopyridine (3.6 mg, 0.03 mmol), triethylamine (0.054 ml, 0.39 mmol) and acetic anhydride (0.036 g, 0.39 mmol) and the mixture was stirred at room temperature for 1 hour. To the reaction solution were further added triethylamine (0.054 ml, 0.39 mmol) and acetic anhydride (0.036 g, 0.39 mmol) and the mixture was further stirred at room temperature for 24 hours. The reaction solution was poured into water and extracted with ethyl acetate. The extract solution was washed with water and then dried over anhydrous magnesium sulfate, followed by concentration under reduced pressure. The residue was solidified from diisopropyl ether to give N-(2-fluorobenzyl)-5-[4-(4-acetamidobenzenesulfonylaminobenzyl)-1-(4-tert-butoxycarbonylaminomethylphenyl)-2,4-dioxo-2,3,4,5-tetrahydro-1H-1,5-benzodiazepine-3-acetamide (0.22 g, 87%) as solid.
$^1$H-NMR($CDCl_3$)δ: 1.41(9H, s), 2.21(1.5H, s), 2.32(1.5H, s), 3.04(2H, d, J=7.0Hz), 4.10(1H, t, J=7.0Hz), 4.24(2H, bs), 4.47-4.53(2H, m), 4.91(1H, d, J=15.2Hz), 4.95(1H, bs), 5.72(1H, d, J=15.2Hz), 6.34(1H, bs), 6.74-7.47(18H, m), 7.62(2H, d, J=8.4Hz), 7.98(2H, d, J=8.4Hz).

(2) Starting with N-(2-fluorobenzyl)-5-[4-(4-acetamidobenzenesulfonylaminobenzyl)-1-(4-tert-butoxycarbonylaminomethylphenyl)-2,4-dioxo-2,3,4,5-tetrahydro-1H-1,5-benzodiazepine-3-acetamide, N-(2-fluorobenzyl)-5-[4-(4-acetamidobenzenesulfonylamino)benzyl]-1-(4-aminomethylphenyl)-2,4-dioxo-2,3,4,5-tetrahydro-lH-1,5-benzodiazepine-3-acetamide hydrochloride was synthesized in a similar manner to (3) of Example 6.
$^1$H-NMR(DMSO-$d_6$)δ: 2.12(1.5H, s), 2.41(1.5H, s), 2.83-2.95(2H, m), 3.89-4.00(3H, m), 4.31(2H, bs), 5.14(1H, d,

J=15.6Hz), 5.63(1H, d, J=15.6Hz), 6.77-8.00(22H, m), 8.40(3H, bs), 8.64(1H, bs).

Example 28

N-(2-Fluorobenzyl)-1-(4-aminomethylphenyl)-5-[4-[4-(phenylcarbamoylamino)benzyl]-2,4-dioxo-2,3,4,5-tetrahydro-1H-1,5-benzodiazepine-3-acetamide hydrochloride

(1) To a tetrahydrofuran (5 ml) solution of methyl N-(2-fluorobenzyl)-5-(4-aminobenzyl)-1-(4-tert-butoxycarbonylaminomethylphenyl)-2,4-dioxo-2,3,4,5-tetrahydro-lH-1,5-benzodiazepine-3-acetate (0.25 g, 0.45 mmol) was added phenyl isocyanate (0.058 ml, 0.54 mmol), and the mixture was stirred at room temperature for 12 hours. The reaction solution was poured into water and extracted with ethyl acetate. The extract solution was washed with water and then dried over anhydrous magnesium sulfate, followed by concentration under reduced pressure. The residue was solidified from diisopropyl ether to give methyl N-(2-fluorobenzyl)-1-(4-tert-butoxycarbonylaminomethylphenyl)-5-[4-[4-(phenylcarbamoyl)amino] benzyl]-2,4-dioxo-2,3,4,5-tetrahydro-1H-1,5-benzodiazepine-3-acetate (0.27 g, 90%) as solid.
$^1$H-NMR($CDCl_3$)δ: 1.42(9H, s), 3.16(2H, d, J=7.0Hz), 3.66(3H, s), 3.92(1H, t, J=7.0Hz), 4.11(2H, d, J=4.0Hz), 4.45 (1H, d, J=14.0Hz), 5.33(1H, bs), 5.90(1H, d, J=14.0Hz), 6.33(2H, d, J=8.4Hz), 6.78-7.61(16H, m), 7.92(1H, bs).

(2) Starting with methyl N-(2-fluorobenzyl)-1-(4-tert-butoxycarbonylaminomethylphenyl)-5-[4-[4-(phenylcarbamoyl)amino] benzyl]-2,4-dioxo-2,3,4,5-tetrahydro-1H-1,5-benzodiazepine-3-acetate, N-(2-fluorobenzyl)-1-(4-aminomethylphenyl)-5-[4-[4-(phenylcarbamoylamino)benzyl]-2,4-dioxo-2,3,4,5-tetrahydro-1H-1,5-benzodiazepine-3-acetamide hydrochloride was synthesized in similar manners to (2),(3) and (4) of Example 6.

Elemental analysis for $C_{39}H_{36}N_6O_4ClF \cdot 1.5H_2O$;
Calcd.: C, 63.80; H, 5.35; N, 11.45.
Found: C, 63.97; H, 5.44; N, 11.24.
$^1$H-NMR(DMSO-$d_6$)δ: 2.71-3.01(2H, m), 3.90(1H, t, J=7.0Hz), 3.98(2H, bs), 4.29(2H, d, J=5.4Hz), 4.84(1H, d, J=14.8Hz), 5.61(1H, d, J=15.0Hz), 6.66(2H, d, J=8.0Hz), 6.77(1H, d, J=8.0Hz), 6.91-7.48(17H, m), 7.82 (1H, d, J=7.4Hz), 8.30(3H, bs), 8.61(1H, t, J=5.4Hz), 9.77(1H, bs), 9.20(1H, s).

Example 29

N-(2-Fluorobenzyl)-1-(4-aminomethylphenyl)-5-(4-bromobenzyl)-2,4-dioxo-2,3,4,5-tetrahydro-lH-1,5-benzodiazepine-3-acetamide hydrochloride

(1) tert-Butyl [4-[2-(4-bromobenzylamino)phenylamino]benzyl]carbamate

Acetic acid (3.66 ml, 64 mmol) was added to a methanol solution (250 ml) of tert-butyl [4-(2-aminophenylamino)benzyl]carbamate (10.0 g, 32.0 mmol) and 4-bromobenzaldehyde (5.92 ml, 32 mmol). The resulting mixture was stirred at 0°C for 30 minutes and then sodium cyanoborohydride (2.44 g, 40 mmol) was added thereto, followed by stirring at 60°C for 1 hour. The reaction solution was poured into water and extracted with ethyl acetate. After washing with water, the extract solution was dried over anhydrous magnesium sulfate and concentrated under reduced pressure. The residue was purified by a silica gel column chromatography to give tert-butyl [4-[2-(4-bromobenzylamino)phenylamino]benzyl]carbamate (15 g, 97%) as crystals.
$^1$H-NMR($CDCl_3$)δ: 1.45(9H, s), 4.21(2H, d, J=5.6Hz), 4.30(2H, s), 4.75(2H, bs), 5.12(1H, bs), 6.59-7.26(10H, m), 7.42 (2H, d, J=8.4Hz).

(2) Ethyl N-(4-bromobenzyl)-N-[2-[4-(tert-butoxycarbonylaminomethyl)phenylamino]phenyl]malonamate

To an ice-cooled stirred tetrahydrofuran (250 ml) solution of tert-butyl [3-[2-(4-bromobenzylamino)phenylamino]benzyl]carbamate was added a tetrahydrofuran (10 ml) solution of triethylamine (4.77 ml, 34.2 mmol) and ethyl malonyl chloride (4.38 ml, 34.2 mmol). The resulting mixture was stirred at 0°C for 1 hour and then a tetrahydrofuran (3 ml) solution of triethylamine (2.17 ml, 15.6 mmol) and ethyl malonyl chloride (2.0 ml, 15.6 mmol) was further added thereto. After stirring at 0°C for 10 minutes, the reaction mixture was poured into water and extracted with ethyl acetate. The extract solution was washed with water and then dried over anhydrous magnesium sulfate. After concentration under reduced pressure, the residue was purified by a silica gel column chromatography to give ethyl N-(4-bromobenzyl)-N-[2-(4-tert-butoxycarbonylaminomethyl)phenylamino]phenylmalonamate (14.9 g, 81%) as an oil.
$^1$H-NMR($CDCl_3$)δ: 1.18(3H, t, J=7.0Hz), 1.47(9H, s), 3.23(1H, d, J=16.2Hz), 3.34(1H, d, J=16.2Hz), 4.08(2H, q, J=7.0Hz). 4.26(2H, d, J=5.6Hz), 4.71(1H, d, J=13.8Hz), 4.82(1H, bs), 4.91(1H, d, J=13.8Hz), 5.83(1H, s), 6.75-7.27 (10H, m), 7.37(2H, d, J=6.2Hz).

(3) N-(4-Bromobenzyl)-N-[2-(4-tert-butoxycarbonylaminomethyl)phenylamino]phenylmalonamic acid

To an ice-cooled stirred tetrahydrofuran (52 ml) and methanol (154 ml) solution of ethyl N-(4-bromobenzyl)-N-[2-(4-tert-butoxycarbonylaminomethyl)phenylamino]phenylmalonamate (14.3 g, 24.0 mmol) was added 1N sodium hydroxide aqueous solution (48 ml, 48 mmol). The resulting mixture was stirred at 0°C for 10 minutes and at room temperature for 3 hours. Water was added to the reaction solution and the mixture was washed with diisopropyl ether. Potassium hydrogen sulfate (6.54 g, 48 mmol) was added thereto and then the mixture was extracted with ethyl acetate. After washing with water, the extract solution was dried over anhydrous magnesium sulfate. The solution was concentrated under reduced pressure to give N-(4-bromobenzyl)-N-[2-(3-tert-butoxycarbonylaminomethyl)phenylamino]phenylmalonamic acid (11.0 g, 81%) as an oil.
$^1$H-NMR($CDCl_3$)δ: 1.46(9H, s), 3.24(2H, s), 4.23(2H, d, J=5.8Hz), 4.63(1H, d, J=14.4Hz), 4.86(1H, bs), 5.03(1H, d, J=14.4Hz), 5.25(1H, bs), 6.72(2H, d, J=8.4Hz), 6.86-7.26(8H, m), 7.42(2H, d, J=8.4Hz).

(4) 5-(4-Bromobenzyl)-1-(4-tert-butoxycarbonylaminomethylphenyl)-2,4-dioxo-2,3,4,5-tetrahydro-1H-1,5-benzodiazepine

To an ice-cooled stirred N,N-dimethylformamide (500 ml) solution of N-(4-bromobenzyl)-N-[2-(3-tert-butoxycarbonylaminomethyl)phenylamino]phenylmalonamic acid (10.8 g, 19 mmol) were added 4-dimethylaminopyridine (2.32 g, 19 mmol) and 1-ethyl-3-(3-dimethylaminopropyl)carbodiimide hydrochloride (10.9 g, 57 mmol). The resulting mixture was stirred at room temperature for 18 hours. The reaction solution was poured into water and extracted with ethyl acetate. The extract solution was washed with water, a saturated sodium hydrogen carbonate aqueous solution, and water, successively, and then dried over anhydrous magnesium sulfate. After concentration under reduced pressure, the residue was purified by a silica gel column chromatography to give 5-(4-bromobenzyl)-1-(3-tert-butoxycarbonylaminomethylphenyl)-2,4-dioxo-2,3,4,5-tetrahydro-1H-1,5-benzodiazepine (6.11 g, 58%) as an oil.
$^1$H-NMR($CDCl_3$)δ: 1.47(9H, s), 3.55(2H, s), 4.30(2H, d, J=5.8Hz), 4.64(1H, d, J=14.8Hz), 4.87(1H, bs), 5.79(1H, d, J=14.8Hz), 6.63(2H, d, J=8.4Hz), 6.84(1H, d, J=8.2Hz), 7.04-7.40(8H, m), 7.47(1H, d, J=8.2Hz).

(5) Methyl 5-(4-bromobenzyl)-1-(4-tert-butoxycarbonylaminomethylphenyl)-2,4-dioxo-2,3,4,5-tetrahydro-1H-1,5-benzodiazepine-3-acetate

To a stirred N,N-dimethylformamide (100 ml) solution of 5-(4-bromobenzyl)-1-(4-tert-butoxycarbonylaminomethylphenyl)-2,4-dioxo-2,3,4,5-tetrahydro-1H-1,5-benzodiazepine (6.0 g, 10.9 mmol) was added 60% oily sodium hydride (0.959 g, 24.0 mmol). After stirring at room temperature for 30 minutes, methyl bromoacetate (1.24 ml, 13.1 mmol) was added thereto. The resulting mixture was stirred at room temperature for 10 minutes. Thereafter, the reaction solution was poured into ice-water and extracted with ethyl acetate. After washing with water, the extract solution was dried over anhydrous magnesium sulfate. After concentration under reduced pressure, the residue was purified by a silica gel column chromatography to give methyl 5-(4-bromobenzyl)-1-(4-tert-butoxycarbonylaminomethylphenyl)-2,4-dioxo-2,3,4,5-tetrahydro-1H-1,5-benzodiazepine-3-acetate (3.5 g, 52%) as solid.
$^1$H-NMR($CDCl_3$)δ: 1.46(9H, s), 3.13-3.17(2H, m), 3.70(3H, s), 3.93(1H, t, J=6.8Hz), 4.30(2H, d, J=6.2Hz), 4.69(1H, d, J=15.0Hz), 4.86(1H, bs), 5.74(1H, d, J=15.0Hz). 6.59(2H, d, J=8.2Hz), 6.87(1H, d, J=8.2Hz), 7.02-7.39(8H, m), 7.50 (1H, d, J=7.0Hz).

(6) 5-(4-Bromobenzyl)-1-(4-tert-butoxycarbonylaminomethylphenyl)-2,4-dioxo-2,3,4,5-tetrahydro-1H-1,5-benzodiazepine-3-acetic acid

To a stirred tetrahydrofuran (100 ml) and methanol (100 ml) solution of methyl 5-(4-bromobenzyl)-1-(4-tert-butoxycarbonylaminomethylphenyl)-2,4-dioxo-2,3,4,5-tetrahydro-1H-1,5-benzodiazepine-3-acetate (3.40 g, 5.46 mmol) was added 1N sodium hydroxide aqueous solution (25 ml, 25 mmol). The resulting mixture was stirred at 60°C for 1 hour. After cooling of the reaction solution, water and potassium hydrogen sulfate (3.40 g, 25 mmol) were added thereto. The mixture was extracted with ethyl acetate and, after washing with water, the extract solution was dried over anhydrous magnesium sulfate. The solution was concentrated under reduced pressure to give 5-(4-bromobenzyl)-1-(4-tert-butoxycarbonylaminomethylphenyl)-2,4-dioxo-2,3,4,5-tetrahydro-1H-1,5-benzodiazepine-3-acetic acid (3.3 g, 98%) as amorphous solid.

$^1H$-NMR($CDCl_3$)δ: 1.46(9H, s), 3.16(2H, t, J=7.6Hz), 3.92(1H, t, J=6.8Hz), 4.29(2H, d, J=5.4Hz), 4.73(1H, d, J=15.0Hz), 5.01(1H, t, J=5.4Hz), 5.68(1H, d, J=15.0Hz). 6.62(2H, d, J=8.6Hz), 6.85(1H, d, J=8.0Hz), 7.03-7.29(6H, m), 7.36(2H, d, J=8.6Hz), 7.47(1H, d, J=8.0Hz).

(7) N-(2-Fluorobenzyl)-5-(4-bromobenzyl)-1-(4-tert-butoxycarbonylaminomethylphenyl)-2,4-dioxo-2,3,4,5-tetrahydro-1H-1,5-benzodiazepine-3-acetamide

To an ice-cooled stirred N,N-dimethylformamide (25 ml) solution of 5-(4-bromobenzyl)-1-(4-tert-butoxycarbonylaminomethylphenyl)-2,4-dioxo-2,3,4,5-tetrahydro-1H-1,5-benzodiazepine-3-acetic acid (2.5 g, 4.1 mmol) were added 2-fluorobenzylamine (0.52 ml, 4.5 mmol), diethyl cyanophosphate (0.74 ml, 4.9 mmol) and triethylamine (0.69 ml, 4.9 mmol). The resulting mixture was stirred at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-fluorobenzyl)-5-(4-bromobenzyl)-1-(4-tert-butoxycarbonylaminomethylphenyl)-2,4-dioxo-2,3,4,5-tetrahydro-1H-1,5-benzodiazepine-3-acetamide (1.9 mg, 65%) as amorphous solid.
$^1H$-NMR($CDCl_3$)δ: 1.46(9H, s), 3.00(2H, d, J=6.6Hz), 4.04(1H, t, J=6.6Hz), 4.29(2H, d, J=6.0Hz), 4.39-4.59(2H, m), 4.68(1H, d, J=15.0Hz). 4.85(1H, bs), 5.71(1H, d, J=15.0Hz), 6.31(1H, bs), 6.54(2H, d, J=8.4Hz), 6.82(1H, d, J=8.0Hz), 7.00-7.37(12H, m), 7.46(1H, d, J=8.4Hz).

(8) N-(2-Fluorobenzyl)-1-(4-aminomethylphenyl)-5-(4-bromobenzyl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-5-(4-bromobenzyl)-1-(4-tert-butoxycarbonylaminomethylphenyl)-2,4-dioxo-2,3,4,5-tetrahydro-1H-1,5-benzodiazepine-3-acetamide (200 mg, 0.28 mmol). The resulting mixture was stirred at room temperature for 1 hour and then concentrated under reduced pressure. The residue was crystallized from ethyl ether to give N-(2-fluorobenzyl)-1-(4-aminomethylphenyl)-5-(4-bromobenzyl)-2,4-dioxo-2,3,4,5-tetrahydro-lH-1,5-benzodiazepine-3-acetamide hydrochloride (180 mg, 97%) as crystals.

Melting point 176-178°C.
Elemental analysis for $C_{32}H_{29}N_4O_3BrClF \cdot 1.5H_2O$;
Calcd.: C, 57.37; H, 4.66; N, 8.36.
Found: C, 57.43; H, 4.60; N, 8.10.
$^1H$-NMR(DMSO-$d_6$)δ: 2.74-2.97(2H, m), 3.91(1H, t, J=4.8Hz), 4.04(2H, d, J=6.2Hz), 4.29(2H, d, J=5.4Hz), 4.97 (1H, d, J=15.6Hz), 5.56(1H, d, J=15.6Hz), 6.72-7.49(15H, m), 7.77(1H, d, J=7.4Hz), 8.23(3H, bs), 8.61(1H, t, J=5.4Hz).

Example 30

N-(2-Fluorobenzyl)-1-(4-aminomethylphenyl)-5-[4-(3-thienyl)benzyl]-2,4-dioxo-2,3,4,5-tetrahydro-1H-1,5-benzodiazepine-3-acetamide hydrochloride

(1) A mixture of N-(2-fluorobenzyl)-5-(4-bromobenzyl)-1-(4-tert-butoxycarbonylaminomethylphenyl)-2,4-dioxo-2,3,4,5-tetrahydro-lH-1,5-benzodiazepine-3-acetamide (0.36 g, 0.5 mmol), 3-thiopheneboronic acid (0.077 g, 0.6 mmol), sodium carbonate (0.132 g, 1.25 mmol), toluene (25 ml), ethanol (5 ml) and water (5 ml) was stirred at room temperature for 30 minutes under argon atmosphere. Tetrakis(triphenylphosphine)palladium(0) (34.7 mg, 0.03 mmol) was added thereto and then the mixture was heated to reflux for 15 hours. After cooling, the reaction solution was poured into water and extracted with ethyl acetate. The extract solution was washed with water and brine, and then dried over anhydrous magnesium sulfate, followed by concentration under reduced pressure. The residue was solidified from ethyl acetate to give N-(2-fluorobenzyl)-1-(4-tert-butoxycarbonylaminomethylphenyl)-5-[4-(3-thienyl)benzyl]-2,4-dioxo-2,3,5-tetrahydro-1H-1,5-benzodiazepine-3-acetamide (0.17 g, 47%) as solid.
$^1H$-NMR($CDCl_3$)δ: 1.45(9H, s), 3.02(2H, d, J=7.0Hz), 4.05(1H, t, J=7.0Hz), 4.21(2H, d, J=5.8Hz), 4.38-4.58(2H, m), 4.73(1H, d, J=15.0Hz), 4.77(1H, bs), 5.80(1H, d, J=15.0Hz), 6.31(1H, bs), 6.55(2H, d, J=8.4Hz), 6.81(1H, d, J=8.4Hz), 6.99-7.62(16H, m).

(2) N-(2-Fluorobenzyl)-1-(4-aminomethylphenyl)-5-[4-(3-thienyl)benzyl]-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-1-(4-tert-butoxycarbonylaminomethylphenyl)-5-[4-(3-thienyl)benzyl]-2,4-dioxo-2,3,5-tetrahydro-1H-1,5-benzodiazepine-3-acetamide (168 mg, 0.23 mmol). The resulting mixture was stirred at room temperature for 1 hour and then concentrated under reduced pressure. The residue was crystallized from ethyl ether to give N-(2-fluorobenzyl)-1-(4-aminomethylphenyl)-5-[4-(3-thienyl)benzyl]-2,4-dioxo-2,3,4,5-tetrahydro-1H-1,5-benzodiazepine-3-acetamide hydrochloride (142 mg, 93%) as crystals.

Melting point 188-189°C.
Elemental analysis for $C_{36}H_{32}N_4O_3ClFS \cdot 1.8H_2O$;
Calcd.: C, 62.88; H, 5.22; N, 8.15.
Found: C, 62.94; H, 5.12; N, 8.06.
$^1$H-NMR(DMSO-$d_6$)δ: 2.81-3.03(2H, m), 3.91-4.05(3H, m), 4.31(2H, d, J=5.8Hz), 4.99(1H, d, J=15.2Hz), 5.63(1H, d, J=15.2Hz), 6.77-7.83(19H, m), 8.28(3H, bs), 8.61(1H, t, J=6.2Hz).

Example 31

N-(2-Fluorobenzyl)-1-(4-aminomethylphenyl)-5-[4-(2-thienyl)benzyl]-2,4-dioxo-2,3,4,5-tetrahydro-1H-1,5-benzodiazepine-3-acetamide hydrochloride

(1) A mixture of N-(2-fluorobenzyl)-5-(4-bromobenzyl)-1-(4-tert-butoxycarbonylaminomethylphenyl)-2,4-dioxo-2,3,4,5-tetrahydro-1H-1,5-benzodiazepine-3-acetamide (0.36 g, 0.5 mmol), 2-thiopheneboronic acid (0.077 g, 0.6 mmol), sodium carbonate (0.132 g, 1.25 mmol), toluene (25 ml), ethanol (5 ml) and water (5 ml) was stirred at room temperature for 30 minutes under argon atmosphere. Tetrakis(triphenylphosphine)palladium(0) (34.7 mg, 0.03 mmol) was added thereto and then the mixture was heated to reflux for 15 hours. After cooling, the reaction solution was poured into water and extracted with ethyl acetate. The extract solution was washed with water and brine, and then dried over anhydrous magnesium sulfate, followed by concentration under reduced pressure. The residue was solidified from ethyl acetate to give N-(2-fluorobenzyl)-1-(4-tert-butoxycarbonylaminomethylphenyl)-5-[4-(2-thienyl)benzyl]-2,4-dioxo-2,3,5-tetrahydro-1H-1,5-benzodiazepine-3-acetamide (0.14 g, 39%) as solid.
$^1$H-NMR($CDCl_3$)δ: 1.45(9H, s), 3.02(2H, d, J=7.0Hz), 4.06(1H, t, J=7.0Hz), 4.21(2H, d, J=6.0Hz), 4.41-4.59(2H, m), 4.72(1H, d, J=14.6Hz), 4.75(1H, bs), 5.80(1H, d, J=14.6Hz), 6.29(1H, bs), 6.57(2H, d, J=8.4Hz), 6.82(1H, d, J=7.2Hz), 6.99-7.54(16H, m).

(2) N-(2-Fluorobenzyl)-1-(4-aminomethylphenyl)-5-[4-(2-thienyl)benzyl]-2,4-dioxo-2,3,4,5-tetrahydro-lH-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-1-(4-tert-butoxycarbonylaminomethylphenyl)-5-[4-(2-thienyl)benzyl]-2,4-dioxo-2,3,5-tetrahydro-1H-1,5-benzodiazepine-3-acetamide (0.14 g, 0.2 mmol). The resulting mixture was stirred at room temperature for 1 hour and then concentrated under reduced pressure. The residue was crystallized from ethyl ether to give N-(2-fluorobenzyl)-1-(4-aminomethylphenyl)-5-[4-(2-thienyl)benzyl]-2,4-dioxo-2,3,4,5-tetrahydro-1H-1,5-benzodiazepine-3-acetamide hydrochloride (113 mg, 88%) as crystals.

Melting point 185-187°C.
Elemental analysis for $C_{36}H_{32}N_4O_3ClFS \cdot 1.5H_2O$;
Calcd.: C, 63.38; H, 5.17; N, 8.21.
Found: C, 63.64; H, 5.17; N, 8.23.
$^1$H-NMR(DMSO-$d_6$)δ: 2.82-2.94(2H, m), 3.90-4.05(3H, m), 4.30(2H, d, J=6.4Hz), 5.00(1H, d, J=15.6Hz), 5.60(1H, d, J=15.6Hz), 6.75-7.57(18H, m), 7.80(1H, d, J=8.0Hz), 8.19(3H, bs), 8.61(1H, t, J=5.4Hz).

Example 32

1-(4-Aminomethylphenyl)-5-(4-biphenylmethyl)-3-(2-fluorobenzyl)-2,4-dioxo-2,3,4,5-tetrahydro-1H-1,5-benzodiazepine hydrochloride

(1) To a stirred N,N-dimethylformamide (3 ml) solution of 5-(4-biphenylmethyl)-1-(4-tert-butoxycarbonylaminomethylphenyl)-2,4-dioxo-2,3,4,5-tetrahydro-1H-1,5-benzodiazepine (310 mg, 0.57 mmol) was added 60% oily sodium hydride (50 mg, 1.25 mmol). After stirring at room temperature for 20 minutes, 2-fluorobenzyl chloride (0.081 ml, 0.68 mmol) was added thereto. The resulting mixture was stirred at room temperature for 10 minutes. Thereafter, the reaction solution was poured into ice-water and extracted with ethyl acetate. After washing with water, the extract solution was dried over anhydrous magnesium sulfate. After concentration under reduced pressure, the residue was purified by a silica gel column chromatography to give 5-(4-biphenylmethyl)-1-(4-tert-butoxycarbonylaminomethylphenyl)-3-(2-fluorobenzyl)-2,4-dioxo-2,3,4,5-tetrahydro-lH-1,5-benzodiazepine (73 mg, 20%) as solid.
$^{1}$H-NMR($CDCl_3$)δ: 1.44(9H, s), 3.43-3.53(2H, m), 3.72(1H, t, J=7.0Hz), 4.20(2H, d, J=5.8Hz), 4.71(1H, d, J=14.6Hz), 4.75(1H, bs), 5.85(1H, d, J=14.6Hz), 6.54(2H, d, J=8.0Hz). 6.80(1H, d, J=8.4Hz), 6.89 - 7.55(18H, m).

(2) 1-(4-Aminomethylphenyl)-5-(4-biphenylmethyl)-3-(2-fluorobenzyl)-2,4-dioxo-2,3,4,5-tetrahydro-1H-1,5-benzodiazepine hydrochloride

A 4N ethyl acetate solution (1 ml) of hydrogen chloride was added to a stirred ethyl acetate (1 ml) solution of 5-(4-biphenylmethyl)-1-(4-tert-butoxycarbonylaminomethylphenyl)-3-(2-fluorobenzyl)-2,4-dioxo-2,3,4,5-tetrahydro-1H-1,5-benzodiazepine *(73 mg,* 0.11 mmol). The resulting mixture was stirred at room temperature for 1 hour and then concentrated under reduced pressure. The residue was crystallized from ethyl ether to give 1-(4-aminomethylphenyl)-5-(4-biphenylmethyl)-3-(2-fluorobenzyl)-2,4-dioxo-2,3,4,5-tetrahydro-1H-1,5-benzodiazepine hydrochloride (61 mg, 93%) as crystals.

Melting point 172-174°C.
Elemental analysis for $C_{36}H_{31}N_3O_2ClF \cdot 1/2H_2O$;
Calcd.: C, 71.93; H, 5.37; N, 6.99.
Found: C, 71.55; H, 5.53; N, 6.83.
$^{1}$H-NMR(DMSO-$d_6$)δ: 3.14-3.36(2H, m), 3.86(1H, t, J=7.6Hz), 3.96(2H, s), 4.98(1H, d, J=15.0Hz), 5.65(1H, d, J=15.0Hz), 6.74-7.64(20H, m), 7.82(1H, d, J=8.0Hz), 8.32(3H, bs).

Example 33

1-(4-Aminomethylphenyl)-5-(4-biphenylmethyl)-3-(3,4-dichlorobenzyl)-2,4-dioxo-2,3,4,5-tetrahydro-1H-1,5-benzodiazepine hydrochloride

(1) To a stirred N,N-dimethylformamide (3 ml) solution of 5-(4-biphenylmethyl)-1-(4-tert-butoxycarbonylaminomethylphenyl)-2,4-dioxo-2,3,4,5-tetrahydro-1H-1,5-benzodiazepine (310 mg, 0.57 mmol) was added 60% oily sodium hydride (50 mg, 1.25 mmol). After stirring at room temperature for 20 minutes, 3,4-dichlorobenzyl chloride (0.094 ml, 0.68 mmol) was added thereto. The resulting mixture was stirred at room temperature for 10 minutes. Thereafter, the reaction solution was poured into ice-water and extracted with ethyl acetate. After washing with water, the extract solution was dried over anhydrous magnesium sulfate. After concentration under reduced pressure, the residue was purified by a silica gel column chromatography to give 5-(4-biphenylmethyl)-1-(4-tert-butoxycarbonylaminomethylphenyl)-3-(3,4-dichlorobenzyl)-2,4-dioxo-2,3,4,5-tetrahydro-1H-1,5-benzodiazepine (220 mg, 55%) as solid.
$^{1}$H-NMR($CDCl_3$)δ: 1.44(9H, s), 3.34-3.56(3H, m). 4.21(2H, d, J=6.0Hz). 4.74(1H, d, J=14.8Hz), 4.78(1H, bs), 5.83 (1H, d, J=14.8Hz), 6.54(2H, d, J=8.4Hz), 6.82(1H, d, J=8.0Hz), 7.05-7.56(17H, m).

(2) 1-(4-Aminomethylphenyl)-5-(4-biphenylmethyl)-3-(3,4-dichlorobenzyl)-2,4-dioxo-2,3,4,5-tetrahydro-1H-1,5-benzodiazepine hydrochloride

A 4N ethyl acetate solution (1 ml) of hydrogen chloride was added to a stirred ethyl acetate (1 ml) solution of 5-(4-biphenylmethyl)-1-(4-tert-butoxycarbonylaminomethylphenyl)-3-(3,4-dichlorobenzyl)-2,4-dioxo-2,3,4,5-tetrahydro-lH-1,5-benzodiazepine (180 mg, 0.25 mmol). The resulting mixture was stirred at room temperature for 1 hour and then concentrated under reduced pressure. The residue was crystallized from ethyl ether to give 1-(4-aminomethylphenyl)-5-(4-biphenylmethyl)-3-(3,4-dichlorobenzyl)-2,4-dioxo-2,3,4,5-tetrahydro-1H-1,5-benzodiazepine hydrochloride (162 mg, 98%) as crystals.

Melting point 175-176°C.
Elemental analysis for $C_{36}H_{30}N_3O_2Cl_3 \cdot 0.6H_2O$;
Calcd.: C, 66.13; H, 4.81; N, 6.43.
Found: C, 66.00; H, 5.04; N, 6.35.
$^1$H-NMR(DMSO-$d_6$)δ: 3.17-3.35(2H, m), 3.85(1H, t, J=6.4Hz), 3.97(2H, s), 5.00(1H, d, J=15.4Hz), 5.62(1H, d, J=15.4Hz), 6.75-7.64(19H, m), 7.77(1H, d, J=8.8Hz), 8.28(3H, bs).

Example 34

N-(2-Fluorobenzyl)-1-(4-aminomethylphenyl)-5-(benzyl)-2,4-dioxo-2,3,4,5-tetrahydro-1H-1,5-benzodiazepine-3-acetamide hydrochloride

Starting with tert-butyl [4-(2-aminophenylamino)benzyl]carbamate given in (3) of Example 1 and benzaldehyde, N-(2-fluorobenzyl)-1-(4-aminomethylphenyl)-5-(benzyl)-2,4-dioxo-2,3,4,5-tetrahydro-1H-1,5-benzodiazepine-3-acetamide hydrochloride was given as crystals in a similar manner to Example 11.

Melting point 176-178°C.
Elemental analysis for $C_{32}H_{30}N_4O_3ClF \cdot 1.5H_2O$;
Calcd.: C, 64.05; H, 5.54; N, 9.34.
Found: C, 63.98; H, 5.55; N, 9.16.
$^1$H-NMR(DMSO-$d_6$)δ: 2.78-3.02(2H, m), 3.93(1H, t, J=7.0Hz), 4.05(2H, d, J=7.0Hz), 4.31(2H, d, J=5.6Hz), 4.97 (1H, d, J=15.0Hz), 5.67(1H, d, J=15.0Hz), 6.66(2H, d, J=8.4Hz), 6.89(1H, dd, J=1.4,8.2Hz), 7.13-7.45(13H, m), 7.84(1H, dd, J=1.4,8.2Hz), 8.34(3H, bs), 8.63(1H, t, J=6.0Hz).

Example 35

N-(2-Fluorobenzyl)-1-(4-aminomethylphenyl)-5-(4-benzyloxybenzyl)-2,4-dioxo-2,3,4,5-tetrahydro-1H-1,5-benzodiazepine-3-acetamide hydrochloride

(1) N-(2-Fluorobenzyl)-5-(4-benzyloxybenzyl)-1-(4-tert-butoxycarbonylaminomethylphenyl)-2,4-dioxo-2,3,4,5-tetrahydro-1H-1,5-benzodiazepine-3-acetamide

Starting with tert-butyl [4-(2-aminophenylamino)benzyl]carbamate given in (3) of Example 1 and 4-benzyloxybenzaldehyde, N-(2-fluorobenzyl)-5-(4-benzyloxybenzyl)-1-(4-tert-butoxycarbonylaminomethylphenyl)-2,4-dioxo-2,3,4,5-tetrahydro-1H-1,5-benzodiazepine-3-acetamide was given as amorphous solid in similar manners to (1) and (2) of Example 11.

$^1$H-NMR($CDCl_3$)δ: 1.45(9H, s), 3.01(2H, d, J=6.8Hz), 3.99(1H, t, J=6.8Hz), 4.24(2H, d, J=5.6Hz), 4.48(2H, t, J=5.8Hz), 4.67(1H, d, J=15.0Hz), 4.79(1H, bs), 5.00(2H, s), 5.71(1H, d, J=15.0Hz), 6.34(1H, t, J=5.8Hz), 6.61(2H, d, J=8.4Hz), 6.79-7.41(18H, m), 7.48(1H, d, J=8.0Hz).

(2) A 4N ethyl acetate solution (1 ml) of hydrogen chloride was added to a stirred ethyl acetate (1 ml) solution of N-(2-fluorobenzyl)-5-(4-benzyloxybenzyl)-1-(4-tert-butoxycarbonylaminomethylphenyl)-2,4-dioxo-2,3,4,5-tetrahydro-lH-1,5-benzodiazepine-3-acetamide (50 mg, 0.067 mmol). The resulting mixture was stirred at room temperature for 1 hour and then concentrated under reduced pressure. The residue was crystallized from ethyl ether to give N-(2-fluorobenzyl)-1-(4-aminomethylphenyl)-5-(4-benzyloxybenzyl)-2,4-dioxo-2,3,4,5-tetrahydro-1H-1,5-benzodiazepine-3-acetamide hydrochloride (41 mg, 90%) as crystals.

Melting point 155-157°C.
Elemental analysis for $C_{39}H_{36}N_4O_4ClF \cdot H_2O$;
Calcd.: C, 67.19; H, 5.49; N, 8.09.

Found: C, 67.46; H, 5.25; N, 7.96.
$^1$H-NMR(DMSO-$d_6$)δ: 2.76-3.02(2H, m), 3.91(1H, t, J=7.4Hz), 4.02(2H, d, J=5.6Hz), 4.30(2H, d, J=5.6Hz), 4.89(1H, d, J=15.0Hz), 5.08(2H, s), 5.59(1H, d, J=15.0Hz), 6.72-7.55(20H, m), 7.82(1H, d, J=8.4Hz), 8.28(3H, bs), 8.62(1H, t, J=5.2Hz).

Example 36

N-(2-Fluorobenzyl)-1-(4-aminomethylphenyl)-5-(4-hydroxybenzyl)-2,4-dioxo-2,3,4,5-tetrahydro-1H-1,5-benzodiazepine-3-acetamide hydrochloride

(1) N-(2-Fluorobenzyl)-1-(4-tert-butoxycarbonylaminomethylphenyl)-5-(4-hydroxybenzyl)-2,4-dioxo-2,3,4,5-tetrahydro-lH-1,5-benzodiazepine-3-acetamide

Palladium supported on carbon (5%, 60 mg) was added to an ethyl acetate (2 ml) and methanol (2 ml) solution of N-(2-fluorobenzyl)-5-(4-benzyloxybenzyl)-1-(4-tert-butoxycarbonylaminomethylphenyl)-2,4-dioxo-2,3,4,5-tetrahydro-1H-1,5-benzodiazepine-3-acetamide (145 mg, 0.196 mmol) given in (1) of Example 35. The resulting mixture was subjected to hydrogenation for 4 hours under the conditions of ambient temperature and normal pressure. After the completion of the reaction, the catalyst was removed by filtration and the filtrate was concentrated under reduced pressure to give N-(2-fluorobenzyl)-1-(4-tert-butoxycarbonylaminomethylphenyl)-5-(4-hydroxylbenzyl)-2,4-dioxo-2,3,4,5-tetrahydro-lH-1,5-benzodiazepine-3-acetamide (108 mg, 85%) as crystals.
$^1$H-NMR($CDCl_3$)δ: 1.44(9H, s), 2.90(1H, dd, J=7.4, 15.4Hz), 3.15(1H, dd, J=7.4, 15.4Hz), 4.05(1H, t, J=7.4Hz), 4.24 (2H, d, J=5.4Hz), 4.38-4.51(4H, m), 5.49(1H, bs), 5.78(1H, d, J=15.2Hz), 6.11(1H, bs), 6.23-6.33(2H, m), 6.71-7.51 (13H, m), 7.53(1H, d, J=8.8Hz).

(2) N-(2-Fluorobenzyl)-1-(4-aminomethylphenyl)-5-(4-hydroxybenzyl)-2,4-dioxo-2,3,4,5-tetrahydro-lH-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-1-(4-tert-butoxycarbonylaminomethylphenyl)-5-(4-hydroxylbenzyl)-2,4-dioxo-2,3,4,5-tetrahydro-lH-1,5-benzodiazepine-3-acetamide (100 mg, 0.15 mmol). The resulting mixture was stirred at room temperature for 1 hour and then concentrated under reduced pressure. The residue was crystallized from ethyl ether to give N-(2-fluorobenzyl)-1-(4-aminomethylphenyl)-5-(4-hydroxybenzyl)-2,4-dioxo-2,3,4,5-tetrahydro-1H-1,5-benzodiazepine-3-acetamide hydrochloride (81 mg, 90%) as crystals.

Melting point 196-199°C.
Elemental analysis for $C_{32}H_{30}N_4O_4ClF \cdot 2H_2O$;
Calcd.: C, 61.49; H, 5.48; N, 8.96.
Found: C, 61.12; H, 5.12; N, 8.55.
$^1$H-NMR(DMSO-$d_6$)δ: 2.52-2.69(2H, m), 3.66(1H, t, J=7.0Hz), 3.69-3.80(2H, m), 4.07(2H, d, J=5.8Hz), 4.57(1H, d, J=14.6Hz), 5.34(1H, d, J=14.6Hz), 6.40-6.57(5H, m), 6.70(2H, d, J=8.4Hz), 6.90-7.22(8H, m), 7.60(1H, d, J=8.0Hz), 8.09(3H, bs), 8.39(1H, t, J=5.8Hz), 9.20(1H, bs).

Example 37

N-(2-Fluorobenzyl)-5-(4-acetamidobenzyl)-1-(4-aminomethylphenyl)-2,4-dioxo-2,3,4,5-tetrahydro-1H-1,5-benzodiazepine-3-acetamide hydrochloride

Starting with tert-butyl [4-(2-aminophenylamino)benzyl]carbamate given in (3) of Example 1 and 4-acetamidobenzaldehyde, N-(2-fluorobenzyl)-5-(4-acetamidobenzyl)-1-(4-aminomethylphenyl)-2,4-dioxo-2,3,4,5-tetrahydro-lH-1,5-benzodiazepine-3-acetamide hydrochloride was given as crystals in a similar manner to Example 11.

Melting point 206-208°C.
Elemental analysis for $C_{34}H_{33}N_5O_4ClF \cdot 1.2H_2O$:
Calcd.: C, 62.66; H, 5.47; N, 10.74.
Found: C, 62.85; H, 5.69; N, 10.44.
$^1$H-NMR(DMSO-$d_6$)δ: 2.05(3H, s), 2.68-3.00(2H, m), 3.90(1H, t, J=7.2Hz), 4.01(2H, d, J=5.4Hz), 4.29(2H, d, J=5.0Hz), 4.89(1H, d, J=15.0Hz), 5.56(2H, d, J=15.0Hz), 6.72-7.48(15H, m), 7.78(1H, d, J=8.0Hz), 8.35(3H, bs), 8.62(1H, t, J=5.0Hz), 10.07(1H, bs).

Example 38

N-(2-Fluorobenzyl)-1-(4-aminomethylphenyl)-5-(4-chlorobenzyl)-2,4-dioxo-2,3,4,5-tetrahydro-1H-1,5-benzodiazepine-3-acetamide hydrochloride

Starting with tert-butyl [4-(2-aminophenylamino)benzyl]carbamate given in (3) of Example 1 and 4-chlorobenzaldehyde, N-(2-fluorobenzyl)-1-(4-aminomethylphenyl)-5-(4-chlorobenzyl)-2,4-dioxo-2,3,4,5-tetrahydro-1H-1,5-benzodiazepine-3-acetamide hydrochloride was given as crystals in a similar manner to Example 11.

Melting point 177-179°C.
Elemental analysis for $C_{32}H_{29}N_4O_3Cl_2F \cdot 0.9H_2O$;
Calcd.: C, 61.62; H, 4.98; N, 8.98.
Found: C, 61.80: H, 4.97; N, 8.85.
$^1$H-NMR(DMSO-$d_6$)δ: 2.81-3.02(2H, m), 3.94(1H, t, J=6.8Hz), 4.05(2H, d, J=6.6Hz), 4.31(2H, d, J=5.2Hz), 5.00 (1H, d, J=15.6Hz), 5.60(2H, d, J=15.6Hz), 6.73-7.50(15H, m), 7.80(1H, d, J=8.4Hz), 8.33(3H, bs), 8.64(1H, t, J=5.2Hz).

Example 39

N-(2-Fluorobenzyl)-1-(4-aminomethylphenyl)-5-(2,2-dimethylpropyl)-2,4-dioxo-2,3,4,5-tetrahydro-1H-1,5-benzodiazepine-3-acetamide hydrochloride

Starting with tert-butyl [4-(2-aminophenylamino)benzyl]carbamate given in (3) of Example 1 and pivalaldehyde, N-(2-fluorobenzyl)-1-(4-aminomethylphenyl)-5-(2,2-dimethylpropyl)-2,4-dioxo-2,3,4,5-tetrahydro-lH-1,5-benzodiazepine-3-acetamide hydrochloride was given as crystals in a similar manner to Example 11.

Melting point 187-189°C.
Elemental analysis for $C_{30}H_{34}N_4O_3ClF \cdot H_2O$;
Calcd.: C, 63.09; H, 6.35; N, 9.81.
Found: C, 63.11; H, 6.11; N, 9.71.
$^1$H-NMR(DMSO-$d_6$)δ: 0.75(9H, s), 2.82(2H, d, J=6.2Hz), 3.66(1H, d, J=13.8Hz), 3.85(1H, t, J=6.2Hz), 4.06(2H, d, J=6.6Hz), 4.27(2H, d, J=5.6Hz), 4.42(1H, d, J=13.8Hz), 6.86(1H, d, J=8.2Hz), 7.11-7.46(8H, m), 7.59(2H, d, J=8.4Hz), 7.79(1H, d, J=7.6Hz), 8.32(3H, bs), 8.58(1H, t, J=5.6Hz).

Example 40

N-(2-Fluorobenzyl)-1-(4-aminomethylphenyl)-5-(4-trifluoromethylbenzyl)-2,4-dioxo-2,3,4,5-tetrahydro-1H-1,5-benzodiazepine-3-acetamide hydrochloride

Starting with tert-butyl [4-(2-aminophenylamino)benzyl]carbamate given in (3) of Example 1 and 4-trifluoromethylbenzaldehyde, N-(2-fluorobenzyl)-1-(4-aminomethylphenyl)-5-(4-trifluoromethylbenzyl)-2,4-dioxo-2,3,4,5-tetrahydro-lH-1,5-benzodiazepine-3-acetamide hydrochloride was given as crystals in a similar manner to Example 11. $^1$H-NMR(DMSO-$d_6$)δ: 2.69-3.08(2H, m), 3.97(1H, t, J=7.0Hz), 4.04(2H, d, J=5.6Hz), 4.31(2H, d, J=5.8Hz), 5.14(1H, d, J=16.0Hz), 5.64(1H, d, J=16.0Hz), 6.76-7.43(13H, m), 7.67(2H, d, J=8.0Hz), 7.88(1H, d, J=8.2Hz), 8.35(3H, bs), 8.65(1H, t, J=5.8Hz).

Example 41

N-(2-Fluorobenzyl)-1-(4-aminomethylphenyl)-5-(2-naphthylmethyl)-2,4-dioxo-2,3,4,5-tetrahydro-1H-1,5-benzodiazepine-3-acetamide hydrochloride

Starting with tert-butyl [4-(2-aminophenylamino)benzyl]carbamate given in (3) of Example 1 and 2-naphthylaldehyde, N-(2-fluorobenzyl)-1-(4-aminomethylphenyl)-5-(2-naphthylmethyl)-2,4-dioxo-2,3,4,5-tetrahydro-1H-1,5-benzodiazepine-3-acetamide hydrochloride was given as crystals in a similar manner to Example 11.

Melting point 180-182°C.
Elemental analysis for $C_{36}H_{32}N_4O_3ClF \cdot 1.5H_2O$;
Calcd.: C, 66.51; H, 5.43; N, 8.62.
Found: C, 66.68; H, 5.28; N, 8.59.

$^1$H-NMR(DMSO-$d_6$)δ: 2.79-3.04(2H, m), 3.95-4.01(3H, m), 4.31(2H, d, J=4.8Hz), 5.17(1H, d, J=15.4Hz), 5.74(1H, d, J=15.4Hz), 6.71-7.89(19H, m), 8.31(3H, bs), 8.63(1H, t, J=4.8Hz).

Example 42

N-(2-Fluorobenzyl)-1-(4-aminomethylphenyl)-5-(4-methoxybenzyl)-2,4-dioxo-2,3,4,5-tetrahydro-1H-1,5-benzodiazepine-3-acetamide hydrochloride

Starting with tert-butyl [4-(2-aminophenylamino)benzyl]carbamate given in (3) of Example 1 and 4-methoxybenzaldehyde, N-(2-fluorobenzyl)-1-(4-aminomethylphenyl)-5-(4-methoxybenzyl)-2,4-dioxo-2,3,4,5-tetrahydro-lH-1,5-benzodiazepine-3-acetamide hydrochloride was given as crystals in a similar manner to Example 11.

Melting point 170-177°C.
Elemental analysis for $C_{33}H_{32}N_4O_4ClF \cdot H_2O$;
Calcd.: C, 63.81; H, 5.52; N, 9.02.
Found: C, 64.00; H, 5.55; N, 8.99.
$^1$H-NMR(DMSO-$d_6$)δ: 2.75-2.99(2H, m), 3.73(3H, s), 3.88(1H, t, J=6.2Hz), 3.97-4.04(2H, m), 4.28(2H, d, J=5.0Hz), 4.84(1H, d, J=14.6Hz), 5.60(2H, d, J=14.6Hz), 6.64(2H, d, J=8.0Hz). 6.74-7.42(13H, m), 7.82(1H, d, J=8.0Hz), 8.33(3H, bs), 8.60(1H, t, J=5.0Hz).

Example 43

N-(2-Fluorobenzyl)-1-[4-(1-amino-1-methylethyl)phenyl]-5-(4-biphenylmethyl)-2,4-dioxo-2,3,4,5-tetrahydro-1H-1,5-benzodiazepine-3-acetamide hydrochloride

(1) Ethyl 2-methyl-2-(4-nitrophenyl)propionate

To an N,N-dimethylformamide (250 ml) solution of ethyl 2-(4-nitrophenyl)acetate (25.3 g, 0.121 mol) was added 60% oily sodium hydride (10.6 g, 266 mmol) with stirring under ice cooling. The resulting mixture was stirred at room temperature for 20 minutes. Then, methyl iodide (16.6 ml, 266 mmol) was added to the reaction solution at 0°C and the solution was stirred at room temperature for 12 hours. Ice-water was added to the reaction solution, followed by extraction with ethyl acetate. After washing with water, the extract solution was dried over anhydrous magnesium sulfate. The solution was concentrated under reduced pressure to give ethyl 2-methyl-2-(4-nitrophenyl)propionate (28 g, 98%) as an oil.
$^1$H-NMR($CDCl_3$)δ: 1.19(3H, t, J=7.2Hz), 1.62(6H, s), 4.14(2H, q, J=7.2Hz), 7.50(2H, d, J=8.0Hz), 8.18(2H, d, J=8.0Hz).

(2) 2-Methyl-2-(4-nitrophenyl)propionic acid

To a stirred tetrahydrofuran (200 ml) and methanol (200 ml) solution of ethyl 2-methyl-2-(4-nitrophenyl)propionate (28 g, 118 mmol) was added 1N sodium hydroxide aqueous solution (118 ml, 118 mmol). The resulting mixture was stirred at 50°C for 2 hours. Conc. hydrochloric acid (30 ml) was added to the reaction solution and the mixture was extracted with ethyl acetate. After washing with water, the extract solution was dried over anhydrous magnesium sulfate. After concentration under reduced pressure, the residue was recrystallized from diethyl ether-hexane to give 2-methyl-2-(4-nitrophenyl)propionic acid (23.5 g, 95%) as crystals.

Melting point 133-134°C.
Elemental analysis for $C_{10}H_{11}NO_4$;
Calcd.: C, 57.41; H, 5.30; N, 6.70.
Found: C, 57.39; H, 5.23; N, 6.71. $^1$H-NMR($CDCl_3$)δ: 1.65(6H, s), 7.57(2H, d, J=8.8Hz), 8.20(2H, d, J=8.8Hz).

(3) 1-(4-Nitrophenyl)-1-methylethylamine

A mixture of 2-methyl-2-(4-nitrophenyl)propionic acid (23.5 g, 112 mmol), diphenyl phosphorylazide (DPPA) (24.1 ml, 112 mmol), triethylamine (15.6 ml, 112 mmol) and N,N-dimethylformamide (200 ml) was stirred at 0°C for 1.5 hours. The reaction solution was poured into ice-water and extracted with ethyl acetate. After washing with water, the extract solution was dried over anhydrous magnesium sulfate, followed by removal of the solvent by evaporation.

Toluene (200 ml) was added to the residue and the resulting mixture was stirred under heating and refluxing for 5 hours. Thereafter, 5N hydrochloric acid (125 ml) was added to the reaction solution and the mixture was heated to 100°C under refluxing for 1 hour. After cooling of the reaction solution, 5N sodium hydroxide aqueous solution (125 ml) was added thereto, followed by extraction with ethyl acetate. After washing with water and brine, the extract solution was dried over anhydrous magnesium sulfate. Then, the solvent was removed by evaporation to give 1-(4-nitrophenyl)-1-methylethylamine (20 g, 99%) as an oil.
$^1$H-NMR($CDCl_3$)δ: 1.53(6H, s), 7.70(2H, d, J=9.0Hz), 8.18(2H, d, J=9.0Hz).

(4) tert-Butyl [1-(4-nitrophenyl)-1-methylethyl]carbamate

Di-tert-butyl dicarbonate (30.6 ml, 174 mmol) was added dropwise to a tetrahydrofuran (200 ml) solution of 1-(4-nitrophenyl)-1-methylethylamine (20 g, 111 mmol), followed by stirring at room temperature for 1 hour and at 60°C for 3 hours. The reaction solution was diluted with ethyl acetate and then, after washing with water, the solution was dried over anhydrous magnesium sulfate. The solvent was removed by evaporation under reduced pressure to give tert-butyl [1-(4-nitrophenyl)-1-methylethyl]carbamate (23.6 g, 80%) as solid.
$^1$H-NMR($CDCl_3$)δ: 1.38(9H, s), 1.64(6H, s), 5.04(1H, bs), 7.56(2H, d, J=9.0Hz), 8.18(2H, d, J=9.0Hz).

(5) tert-Butyl [1-(4-aminophenyl)-1-methylethyl]carbamate

Palladium supported on carbon (5%, 2.36 g) was added to a methanol (500 ml) solution of tert-butyl [1-(4-nitrophenyl)-1-methylethyl]carbamate (23.6 g, 84.2 mmol). The resulting mixture was subjected to hydrogenation for 2 hours under the conditions of ambient temperature and normal pressure. After the completion of the reaction, the catalyst was removed by filtration and the filtrate was concentrated under reduced pressure to give tert-butyl [1-(4-aminophenyl)-1-methylethyl]carbamate (21 g, 99.5%) as an oil.
$^1$H-NMR($CDCl_3$)δ: 1.37(9H, s), 1.60(6H, s), 4.87(1H, bs), 6.64(2H, d, J=8.6Hz), 7.19(2H, d, J=8.6Hz).

(6) tert-Butyl [1-methyl-1-[4-[(2-nitrophenyl)amino]phenyl]ethyl]carbamate

A mixture of tert-butyl [1-(4-aminophenyl)-1-methylethyl]carbamate (21 g. 83.9 mmol), o-fluoronitrobenzene (26.5 ml, 252 mmol) and potassium carbonate (11.6 g, 83.9 mmol) was stirred at 140°C for 3.5 hours. After cooling, the reaction mixture was diluted with ethyl acetate, washed with water, and after drying over anhydrous magnesium sulfate, concentrated under reduced pressure. The residue was solidified from hexane-diisopropyl ether to give tert-butyl [1-methyl-1-[4-[(2-nitrophenyl)amino]phenyl]ethyl]carbamate (12 g, 39%) as solid.
$^1$H-NMR($CDCl_3$)δ: 1.39(9H, bs), 1.65(6H, s), 4.96(1H, bs), 6.76(1H, t, J=8.4Hz), 7.17-7.24(3H, m), 7.35(1H, d, J=8.4Hz), 7.44(2H, d, J=8.8Hz), 8.19(1H, d, J=8.4Hz), 9.48(1H, bs).

(7) tert-Butyl [1-[4-[(2-aminophenyl)amino]phenyl]-1-methylethyl]carbamate

Palladium supported on carbon (5%, 1.6 g) was added to a methanol (500 ml) solution of tert-butyl [1-methyl-1-[4-[(2-nitrophenyl)amino]phenyl]ethyl]carbamate (15.5 g, 41.6 mmol). The resulting mixture was subjected to hydrogenation for 2 hours under the conditions of ambient temperature and normal pressure. The catalyst was removed by filtration and the filtrate was concentrated under reduced pressure to give tert-butyl [1-[4-[(2-aminophenyl)amino]phenyl]-1-methylethyl]carbamate (12.7 g, 89%) as solid.
$^1$H-NMR($CDCl_3$)δ: 1.37(9H, bs), 1.61(6H, s), 3.75(2H, bs), 4.87(1H, bs), 5.16(1H, bs), 6.68-7.26(8H, m).

(8) tert-Butyl [1-[4-[[2-(4-biphenylmethyl)aminophenyl]amino]phenyl]-1-methylethyl]carbamate

Acetic acid (1.67 ml) and sodium cyanoborohydride (1.11 g, 18.3 mmol) were added to a methanol solution (125 ml) of tert-butyl [1-[4-[(2-aminophenyl)amino]phenyl]-1-methylethyl]carbamate (5.0 g, 14.6 mmol) and 4-phenylbenzaldehyde (2.66 g, 14.6 mmol), followed by stirring at 60°C for 1 hour. The reaction solution was poured into water and extracted with ethyl acetate. After washing with water, the extract solution was dried over anhydrous magnesium sulfate. After concentration under reduced pressure, the residue was purified by a silica gel column chromatography to give tert-butyl [1-[4-[[2-(4-biphenylmethyl)aminophenyl]amino]phenyl]-1-methylethyl]carbamate (7.2 g, 98%) as an oil.
$^1$H-NMR($CDCl_3$)δ: 1.37(9H, bs), 1.61(6H, s), 4.39(2H, bs), 4.60(1H, bs), 4.87(1H, bs), 5.09(1H, bs), 6.67-7.60(17H, m).

(9) Ethyl N-(4-biphenylmethyl)-N-[2-[4-(1-tert-butoxycarbonylamino-1-methyl)phenylamino]phenyl]malonamate

To an ice-cooled stirred tetrahydrofuran (100 ml) solution of tert-butyl [1-[4-[[2-(4-biphenylmethyl)aminophenyl]amino]phenyl]-1-methylethyl]carbamate (6.0 g, 11.8 mmol) was added a tetrahydrofuran (3 ml) solution of triethylamine (1.81 ml, 13 mmol) and ethyl malonyl chloride (1.66 ml, 13 mmol). The resulting mixture was stirred at 0°C for 1 hour and then triethylamine (0.822 ml, 5.90 mmol) and ethyl malonyl chloride (0.755 ml, 5.9 mmol) was further added thereto. After stirring at 0°C for 5 minutes, the reaction solution was poured into ice-water and extracted with ethyl acetate. The extract solution was washed with water and then dried over anhydrous magnesium sulfate. After concentration under reduced pressure, the residue was purified by a silica gel column chromatography to give ethyl N-(4-biphenylmethyl)-N-[2-[4-(1-tert-butoxycarbonylamino-1-methyl)phenylamino]phenyl]malonamate (4.8 g, 65%) as an oil.
$^1$H-NMR($CDCl_3$)δ: 1.27(3H, t, J=7.4Hz), 1.37(9H, bs), 1.58(6H, s), 3.22(1H, d, J=15.7Hz), 3.33(1H, d, J=15.7Hz), 4.07 (2H, q, J=7.4Hz), 4.82(1H, d, J=13.4Hz), 4.91(1H, bs), 5.01(1H, d, J=13.4Hz), 5.64(1H, bs), 6.76-7.59(17H, m).

(10) N-(4-Biphenylmethyl)-N-[2-[4-(1-tert-butoxycarbonylamino-1-methyl)phenylamino]phenyl]malonamic acid

To an ice-cooled stirred tetrahydrofuran (18 ml) and methanol (54 ml) solution of ethyl N-(4-biphenylmethyl)-N-[2-[4-(1-tert-butoxycarbonylamino-1-methyl)phenylamino]phenyl]malonamate (4.62 g, 7.43 mmol) was added IN sodium hydroxide aqueous solution (14.9 ml). The resulting mixture was stirred at 0°C for 10 minutes and at room temperature for 2 hours. Water was added to the reaction solution and the mixture was washed with diisopropyl ether. Potassium hydrogen sulfate (2.0 g, 14.9 mmol) was added thereto and then the mixture was extracted with ethyl acetate. After washing with water, the extract solution was dried.over anhydrous magnesium sulfate. The solution was concentrated under reduced pressure to give N-(4-biphenylmethyl)-N-[2-[4-(1-tert-butoxycarbonylamino-1-methyl) phenylamino]phenyl]malonamic acid (3.3 g, 74%) as an oil.
$^1$H-NMR($CDCl_3$)δ: 1.36(9H, bs), 1.54(6H, s), 3.20(1H, d, J=19.6Hz), 3.31(1H, d, J=19.6Hz), 4.43(1H, d, J=14.4Hz), 4.91(1H, bs), 5.50(1H, d, J=14.4Hz), 6.58(2H, d, J=8.4Hz), 6.88-7.62(15H, m).

(11) 5-(4-Biphenylmethyl)-1-[4-(1-tert-butoxycarbonylamino-1-methylethyl)phenyl]-2,4-dioxo-2,3,4,5-tetrahydro-1H-1,5-benzodiazepine

To an ice-cooled stirred N,N-dimethylformamide (270 ml) solution of N-(4-biphenylmethyl)-N-[2-[4-(1-tert-butoxycarbonylamino-1-methyl)phenylamino]phenyl]malonamic acid (2.69 g, 4.53 mmol) were added 4-dimethylaminopyridine (553 mg, 4.53 mmol) and 1-ethyl-3-(3-dimethylaminopropyl)carbodiimide hydrochloride (2.61 g, 13.6 mmol). The resulting mixture was stirred at room temperature for 48 hours. The reaction solution was poured into water and extracted with ethyl acetate. The extract solution was washed with water, a saturated sodium hydrogen carbonate aqueous solution, and water, successively, and then dried over anhydrous magnesium sulfate. After concentration under reduced pressure, the residue was purified by a silica gel column chromatography to give 5-(4-biphenylmethyl)-1-[4-(1-tert-butoxycarbonylamino-1-methylethyl)phenyl]-2,4-dioxo-2,3,4,5-tetrahydro-1H-1,5-benzodiazepine (1.41 g, 54%) as amorphous solid.
$^1$H-NMR($CDCl_3$)δ: 1.34(9H, bs), 1.49(3H, s), 1.52(3H, s), 3.57(2H, s), 4.72(1H, d, J=14.8Hz), 4.82(1H, bs), 5.90(1H, d, J=14.8Hz), 6.57(2H, d, J=8.6Hz), 6.85(1H, d, J=7.6Hz), 7.05-7.58(14H, m).

(12) Methyl 5-(4-biphenylmethyl)-1-[4-(1-tert-butoxycarbonylamino-1-methylethyl)phenyl]-2,4-dioxo-2,3,4,5-tetrahydro-1H-1,5-benzodiazepine-3-acetate

To a stirred N,N-dimethylformamide (20 ml) solution of 5-(4-biphenylmethyl)-1-[4-(1-tert-butoxycarbonylamino-1-methylethyl)phenyl]-2,4-dioxo-2,3,4,5-tetrahydro-1H-1,5-benzodiazepine (1.3 g, 2.26 mmol) was added 60% oily sodium hydride (199 mg, 4.97 mmol). After stirring at room temperature for 30 minutes, methyl bromoacetate (0.257 ml, 2.71 mmol) was added thereto. The resulting mixture was stirred at room temperature for 1 hour. Thereafter, the reaction solution was poured into ice-water and extracted with ethyl acetate. After washing with water, the extract solution was dried over anhydrous magnesium sulfate. After concentration under reduced pressure, the residue was purified by a silica gel column chromatography to give methyl 5-(4-biphenylmethyl)-1-[4-(1-tert-butoxycarbonylamino-1-methylethyl)phenyl]-2,4-dioxo-2,3,4,5-tetrahydro-1H-1,5-benzodiazepine-3-acetate (0.81 g, 56%) as an oil.
$^1$H-NMR($CDCl_3$)δ: 1.34(9H, bs), 1.47(3H, s), 1.51(3H, s), 3.15-3.20(2H,m), 3.71(3H, s), 3.96(1H, t, J=7.0Hz), 4.75(1H, d, J=14.8Hz), 4.79(1H, bs), 5.86(1H, d, J=14.8Hz), 6.51(2H, d, J=8.8Hz), 6.87(1H, d, J=8.4Hz), 7.07-7.59(14H, m).

(13) 5-(4-Biphenylmethyl)-1-[4-(1-tert-butoxycarbonylamino-1-methylethyl)phenyl]-2,4-dioxo-2,3,4,5-tetrahydro-1H-1,5-benzodiazepine-3-acetic acid

To a stirred tetrahydrofuran (8 ml) and methanol (8 ml) solution of methyl 5-(4-biphenylmethyl)-1-[4-(1-tert-butoxycarbonylamino-1-methylethyl)phenyl]-2,4-dioxo-2,3,4,5-tetrahydro-1H-1,5-benzodiazepine-3-acetate (0.78 g, 1.2 mmol) was added IN sodium hydroxide aqueous solution (3 ml, 3 mmol). The resulting mixture was stirred at 60°C for 1 hour. After cooling of the reaction solution, water and potassium hydrogen sulfate (0.41 g, 3 mmol) were added thereto. The mixture was extracted with ethyl acetate and, after washing with water, the extract solution was dried over anhydrous magnesium sulfate. The solution was concentrated under reduced pressure to give 5-(4-biphenylmethyl)-1-[4-(1-tert-butoxycarbonylamino-1-methylethyl)phenyl]-2,4-dioxo-2,3,4,5-tetrahydro-1H-1,5-benzodiazepine-3-acetic acid (0.75 g, 98%) as an oil. $^1$H-NMR($CDCl_3$)δ: 1.34(9H, s), 1.47(3H, s), 1.50(3H, s), 3.20(2H, d, J=6.6Hz), 3.92 (1H, t, J=6.6Hz), 4.76(1H, d, J=14.4Hz), 4.80(1H, bs), 5.85(1H, d, J=14.4Hz), 6.52(2H, d, J=8.8Hz), 6.87(1H, d, J=7.4Hz), 7.13-7.59(14H, m).

(14) N-(2-Fluorobenzyl)-5-(4-biphenylmethyl)-1-[4-(1-tert-butoxycarbonylamino-1-methylethyl)phenyl]-2,4-dioxo-2,3,4,5-tetrahydro-1H-1,5-benzodiazepine-3-acetamide

To an ice-cooled stirred N,N-dimethylformamide (2 ml) solution of 5-(4-biphenylmethyl)-1-[4-(1-tert-butoxycarbonylamino-1-methylethyl)phenyl]-2,4-dioxo-2,3,4,5-tetrahydro-lH-1,5-benzodiazepine-3-acetic acid (0.2 g, 0.32 mmol) were added 2-fluorobenzylamine (0.057 ml, 0.35 mmol), diethyl cyanophosphate (0.057 ml, 0.38 mmol) and triethylamine (0.057 ml, 0.38 mmol). The resulting mixture was stirred at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-fluorobenzyl)-5-(4-biphenylmethyl)-1-[4-(1-tert-butoxycarbonylamino-1-methylethyl) phenyl]-2,4-dioxo-2,3,4,5-tetrahydro-1H-1,5-benzodiazepine-3-acetamide (160mg, 69%) as amorphous solid.
$^1$H-NMR($CDCl_3$)δ: 1.35(9H, bs), 1.47(3H, s), 1.51(3H, s), 3.02(2H, d, J=6.8Hz), 4.06(1H, t, J=6.8Hz), 4.41-4.62(2H, m), 4.73(1H, d, J=14.6Hz), 4.81(1H, bs), 5.84(1H, d, J=14.6Hz), 6.37(1H, t, J=7.0Hz), 6.48(2H, d, J=8.4Hz), 6.84(1H, d, J=8.0Hz), 6.99-7.57(18H, m).

(15) N-(2-Fluorobenzyl)-1-[4-(1-amino-1-methylethyl)phenyl]-5-(4-biphenylmethyl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-fluorobenzyl)-5-(4-biphenylmethyl)-1-[4-(1-tert-butoxycarbonylamino-1-methylethyl)phenyl]-2,4-dioxo-2,3,4,5-tetrahydro-1H-1,5-benzodiazepine-3-acetamide (160 mg, 0.2 mmol). The resulting mixture was stirred at room temperature for 1 hour and then concentrated under reduced pressure. The residue was crystallized from ethyl ether to give N-(2-fluorobenzyl)-1-[4-(1-amino-1-methylethyl)phenyl]-5-(4-biphenylmethyl)-2,4-dioxo-2,3,4,5-tetrahydro-1H-1,5-benzodiazepine-3-acetamide hydrochloride (140 mg, 86%) as amorphous solid. $^1$H-NMR(DMSO-$d_6$)δ: 1.53(6H, s), 2.76-2.99(2H, m), 3.94(1H, t, J=6.6Hz), 4.30(2H, d, J=5.6Hz), 4.99(1H, d, J=15.2Hz), 5.70(1H, d, J=15.2Hz), 6.66 (2H, d, J=8.8Hz), 6.80(1H, d, J=8.4Hz), 7.12-7.68(17H, m), 7.88(1H, d, J=8.4Hz), 8.51(3H, bs), 8.63(1H, t, J=5.6Hz).

Example 44

N-(2-Fluorobenzyl)-1-[4-(aminomethyl)phenyl]-5-(4-biphenylmethyl)-7-chloro-2,4-dioxo-2,3,4,5-tetrahydro-1H-1,5-benzodiazepine-3-acetamide hydrochloride

(1) tert-Butyl [4-[(5-chloro-2-nitrophenyl)amino]benzyl]carbamate

The compound was synthesized in a similar manner to (1) of Example 1 starting with tert-butyl (4-aminobenzyl) carbamate and 4-chloro-2-fluoronitrobenzene. $^1$H-NMR($CDCl_3$)δ: 1.48(9H, s), 4.35(2H, d, J=5.6Hz), 4.92(1H, bs), 6.72 (1H, dd, J=1.0,9.2Hz), 7.11(1H, d, J=1.0Hz), 7.23(2H, d, J=8.5Hz), 7.37(2H, d, J=8.5Hz), 8.16(1H, d, J=9.2Hz), 9.52 (1H, bs).

(2) tert-Butyl [4-[(2-amino-5-chlorophenyl)amino]benzyl]carbamate

The compound was synthesized in a similar manner to (2) of Example 1 starting with tert-butyl [4-[(5-chloro-2-nitrophenyl)amino]benzyl]carbamate.
$^1$H-NMR($CDCl_3$)δ: 1.46(9H, s), 2.40-2.60(2H, m), 4.23(2H, d, J=5.4Hz), 4.78(1H, bs), 5.23(1H, bs), 6.70-7.18(7H, m).

(3) tert-Butyl [4-[[2-(4-biphenylmethylamino)-5-chlorophenyl]amino]benzyl]carbamate

The compound was synthesized in a similar manner to (3) of Example 1 starting with tert-butyl [4-[(2-amino-5-chlorophenyl)amino]benzyl]carbamate. $^1$H-NMR($CDCl_3$)δ: 1.46(9H, s), 4.23(2H, d, J=5.4Hz), 4.36(2H, s), 4.75(1H, bs), 5.13(1H, bs), 6.60-7.60(15H, m).

(4) Ethyl N-(4-biphenylmethyl)-N-[2-[4-(tert-butoxycarbonylaminomethyl)phenylamino]phenyl]malonamate

The compound was synthesized in a similar manner to (9) of Example 43 starting with tert-butyl [4-[[2-(4-biphenylmethylamino)-5-chlorophenyl]amino]benzyl]carbamate.
$^1$H-NMR($CDCl_3$)δ: 1.26(3H, t, J=7.0Hz), 1.47(9H, s), 3.29(1H, d, J=18Hz), 3.30(1H, d, J=18Hz), 4.09(2H, q, J=7.0Hz). 4.22(2H, d, J=5.8Hz), 4.76(1H, d, J=14.0Hz), 4.75(1H, bs), 5.04(1H, d, J=14.0Hz), 5.76(1H, s), 6.73-6.89(4H, m), 7.12-7.16(2H, m), 7.32-7.56(10H, m).

(5) 5-(4-biphenylmethyl)-1-[4-(tert-butoxycarbonylaminomethyl)phenyl]-7-chloro-2,4-dioxo-2,3,4,5-tetrahydro-lH-1,5-benzodiazepine

The compound was synthesized in similar manners to (10) and (11) of Example 43 starting with ethyl N-(4-biphenylmethyl)-N-[2-[4-(tert-butoxycarbonylaminomethyl)phenylamino]phenyl]malonamate.
$^1$H-NMR($CDCl_3$)δ: 1.45(9H, s), 3.56(2H, s), 4.23(2H, d, J=5.7Hz), 4.66(1H, d, J=14.8Hz), 4.79(1H, bs), 5.87(1H, d, J=14.8Hz), 6.63(2H, d, J=8.6Hz), 6.80(1H, d, J=2.6Hz), 7.10-7.56(13H, m).

(6) Methyl 5-(4-biphenylmethyl)-1-[4-(tert-butoxycarbonylaminomethyl)phenyl]-7-chloro-2,4-dioxo-2,3,4,5-tetrahydro-lH-1,5-benzodiazepine-3-acetate

The compound was synthesized in a similar manner to (12) of Example 43 starting with 5-(4-biphenylmethyl)-1-[4-(tert-butoxycarbonylaminomethyl)phenyl]-7-chloro-2,4-dioxo-2,3,4,5-tetrahydro-1H-1,5-benzodiazepine.
$^1$H-NMR($CDCl_3$)δ: 1.44(9H, s), 3.16(2H, d, J=7.2Hz), 3.71(3H, s), 3.93(1H, t, J=7.2Hz), 4.22(2H, m), 4.70(1H, d, J=14.6Hz), 4.79(1H, bs), 5.84(1H, d, J=14.6Hz), 6.56(2H, d, J=8.2Hz), 6.82(1H, d, J=2.2Hz), 7.10(1H, d, J=8.2Hz), 7.08-7.57(11H, m).

(7) N-(2-Fluorobenzyl)-5-(4-biphenylmethyl)-1-[4-(tert-butoxycarbonylaminomethyl)phenyl]-7-chloro-2,4-dioxo-2,3,4,5-tetrahydro-1H-1,5-benzodiazepine-3-acetamide

The compound was synthesized in similar manners to (13) and (14) of Example 43 starting with methyl 5-(4-biphenylmethyl)-1-[4-(tert-butoxycarbonylaminomethyl)phenyl]-7-chloro-2,4-dioxo-2,3,4,5-tetrahydro-lH-1,5-benzodiazepine-3-acetate.
$^1$H-NMR($CDCl_3$)δ: 1.45(9H, s), 3.02(2H, d, J=6.6Hz), 4.04(1H, t, J=6.6Hz), 4.24(2H, m), 4.50(2H, t, J=5.1Hz). 4.68 (1H, d, J=14.8Hz), 4.75(1H, bs), 5.83(1H, d, J=14.8Hz), 6.27(1H, m), 6.54(2H, d, J=8.4Hz), 6.79(1H, d, =2.2Hz), 7.05-7.55(17H, m).

(8) N-(2-Fluorobenzyl)-1-[4-(aminomethyl)phenyl]-5-(4-biphenylmethyl)-7-chloro-2,4-dioxo-2,3,4,5-tetrahydro-1H-1,5-benzodiazepine-3-acetamide hydrochloride

The compound was synthesized in a similar manner to (15) of Example 43 starting with N-(2-fluorobenzyl)-5-(4-biphenylmethyl)-1-[4-(tert-butoxycarbonylaminomethyl)phenyl]-7-chloro-2,4-dioxo-2,3,4,5-tetrahydro-1H-1,5-benzodiazepine-3-acetamide.
$^1$H-NMR(DMSO-$d_6$)δ: 2.76-3.03(2H, m), 3.94-4.00(3H, m), 4.30(2H, d, J=5.4Hz), 5.50(1H, d, J=15.2Hz), 5.64(1H, d, J=15.2Hz), 6.75(1H, d, J=2.6Hz), 6.82(2H, d, J=8.0Hz). 7.10-7.65(16H, m), 7.88(1H, d, J=9.0Hz), 8.32(3H, bs), 8.63 (1H, t, J=5.8Hz).

Example 45

N-Phenyl-1-(4-aminomethylphenyl)-5-(4-biphenylmethyl)-2,4-dioxo-2,3,4,5-tetrahydro-1H-1,5-benzodiazepine-3-acetamide hydrochloride

(1) N-Phenyl-5-(4-biphenylmethyl)-1-(4-tert-butoxycarbonylaminomethylphenyl)-2,4-dioxo-2,3,4,5-tetrahydro-1H-1,5-benzodiazepine-3-acetamide

To an ice-cooled stirred N,N-dimethylformamide (2 ml) solution of 5-(4-biphenylmethyl)-1-(4-tert-butoxycarbonylaminomethylphenyl)-2,4-dioxo-2,3,4,5-tetrahydro-1H-1,5-benzodiazepine-3-acetic acid (0.2 g, 0.33 mmol) given in a similar manner to (6) of Example 1 were added aniline (62 mg, 0.66 mmol), 4-dimethylaminopyridine (40 mg, 0.32 mmol) and WSC (73 mg, 0.38 mmol). The resulting mixture was stirred at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-phenyl-5-(4-biphenylmethyl)-1-(4-tert-butoxycarbonylaminomethylphenyl)-2,4-dioxo-2,3,4,5-tetrahydro-1H-1.5-benzodiazepine-3-acetamide (144 mg. 63%) as solid.
$^1$H-NMR($CDCl_3$)δ: 1.44(9H, s), 3.16-3.23(2H. m), 4.09(1H, t, J=7.0Hz), 4.21(2H, d, J=6.2Hz), 4.75(1H, bs), 4.79(1H, d, J=15.0Hz), 5.82(1H, d, J=15.0Hz), 6.62(2H, d, J=8.4Hz), 6.83(1H, dd, J=1.4,8.2Hz), 7.06-7.56(18H, m), 7.96(1H. s).

(2) N-Phenyl-1-(4-aminomethylphenyl)-5-(4-biphenylmethyl)-2,4-dioxo-2,3,4,5-tetrahydro-1H-1,5-benzodiazepine-3-acetamide

A 4N ethyl acetate solution (1 ml) of hydrogen chloride was added to a stirred ethyl acetate (1 ml) solution of N-phenyl-5-(4-biphenylmethyl)-1-(4-tert-butoxycarbonylaminomethylphenyl)-2,4-dioxo-2,3,4,5-tetrahydro-1H-1,5-benzodiazepine-3-acetamide (144 mg, 0.21 mmol). The resulting mixture was stirred at room temperature for 1 hour and then concentrated under reduced pressure. The residue was solidified from ethyl ether to give N-phenyl-1-(4-aminomethylphenyl)-5-(4-biphenylmethyl)-2,4-dioxo-2,3,4,5-tetrahydro-1H-1,5-benzodiazepine-3-acetamide hydrochloride (110 mg, 84%) as amorphous solid. $^1$H-NMR(DMSO-$d_6$)δ: 3.06-3.14(2H, m), 3.97-4.10(3H, m), 5.07(1H, d, J=15.0Hz). 5.70(1H, d, J=15.0Hz), 6.85(2H, d, J=8.0Hz), 7.05(1H, t, J=7.2Hz), 7.23-7.63(18H, m), 7.89(1H, d, J=7.8Hz), 8.30(3H, bs), 10.23(1H, s).

Example 46

N-(2-Phenylethyl) -1-(4-aininomethylphenyl)-5-(4-biphenylmethyl)-2,4-dioxo-2,3,4,5-tetrahydro-1H-1,5-benzodiazepine-3-acetamide hydrochloride

(1) N-(2-Phenylethyl)-5-(4-biphenylmethyl)-1-(4-tert-butoxycarbonylaminomethylphenyl)-2,4-dioxo-2,3,4,5-tetrahydro-1H-1,5-benzodiazepine-3-acetamide

To an ice-cooled stirred N,N-dimethylformamide (2 ml) solution of 5-(4-biphenylmethyl)-1-(4-tert-butoxycarbonylaminomethylphenyl)-2,4-dioxo-2,3,5-tetrahydro-1H-1,5-benzodiazepine-3-acetic acid (0.2 g, 0.33 mmol) were added 2-phenylethylamine (0.046 ml, 0.35 mmol), diethyl cyanophosphate (0.059 ml, 0.40 mmol) and triethylamine (0.055 ml, 0.40 mmol). The resulting mixture was stirred at room temperature for 12 hours. The reaction solution was poured into water and extracted with ethyl acetate. The extract solution was washed with water and dried over anhydrous magnesium sulfate, followed by concentration under reduced pressure. The residue was solidified from diethyl ether to give N-(2-phenylethyl)-5-(4-biphenylmethyl)-1-(4-tert-butoxycarbonylaminomethylphenyl)-2,4-dioxo-2,3,5-tetrahydro-1H-1,5-benzodiazepine-3-acetamide (84 mg, 36%) as amorphous solid.
$^1$H-NMR($CDCl_3$)δ: 1.44(9H, s), 2.81(2H, t, J=7.4Hz), 2.91-2.96(2H, m), 3.50-3.55(2H, m), 4.05(1H, t, J=6.8Hz), 4.21 (2H, d, J=5.8Hz), 4.73(1H, bs), 4.77(1H, d, J=14.6Hz), 5.81(1H, d, J=14.6Hz), 5.95(1H, bs), 6.59(2H, d, J=8.4Hz), 6.82 (1H, d, J=8.4Hz), 7.07(2H, d, J=8.4Hz), 7.19-7.56(17H, m).

(2) N-(2-Phenylethyl)-1-(4-aminomethylphenyl)-5-(4-biphenylmethyl)-2,4-dioxo-2,3,4,5-tetrahydro-1H-1,5-benzodiazepine-3-acetamide hydrochloride

A 4N ethyl acetate solution (1 ml) of hydrogen chloride was added to a stirred ethyl acetate (1 ml) solution of N-(2-phenylethyl)-5-(4-biphenylmethyl)-1-(4-tert-butoxycarbonylaminomethylphenyl)-2,4-dioxo-2,3,4,5-tetrahydro-1H-1,5-benzodiazepine-3-acetamide (84 mg, 0.12 mmol). The resulting mixture was stirred at room temperature for 1 hour and then concentrated under reduced pressure. The residue was solidified from ethyl ether to give N-(2-phenylethyl)-1-(4-aminomethylphenyl)-5-(4-biphenylmethyl)-2,4-dioxo-2,3,4,5-tetrahydro-1H-1,5-benzodiazepine-3-acetamide hydrochloride (65 mg, 85%) as amorphous solid.

$^1$H-NMR(DMSO-$d_6$)δ: 2.66-2.95(2H, m), 3.20-3.27(2H, m), 3.93(1H, t, J=7.0Hz). 3.98(2H, d, J=6.0Hz), 5.05(1H, d, J=15.4Hz), 5.65(1H, d, J=15.4Hz), 6.78-7.65(21H, m), 7.84(1H, d, J=7.6Hz), 8.22(1H, t, J=5.6Hz), 8.23(3H, bs).

The followings are some examples of the pharmacological actions of the compounds of the invention, but the invention is not limited to them. The genetic operation using E. coli was conducted in accordance with the method described in the 1989 Edition of Molecular Cloning (T. Maniatis, et al.).

(1) Cloning of human somatostatin receptor protein subtype 1 (SSTR1) DNA

DNA oligomers S1-1 and S1-2 were synthesized based on the known human SSTRIc DNA sequence (Proc. Natl. Acad. Sci., USA, vol. 89, pp. 251-255, 1992). The sequence of S1-1 is 5'-GGTCGACCTCAGCTAGGATGTTCCCCAATG-3' (Sequence No. 1) and that of S1-2 is 5'-GGTCGACCCGGGCTCAGAGCGTCGTGAT-3' (Sequence No. 2). Human chromosome DNA (Clone Tech Inc. Catalog No. CL6550-1) was used as the template. To 0.5 ng of said DNA was added 25 pmol of each of the above DNA oligomers and the polymerase chain reaction was carried out using 2.5 units of PfuDNA polymerase (Stratagene). The composition of the reaction solution was in accordance with the directions attached to said PfuDNA polymerase. The conditions of the reaction were as follows: One cycle consisting of the reactions at 94°C for 1 minute, at 63°C for 1 minute and at 75°C for 2 minutes, and 35 cycles were repeated. The reaction mixture was subjected to electrophoresis on 1% agarose gel to find that the DNA fragments of the intended size (about 1.2 kb) was specifically amplified. Said DNA fragments were recovered from the agarose gel in the usual manner and connected to pUC118 cleaved at the Hinc II site to transform into the competent cells, Escherichia coli JM109. The transformant having plasmid containing said DNA fragments was selected out and the sequence of the inserted DNA fragments was confirmed by the automatic sequence analyzer employing fluorescent pigment, ALF DNA Sequencer (manufactured by Pharmacia). As the results, the amino acid sequence expected from the base sequence was completely in agreement with the sequence described in the above-described literature.

(2) Organization of the expression plasmid of human somatostatin receptor protein subtype 1 (SSTR1) DNA

pAKKO-111 was used as the expression vector in CHO (Chinese Hamster Ovary) cells. pAKKO-111 was organized as follows: The 1.4 kb DNA fragment containing SRα promoter and poly A appositional signal was obtained from pTB1417 described in the official gazette Japanese Patent Laid-Open No. 076385/1993 by treatment with Hind III and Cla I. The 4.5 kb DNA fragment containing dihydrofolic acid reductase (DHFR) gene was obtained from pTB348 [Biochem. Biophys. Res. Commun., vol. 128, pp. 256-264, 1985] by treatment with Cla I and Sal I. These DNA fragments were treated with T4 polymerase to make the terminal blunt-ended and connected with T4 ligase to organize pAKKO-111 plasmid. Then, 5 μg of the plasmid having human SSTR1 DNA fragment obtained in the above (1) was digested with the restriction enzyme Sal I and subjected to electrophoresis on 1% agarose gel to recover the 1.2 kb DNA fragment coded with human SSTR1. Next, 1 μg of the above-described expression vector pAKKO-111 (5.5 kb) was digested with Sal I to prepare the cloning site for insertion of human SSTR1 DNA fragment. Said expression vector fragment and the 1.2 kb DNA fragment were combined using T4DNA ligase. The reaction mixture was transduced into E. coli JM 109 by the calcium chloride method to obtain the expression plasmid pAl-11-SSTR1 in which human SSTR1 DNA fragment was inserted from the transformants in the normal direction toward the promoter. This transformant is expressed as Escherichia coli
JM109/pA-1-11-SSTR1.

(3) Transfection and expression of human somatostatin receptor protein subtype 1 (SSTR1) DNA in CHO (dhfr$^-$) cells

CHO (dhfr$^-$) cells (1 x $10^6$ cells) were cultured for 24 hours in HAM F12 medium containing 10% bovine fetal serum on a laboratory dish of 8 cm in diameter. To the cells was transfected 10 μg of the human SSTR1c DNA expression plasmid 1, pA-1-11-SSTR1, obtained in the above (2) by the calcium phosphate method (Cell Phect Transfection Kit: Pharmacia). The medium was switched to DMEM medium containing 10% dialyzed bovine fetal serum 24 hours after the transfection to select the colony-forming cells (i.e., DHFR+ cells) in this medium. Further, the selected cells were cloned from a single cell by the limiting dilution method and the somatostatin protein receptor activity was measured as follows: Human SSTRc DNA expression cell strain was diluted with a buffer solution for assay [50 mM of tris hydrochloride, 1 mM of EDTA, 5 mM of magnesium chloride, 0.1% of BSA, 0.2 mg/ml of bacitracin, 10 μg/ml of leupeptin, 1 μg/ml of pepstatin and 200 units/ml of aprotinin (pH 7.5)] to adjust the cell count to 2 x $10^4$/200 μl. 200 μl of the dilution was placed in a tube and 2 μl of 5nM [$^{125}$I]-somatostatin-14 (2000 Ci/mmol, Amersham) was added thereto. The mixture was incubated at 25°C for 60 minutes. For measurement of non-specific binding (NSB), the tube to which 2 μl of somatostatin-14 ($10^{-4}$ M) was added was also incubated. To the tube was added 1.5 ml of a buffer solution for washing

[50 mM of tris hydrochloride, 1 mM of EDTA and 5 mM of magnesium chloride (pH 7.5)] and the mixture was filtered by GF/F glass fiber filter paper (Whatman) and washed further with 1.5 ml of the same buffer solution. [$^{125}$I] of the filter was measured by a γ-counter. Thus, a highly somatostatin-binding cell strain, SSTR1-8-3, was selected.

(4) Cloning of human somatostatin receptor protein subtype 2 (SSTR2) DNA

DNA oligomers PT-1 and PT-2 were synthesized based on the known human SSTR2 cDNA sequence (Proc. Natl. Acad. Sci., USA, vol. 89, pp. 251-255, 1992). The sequence of PT-1 is 5'-GGTCGACACCATGGACATGGCGGATGAG-3' (Sequence No. 3) and that of PT-2 is 5'-GGTCGACAGTTCAGATACTGGTTTGG-3' (Sequence No. 4). Human pituitary gland cDNA (Clone Tech Inc. Catalog No. 7173-1) was used as the template. To 1 ng of said cDNA was added 25 pmol of each of the above DNA oligomers and the polymerase chain reaction was carried out using 2.5 units of TaqDNA polymerase (Takara Shuzo). The composition of the reaction mixture was in accordance with the directions attached to the above TaqDNA polymerase. The conditions of the reaction were as follows: One cycle consisting of the reactions at 94°C for 30 seconds, at 52°C for 20 seconds and at 72°C for 60 seconds, and 30 cycles were repeated. The reaction mixture was subjected to electrophoresis on 1% agarose gel to find that the DNA fragments of the intended size (about 1.1 kb) was specifically amplified. Said DNA fragments were recovered from the agarose gel in the usual manner and connected to pUC118 cleaved at the Hinc II site to transform into the competent cells, Escherichia coli JM109. Two strains (No. 5 and No. 7) of the transformant having plasmid containing said DNA fragments were selected out and the sequence of the inserted DNA fragments was confirmed by the automatic sequence analyzer employing fluorescent pigment, 373A DNA Sequencer (Applied Biosystem). As the results, point mutation was confirmed at one site in the sequence of the 770 base fragment of No. 5 strain between Sal I and Bst PI, and point mutation was also confirmed at one site in the sequence of the 360 base fragment of No. 7 strain between Bst PI and Sal I. Therefore, the fragments remaining after removing the Bst PI-Sal I fragment of No. 5 strain and the Bst PI-Sal I fragment of No. 7 strain were purified by electrophoresis on agarose to organize a plasmid in which these fragments were bound by a ligation reaction. Confirmation of the sequence of the inserted DNA fragment of this plasmid revealed that it was completely in agreement with the sequence described in the above literature.

(5) Organization of the expression plasmid of human somatostatin receptor protein subtype 2 (SSTR2) DNA

pAKKO-111 described in the above (2) was used as the expression vector in CHO (Chinese Hamster Ovary) cells. 5 μg of the plasmid having human SSTR2 cDNA fragment obtained in the above (4) was digested with the restriction enzyme Sal I and subjected to electrophoresis on 1% agarose gel to recover the 1.1 kb DNA fragment coded with human SSTR2. Next, 1 μg of the above-described expression vector pAKKO-111 (5.5 kb) was digested with Sal I to prepare the cloning site for insertion of human SSTR2 DNA fragment. Said expression vector fragment and the 1.1 kb DNA fragment were combined using T4DNA ligase. The reaction mixture was transduced into E. coli JM 109 by the calcium chloride method to obtain the expression plasmid pAC01 in which human SSTR2 DNA fragment was inserted from the transformants in the normal direction toward the promoter. This transformant is expressed as Escherichia coli JM109/pAC-01.

(6) Transfection and expression of human somatostatin receptor protein subtype 2 (SSTR2) DNA in CHO (dhfr$^-$) cells

CHO (dhfr$^-$) cells (1 x $10^6$ cells) were cultured for 24 hours in HAM F12 medium containing 10% bovine fetal serum on a laboratory dish of 8 cm in diameter. To the cells was transfected 10 μg of the human SSTR2 cDNA expression plasmid, pAC01, obtained in the above (5) by the calcium phosphate method (Cell Phect Transfection Kit: Pharmacia). The medium was switched to DMEM medium containing 10% dialyzed bovine fetal serum 24 hours after the transfection to select the colony-forming cells (i.e., DHFR$^+$ cells) in this medium. Further, the selected cells were cloned from a single cell by the limiting dilution method and a cell strain which highly expresses human SSTR2, SSTR2-HS5-9, was selected.

(7) Cloning of human somatostatin receptor protein subtype 3 (SSTR3) DNA

DNA oligomers S3-1 and S3-2 were synthesized based on the known human SSTR3 cDNA sequence (Mol. Endocrinol., vol. 6, pp. 2136-2142, 1992). The sequence of S3-1 is 5'-GGTCGACCTCAACCATGGACATGCTTCATC-3' (Sequence No. 5) and that of S3-2 is 5'-GGTCGACTTTCCCCAGGCCCCTACAGGTA-3' (Sequence No. 6). Human chromosome DNA (Clone Tech Inc. Catalog No. CL6550-1) was used as the template. To 0.5 ng of said DNA was added 25 pmol of each of the above DNA oligomers and the polymerase chain reaction was carried out using 2.5 units of PfuDNA polymerase (Strata gene). The composition of the reaction mixture was in accordance with the directions attached to said PfuDNA polymerase. The conditions of the reaction were as follows: One cycle consisting of the

reactions at 94°C for 1 minute, at 63°C for 1 minute and at 75°C for 2 minutes, and 35 cycles were repeated. The reaction mixture was subjected to electrophoresis on 1% agarose gel to find that the DNA fragments of the intended size (about 1.3 kb) was specifically amplified. As the results, the amino acid sequence expected from the base sequence was completely in agreement with the sequence described in the above literature.

(8) Organization of the expression plasmid of human somatostatin receptor protein subtype 3 (SSTR3) DNA

pAKKO-111 mentioned in the above (2) was used as the expression vector in CHO cells. 5 μg of the plasmid having human SSTR3 DNA fragment obtained in the above (7) was digested with the restriction enzyme Sal I and subjected to electrophoresis on 1% agarose gel to recover the 1.3 kb DNA fragment coded with human SSTR3. Next, 1 μg of the above-described expression vector pAKKO-111 (5.5 kb) was digested with Sal I to prepare the cloning site for insertion of human SSTR3 DNA fragment. Said expression vector and the 1.3 kb DNA fragment were combined using T4DNA ligase. The reaction mixture was transduced into E. coli JM 109 by the calcium chloride method to obtain an expression plasmid pAl-11-SSTR3 in which human SSTR3 DNA fragment was inserted from the transformants in the normal direction toward the promoter. This transformant is expressed as Escherichia coli JM109/pA-1-11-SSTR3.

(9) Transfection and expression of human somatostatin receptor protein subtype 3 (SSTR3) DNA in CHO ($dhfr^-$) cells

CHO ($dhfr^-$) cells (1 x $10^6$ cells) were cultured for 24 hours in HAM F12 medium containing 10% bovine fetal serum on a laboratory dish of 8 cm in diameter. To the cells was transfected 10 μg of the human SSTR3 DNA expression plasmid, pA-1-11-SSTR3, obtained in the above (8) by the calcium phosphate method. The medium was switched to DMEM medium containing 10% dialyzed bovine fetal serum 24 hours after the transfection to select the colony-forming cells (i.e., $DHFR^+$ cells) in this medium. Further, the selected cells were cloned from a single cell by the limiting dilution method and the somatostatin receptor protein expression activity of these cells was measured by the binding assay described in the above (3). Thus, a highly somatostatin-binding cell strain, SSTR3-15-19, was selected.

(10) Cloning of human somatostatin receptor protein subtype 4 (SSTR4) DNA

DNA oligomers S4-1 and S4-2 were synthesized based on the known human SSTR4 DNA sequence (Proc. Natl, Acad. Sci., USA, vol. 90, pp. 4196-4200, 1993). The sequence of S4-1 is 5'-GGCTCGAGTCACCATGAGCGCCCCCTCG-3' (Sequence No. 7) and that of S4-2 is 5'-GGGCTCGAGCTCCTCAGAAGGTGGTGG-3' (Sequence No. 8). Human chromosome DNA (Clone Tech Inc. Catalog No. CL6550-1) was used as the template. To 0.5 ng of said DNA was added 25 pmol of each of the above DNA oligomers and the polymerase chain reaction was carried out using 2.5 units of PfuDNA polymerase (Strata gene). The composition of the reaction mixture was in accordance with the directions attached to said PfuDNA polymerase. The conditions of the reaction were as follows: One cycle consisting of the reactions at 94°C for 1 minute, at 66°C for 1 minute and at 75°C for 2 minutes, and 35 cycles were repeated. The reaction mixture was subjected to electrophoresis on 1% agarose gel to find that the DNA fragments of the intended size (about 1.2 kb) was specifically amplified. Confirmation of the sequence of the DNA fragments by the method described in the above (1) revealed that the amino acid sequence expected from the base sequence was completely in agreement with the sequence described in the above literature.

(11) Organization of the expression plasmid of human somatostatin receptor protein subtype 4 (SSTR4) DNA

pAKKO-111 was used as the expression vector in CHO (Chinese Hamster Ovary) cells. 5 μg of the plasmid having human SSTR4 DNA fragment obtained in the above (10) was digested with a restriction enzyme Xho I and then, subjected to electrophoresis on 1% agarose gel to recover the 1.2 kb DNA fragment coded with human SSTR4, Next, 1 μ g of the above-described expression vector pAKKO-111 (5.5 kb) was digested with Sal I to prepare the cloning site for insertion of human SSTR4 DNA fragment. Said expression vector fragment and the 1.2 kb DNA fragment were combined using T4DNA ligase. The reaction mixture was transduced into E. coli JM 109 by the calcium chloride method to obtain the expression plasmid pA1-11-SSTR4 in which human SSTR4 DNA fragment was inserted from the transformants in the normal direction toward the promoter. This transformant is expressed as Escherichia coli JM109/pA-1-11-SSTR4.

(12) Transfection and expression of human somatostatin receptor protein subtype 4 (hSSTR4) DNA in CHO ($dhfr^-$) cells

CHO ($dhfr^-$) cells (1 x $10^6$ cells) were cultured for 24 hours in HAM F12 medium containing 10% bovine fetal serum on a laboratory dish of 8 cm in diameter. To the cells was transfected 10 μg of the human SSTR4 DNA expression plasmid, pA-1-11-SSTR4 obtained in the above (11) by the calcium phosphate method. The medium was switched to

DMEM Medium containing 10% dialyzed bovine fetal serum 24 hours after the transfection to select the colony-forming cells (i.e., DHFR+ cells) in this medium. Further, the selected cells were cloned from a single cell by the limiting dilution method and the somatostatin receptor protein expression activity of these cells was measured by the binding assay described in the above (3). Thus, a highly somatostatin-binding cell strain, SSTR4-1-2, was selected.

(13) Cloning of human somatostatin receptor protein subtype 5(SSTR5) DNA

DNA oligomers S5-1 and S5-2 were synthesized based on the known human SSTR5 cDNA sequence (Biochem Biophys. Res. Commun., vol. 195, pp. 844-852, 1993). The sequence of S5-1 is 5'-GGTCGACCACCATGGAGCCCCTGTTCCC-3' (Sequence No. 9) and that of S5-2 is 5'-CCGTCGACACTCTCACAGCTTGCTGG-3' (Sequence No. 10). Human chromosome DNA (Clone Tech Inc. Catalog No. CL6550-1) was used as the template. To 0.5 ng of said DNA was added 25 pmol of each of the above DNA oligomers and the polymerase chain reaction was carried out using 2.5 units of PfuDNA polymerase (Strata gene). The composition of the reaction mixture was in accordance with the directions attached to PfuDNA polymerase. The conditions of the reaction were as follows: One cycle consisting of the reactions at 94°C for 1 minute, at 66°C for 1 minute and at 75°C for 2 minutes, and 35 cycles were repeated. The reaction mixture was subjected to electrophoresis on 1% agarose gel to find that the DNA fragments of the intended size (about 1.1 kb) were specifically amplified. Confirmation of the sequence of said DNA fragment by the method described in the above (1) revealed that the amino acid sequence expected from the base sequence was completely in agreement with the sequence described in the above literature.

(14) Organization of the expression plasmid of human somatostatin receptor protein subtype 5 (SSTR5) DNA.

pAKKO-111 described in the above (2) was used as the expression vector in CHO cells. 5 µg of the plasmid having human SSTR5 DNA fragment obtained in the above (13) was digested with the restriction enzyme Sal I and subjected to electrophoresis on 1% agarose gel to recover the 1.1 kb DNA fragment coded with human SSTR5. Next, 1 µg of the above-described expression vector pAKKO-111 (5.5 kb) was digested with Sal I to prepare the cloning site for insertion of human SSTR5 DNA fragment. Said expression vector fragment and the 1.1 kb DNA fragment were combined using T4DNA ligase. The reaction mixture was transduced into E. coli JM 109 by the calcium chloride method to obtain the expression plasmid pA1-11-SSTR5 in which human SSTR5 DNA fragment was inserted from the transformants in the normal direction toward the promoter. This transformant is expressed as Escherichia coli JM109/pA-1-11-SSTR5.

(15) Transfection and expression of human somatostatin receptor protein subtype 5 (SSTR5) DNA in CHO (dhfr-) cells

CHO (dhfr-) cells (1 x $10^6$ cells) were cultured for 24 hours in HAM F12 medium containing 10% bovine fetal serum on a laboratory dish of 8 cm in diameter. To the cells was transfected 10 µg of the human SSTR5c DNA expression plasmid, pA-1-11-SSTR5. obtained in the above (14) by the calcium phosphate method. The medium was switched to DMEM medium containing 10% dialyzed bovine fetal serum 24 hours after the transfection to select the colony-forming cells (i.e., DHFR+ cells) in this medium. Further, the selected cells were cloned from a single cell by the limiting dilution method and the somatostatin receptor protein expression activity of these cells was measured by binding assay described in the above (3). Thus, a highly somatostatin-binding cell strain, SSTR5-3-2-4, was selected.

Experimental Example 1 Preparation of CHO cell membrane fractions containing human somatostatin receptor

Human somatostatin receptor expression CHO cell strain, SSTR1-8-3, SSTR2-HS5-9, SSTR3-15-19, SSTR4-1-2, or SSTR5-32-4 ($10^9$ cells) was floated on a phosphate buffered physiological saline supplemented with 5 mM EDTA (PBS-EDTA) and centrifuged. To the cell pellets was added 10ml of a homogenate buffer for cells (10mM $NaHCO_3$, 5 mM EDTA, pH=7.5), and the mixture was homogenated using a Polytron homogenizer. The supernatant obtained by centrifugation at 400 x g for 15 minutes was further centrifuged at 100,000 x g for 1 hour to give a precipitate of the membrane fraction. The precipitates were suspended in 2 ml of a buffer solution for assay [25 ml of Tris-hydrochloride, 1 ml of EDTA, 0.1% of BSA (Bovine Serum Albumin), 0.25 ml of PMSF, 1 µg/ml pepstatin, 20 µg/ml leupeptin, 10 µg/ml phosphoramidone, pH=7.5], and the suspension was centrifuged at 100,000 x g for 1 hour. The membrane fraction recovered as precipitates was suspended again in 20 ml of the buffer solution for assay, and the suspension was placed in tubes and stored at -80°C. The suspension was thawed and used at every use.

Experimental Example 2 Measurement of the binding inhibition rate of $^{125}$I-Somatostatin

The membrane fraction prepared in Experimental Example 1 was diluted with a buffer solution for assay to

adjust the concentration to 3 μg/ml. The diluate was placed in tubes each in an amount of 173 μl. To this were simultaneously added 2 μl of a solution of a compound in DMSO and 25 μl of a 200pM radioisotope-labeled somatostatin ($^{125}$I-somatostatin: Amersham). For measurement of the maximum binding, a reaction mixture added with 2 μl of DMSO and 25 μl of a 200pM $^{125}$I-somatostatin was prepared. For measurement of non-specific binding, a reaction mixture added with 2 μl of a 100 μM somatostatin solution in DMSO and 25 μl of a 200pM $^{125}$I-somatostatin solution was prepared at the same time. The mixtures were allowed to react at 25°C for 60 minutes. Then, the reaction mixture was filtered under suction using a Whatman glass filter (GF-B) treated with polyethylenimine. After filtration, the radioactivity of $^{125}$I-somatostatin remaining on the filter paper was measured by a γ-counter. The binding rate (%) of each test substance was determined by the calculation according to the equation:

$$PBM = (B\text{-}NSB)/(B_o\text{-}NSB) \times 100$$

(PBM: Percent Maximum Binding, B: radioactivity when a compound was added, $B_o$: maximum binding radioactivity, NSB: non-specific binding radioactivity). Further, the binding rates were determined by changing the concentrations of the test substance, and the 50% inhibiting concentration of the test substance ($IC_{50}$ value) was calculated from the Hill plots.

As shown in the reactivity ($IC_{50}$, μM) of the following compounds to each human somatostatin receptor determined by the above-described method, they exhibit an activity of 10 μM or less on at least one of each human somatostatin receptor.

Compounds of Examples 1 to 46

Industrial Applicability

Compounds (I) of the invention or the salts thereof have an excellent somatostatin receptor agonistic action and exhibit low toxicity. Therefore, they can be safe agents for preventing and treating the diseases which relate to these actions.

## Claims

**1.** A compound represented by the formula (I)

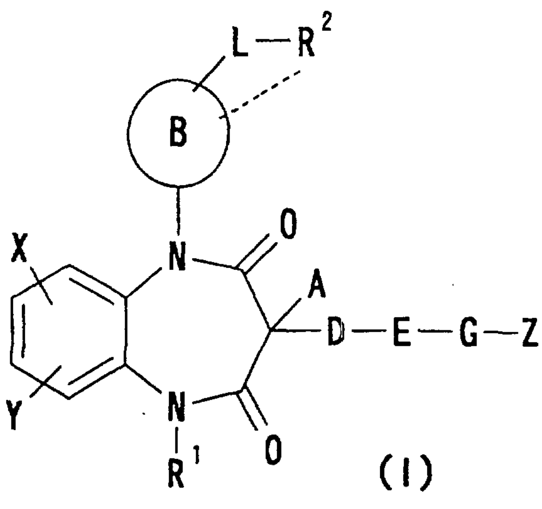

(I)

wherein ring B represents a cyclic hydrocarbon group which may have substituent(s); Z represents hydrogen atom or a cyclic group which may have substituent(s); $R^1$ represents hydrogen atom, a hydrocarbon group which may have substituent(s), a heterocyclic group which may have substituent(s) or an acyl group; $R^2$ represents amino group which may have substituent(s); D represents a bond or a divalent group; E represents a bond, -CO-, -CON($R^a$)-, -COO-, -N($R^a$)CON($R^b$)-, -N($R^a$)COO-, -N($R^a$)$SO_2$-, -N($R^a$)-, -O-, -S-, -SO- or -$SO_2$- ($R^a$ and $R^b$ each independently represents hydrogen atom or a hydrocarbon group which may have substituent(s)); G represents a bond

or a divalent group; L represents a bond or a divalent group; A represents hydrogen atom or a substituent; X and Y each represents hydrogen atom or an independent substituent; and ..... represents that $R^2$ and an atom on ring B may form a ring, or a salt thereof.

**2.** The compound according to claim 1, wherein E is -CO-, -CON($R^a$)-, -COO-, -N($R^a$)CON($R^b$)-, -N($R^a$)COO-, --N($R^a$)$SO_2$-, -N($R^a$)-, -O-, -S-, -SO- or -$SO_2$- ($R^a$ and $R^b$ each independently represents hydrogen atom or a hydrocarbon group which may have substituent(s)).

**3.** The compound according to claim 1, wherein L is

(1) a bond or,
(2) a divalent hydrocarbon group which may contain -O- or -S- and may possess 1 to 5 substituents selected from

i) a $C_{1-6}$ alkyl group,
ii) a halogeno-$C_{1-6}$ alkyl group,
iii) phenyl group,
iv) benzyl group,
v) amino group which may have substituent(s),
vi) hydroxy group which may have substituent(s), and
vii) carbamoyl groups or thiocarbamoyl groups which each may be substituted by:

a) a $C_{1-6}$ alkyl group,
b) a phenyl group which may have substituent(s), or
c) a heterocyclic group which may have substituent(s).

**4.** The compound according to claim 1, wherein Z is a cyclic group which may have substituent(s).

**5.** The compound according to claim 1, wherein D is a divalent group bonded to the ring through a carbon atom.

**6.** The compound according to claim 1, wherein ring B is benzene ring which may have substituent(s) and L is a $C_{1-6}$ alkylene group.

**7.** The compound according to claim 1, wherein G represents a divalent hydrocarbon group which may have substituent(s) and ring B does not form a ring together with $R^2$.

**8.** The compound according to claim 1, wherein A is hydrogen atom, ring B is benzene ring, Z is a phenyl group substituted by a halogen, and $R^1$ is a $C_{1-6}$ alkyl or $C_{7-14}$ aralkyl group which each may be substituted by substituent (s) selected from (1) hydroxy, (2) phenyl, (3) a $C_{1-6}$ alkyl carbonyl or a $C_{6-14}$ aryl-carbonyl, and (4) amino groups which may be substituted by a $C_{1-6}$ alkyl sulfonyl or a $C_{6-14}$ aryl-sulfonyl.

**9.** The compound according to claim 1, wherein X and Y each independently is hydrogen atom, a halogen, hydroxy, a $C_{1-6}$ alkoxy, a halogeno-$C_{1-6}$ alkoxy, a $C_{7-14}$ aralkyloxy, a benzoyl-$C_{1-6}$ alkoxy, a hydroxy-$C_{1-6}$ alkoxy, a $C_{1-6}$ alkoxy-carbonyl-$C_{1-6}$ alkoxy, a $C_{3-14}$ cycloalkyl-$C_{1-6}$ alkoxy, an imidazol-1-yl-$C_{1-6}$ alkoxy, a $C_{7-14}$ aralkyloxy-carbonyl-$C_{1-6}$ alkoxy, or a hydroxyphenyl-$C_{1-6}$ alkoxy;

ring B is benzene ring which may be substituted by a $C_{1-6}$ alkoxy, or tetrahydroisoquinoline ring or isoindoline ring which is formed by combination with $R^2$;
Z is a $C_{6-14}$ aryl group, a $C_{3-10}$ cycloalkyl group, piperidyl group, thienyl group, furyl group, pyridyl group, thiazolyl group, indanyl group or indolyl group which may have 1 to 3 substituents selected from a halogen, formyl, a halogeno-$C_{1-6}$ alkyl, a $C_{1-6}$ alkoxy, a $C_{1-6}$ alkyl-carbonyl, oxo and pyrrolidinyl;
A is hydrogen atom;
D is a $C_{1-6}$ alkylene group;
G is a bond, or a $C_{1-6}$ alkylene group which may contain phenylene and may be substituted by phenyl;
$R^1$ is hydrogen atom, a $C_{1-6}$ alkyl group, a $C_{2-6}$ alkenyl group, a $C_{6-14}$ aryl group or a $C_{7-14}$ aralkyl group which each may be substituted by substituent(s) selected from (1) a halogen, (2) nitro, (3) amino which may have 1 or 2 substituents selected from a $C_{1-6}$ alkyl which may be substituted by a $C_{1-6}$ alkyl-carbonyl, benzoyloxycarbonyl and a $C_{1-6}$ alkylsulfonyl, (4) hydroxy which may be substituted by (i) a $C_{1-6}$ alkyl which may be substituted

by hydroxy, a $C_{1-6}$ alkyl-carbonyl, carboxy or a $C_{1-6}$ alkoxy-carbonyl, (ii) phenyl which may be substituted by hydroxy, (iii) benzoyl or (iv) a mono- or di- $C_{1-6}$ alkylamino-carbonyl, (5) a $C_{3-6}$ cycloalkyl, (6) phenyl which may be substituted by hydroxy or a halogeno-$C_{1-6}$ alkyl and (7) thienyl, furyl, thiazolyl, indolyl or benzyloxy-carbonylpiperidyl;

$R^2$ is (1) unsubstituted amino group, (2) piperidyl group or (3) amino which may have 1 or 2 substituents selected from (i) benzyl, (ii) a $C_{1-6}$ alkyl which may be substituted by amino or phenyl, (iii) a mono- or di-$C_{1-6}$ alkyl-carbamoyl, or a mono- or di-$C_{1-6}$ alkyl-thiocarbamoyl, (iv) a $C_{1-6}$ alkoxy-carbonyl, (v) a $C_{1-6}$ alkyl-sulfonyl, (vi) piperidylcarbonyl and (vii) a $C_{1-6}$ alkyl-carbonyl which may be substituted by a halogen or amino;

E is a bond, -CON($R^a$)-, - N($R^a$)CO-, -N($R^a$)CON($R^b$)- ($R^a$ and $R^b$ each represents hydrogen atom or a $C_{1-6}$ alkyl group);

L is a $C_{1-6}$ alkylene group which may contain -O- and may be substituted by a $C_{1-6}$ alkyl.

**10.** The compound according to claim 1, wherein X and Y each independently is hydrogen atom, a halogen, hydroxy or a $C_{1-6}$ alkoxy;

ring B is benzene ring or, by combination with $R^2$, tetrahydroisoquinoline ring or isoindoline ring;

Z is phenyl group which may be substituted by a halogen, D is a $C_{1-6}$ alkylene group, G is a $C_{1-6}$ alkylene group;

$R^1$ is a $C_{1-6}$ alkyl group or a $C_{7-14}$ aralkyl group which each may be substituted by substituent(s) selected from (1) hydroxy, (2) phenyl and (3) amino which may be substituted by a $C_{1-6}$ alkyl-carbonyl or a $C_{1-6}$ alkylsulfonyl;

$R^2$ is unsubstituted amino group;

E is -CONH-;

L is a $C_{1-6}$ alkylene group.

**11.** A prodrug of the compound according to claim 1 or a salt thereof.

**12.** A process for producing a compound of the formula (I-a)

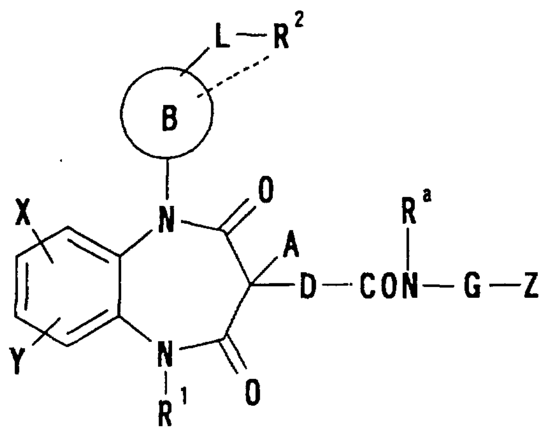

(I-a)

[wherein the symbols have the same meanings as described above] or a salt thereof which comprises:

reacting a compound represented by the formula (IIa)

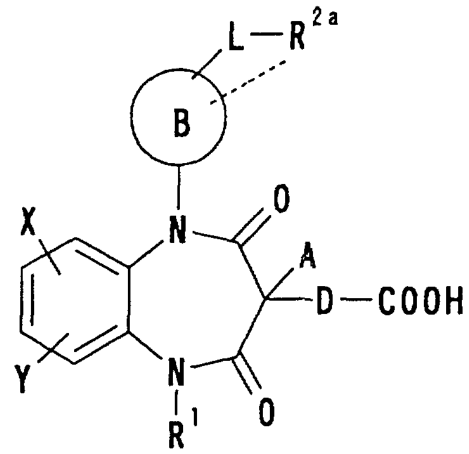

(IIa)

[wherein $R^{2a}$ represents amino group which may be protected and substituted, and other symbols have the same meanings as described in claim 1], a reactive derivative thereof or a salt thereof, with a compound represented by the formula

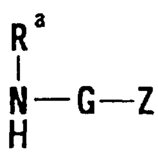

(III)

[wherein the symbols have the same meanings as described in the claim 1] or a salt thereof to produce a compound of the formula (Ia-a)

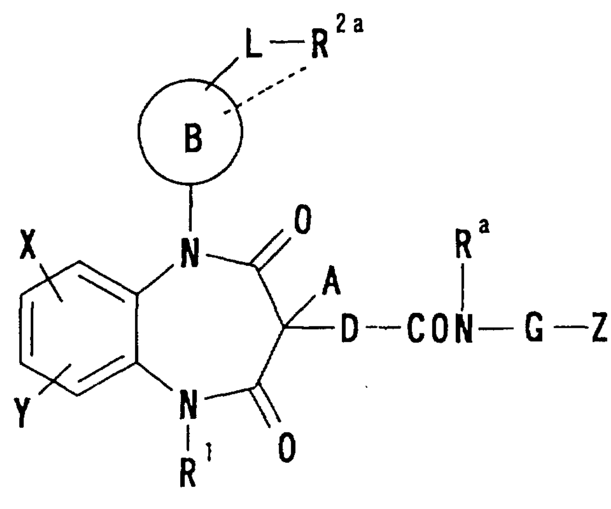

(Ia-a)

[wherein the symbols have the same meanings as described above] or a salt thereof, and optionally, subjecting it to de-protecting reaction.

**13.** A pharmaceutical composition which comprises a compound according to claim 1 or a salt thereof.

**14.** A pharmaceutical composition according to claim 13 which is a somatostatin receptor function regulator.

**15.** A pharmaceutical composition according to claim 14 wherein the somatostatin receptor function regulator is a somatostatin receptor agonist.

**16.** A pharmaceutical composition according to claim 13 which is an agent for preventing or treating diabetes, obesity, diabetic complications or intractable diarrhea.

**17.** A method for regulating a somatostatin receptor function which comprises administering a compound represented by the formula (I)

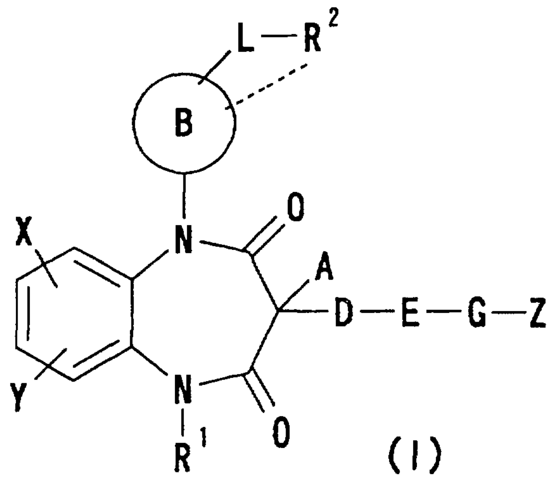

(I)

[wherein ring B represents a cyclic hydrocarbon group which may have substituent(s); Z represents hydrogen atom or a cyclic group which may have substituent(s); $R^1$ represents hydrogen atom, a hydrocarbon group which may have substituent(s), a heterocyclic group which may have substituent(s) or an acyl group; $R^2$ represents amino group which may have substituent(s); D represents a bond or a divalent group; E represents a bond, -CO-, -CON($R^a$)-, -COO-, -N($R^a$)CON($R^b$)-, -N($R^a$)COO-. -N($R^a$)$SO_2$-, -N($R^a$)-, -O-, -S-, -SO- or -$SO_2$- ($R^a$ and $R^b$ each independently represents hydrogen atom or a hydrocarbon group which may have substituent(s)); G represents a bond or a divalent group; L represents a bond or a divalent group; A represents hydrogen atom or a substituent; X and Y each represents hydrogen atom or an independent substituent; and ..... represents that $R^2$ and an atom on ring B may form a ring] or a salt thereof.

**18.** Use of a compound represented by the formula (I)

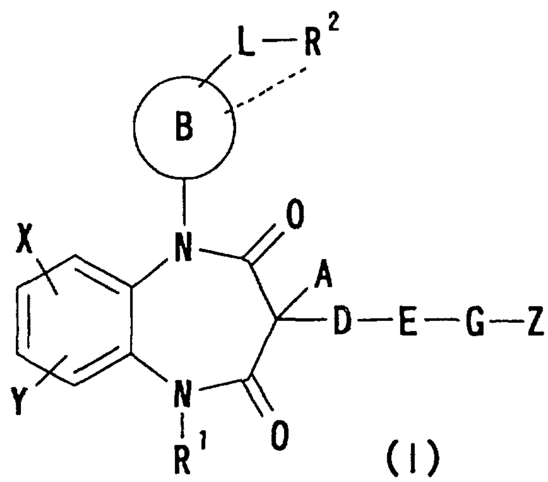

(I)

[wherein ring B represents a cyclic hydrocarbon group which may have substituent(s); Z represents hydrogen atom

or a cyclic group which may have substituent(s); $R^1$ represents hydrogen atom, a hydrocarbon group which may have substituent(s), a heterocyclic group which may have substituent(s) or an acyl group; $R^2$ represents amino group which may have substituent(s); D represents a bond or a divalent group; E represents a bond, -CO-, -CON($R^a$)-, -COO-, -N($R^a$)CON($R^b$)-, -N($R^a$)COO-, -N($R^a$)$SO_2$-, -N($R^a$)-, -O-, -S-, -SO- or -$SO_2$- ($R^a$ and $R^b$ each independently represents hydrogen atom or a hydrocarbon group which may have substituent(s)); G represents a bond or a divalent group; L represents a bond or a divalent group; A represents hydrogen atom or a substituent; X and Y each represents hydrogen atom or an independent substituent; and ..... represents that $R^2$ and an atom on ring B may form a ring] or a salt thereof, for manufacturing a medicament for regulating a somatostatin receptor function.

# INTERNATIONAL SEARCH REPORT

International application No.
PCT/JP99/05754

A. CLASSIFICATION OF SUBJECT MATTER
Int.Cl$^7$ C07D243/12 A61K31/551 C07D401/04, C07D403/04
A61P 3/04, A61P3/10

According to International Patent Classification (IPC) or to both national classification and IPC

B. FIELDS SEARCHED

Minimum documentation searched (classification system followed by classification symbols)
Int.Cl$^7$ C07D243/12 A61K31/551 C07D401/04, C07D403/04

Documentation searched other than minimum documentation to the extent that such documents are included in the fields searched

Electronic data base consulted during the international search (name of data base and, where practicable, search terms used)
CAPLUS, REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | CHRISTOS, P. et al. "A Non-peptide ligand for the somatostatin receptor having a benzodiazepinone structure" Bioorganic & Medicinal Chemistry Letters, vol. 6 (No.3), pages 267-272 (1996), 06 February, 1996 (06.02.96) the whole document | 1-18 |
| A | P.SHAH J. R. et al. "Novel 1,5-benzodiazepidinone Gastrin/CCKB Antagonists", Bioorganic & Medicinal Chemistry Letters, vol. 7 (No.3), pages 281-286 (1997), 04 Fevruary, 1997 (04.02.97) the whole document | 1-18 |
| A | BRAD R. HENKE et al. "Optimization of the 3-(1H-Indazol-3-ylmethyl)-1,5-benzodiazepines as Potent, Orally Active CCK-A Agonists", Journal of Medicinal Chemistry, vol. 40 (No.17), pages 2706-2725 (1997) | 1-18 |
| A | WO, 96/11691, A1 (GLAXO WELCOME Inc. USA), 25 April, 1996 (25.04.96) & EP, 785787, A1 & US, 5910495, A & JP, 10-511931, A | 1-18 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 January, 2000 (31.01.00) | 08 February, 2000 (08.02.00) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP99/05754

**Box I Observations where certain claims were found unsearchable (Continuation of item 1 of first sheet)**

This international search report has not been established in respect of certain claims under Article 17(2)(a) for the following reasons:

1. ☒ Claims Nos.: 17
because they relate to subject matter not required to be searched by this Authority, namely:

The subject matter of claim 17 relates to a method for treatment of the human body by therapy or operation, which does not require an international search report by this International Search Authority in accordance with Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)